# EUROPEAN PATENT SPECIFICATION

(11) **EP 3 409 766 B1**
(45) Date of publication and mention of the grant of the patent: **09.03.2022**
(21) Application number: 18175639.6
(22) Date of filing: 01.06.2018
(51) Int. Cl.: C12P 7/62, C12P 7/40, C12P 17/06

(54) **AN ESTERASE THAT IS CAPABLE OF CONVERTING IRIDOIDS AND SECO-IRIDOIDS**
ESTERASE MIT FÄHIGKEIT ZUR UMWANDLUNG VON IRIDOIDEN UND SECO-IRIDOIDEN
ESTÉRASE CAPABLE DE CONVERTIR DES IRIDOÏDES ET DES SÉCO-IRIDOÏDES

(30) Priority: 02.06.2017 EP 17174221
(43) Date of publication of application: 05.12.2018
(73) Proprietor: N-Zyme BioTec GmbH, 64293 Darmstadt (DE); Technische Universität Darmstadt, 64289 Darmstadt (DE)
(72) Inventor: SARAFEDDINOV, Alla, 64646 Heppenheim (DE); ZOTZEL, Jens, 64291 Darmstadt (DE); MARX, Stefan, 64380 Roßdorf (DE); WARZECHA, Heribert, 64285 Darmstadt (DE); VOLK, Jascha, 65824 Schwalbach (DE)
(74) Representative: Vossius & Partner Patentanwälte Rechtsanwälte mbB

(56) References cited:
- WO-A1-2013/101339
- WO-A1-2017/032722
- ES-A1- 2 233 208
- RAFFAELLA BRIANTE ET AL: "Production of highly purified hydroxytyrosol from Olea europaea leaf extract biotransformed by hyperthermophilic [beta]-glycosidase", JOURNAL OF BIOTECHNOLOGY, vol. 111, no. 1, 1 July 2004 (2004-07-01), pages 67-77, XP055243787, AMSTERDAM, NL ISSN: 0168-1656, DOI: 10.1016/j.jbiotec.2004.03.011
- DATABASE EMBL [Online] 1 July 2009 (2009-07-01), "OEAA-070810_Plate7o04.b1 cDNA library from Olive leaves and fruits Olea europaea cDNA, mRNA sequence.", XP002775583, retrieved from EBI accession no. EM_EST:GO245644 Database accession no. GO245644
- DATABASE Protein [Online] 19 November 2017 (2017-11-19), "salicylic acid-binding protein 2-like [Olea europaea var. sylvestris]", XP002785110, retrieved from NCBI Database accession no. XP_022858350.1

## Description

The present invention relates to a polypeptide having esterase activity selected from the group consisting of (a) a polypeptide comprising the amino acid sequence of SEQ ID NO: 7; (b) a polypeptide that is at least 69 % identical to the amino acid sequence of SEQ ID NO: 7 and which is characterized by having an esterase activity to convert (i) an oleuropein aglycone into oleacein, (ii) a ligstroside aglycone into oleocanthal, or (iii) elenolic acid to decarboxymethyl elenolic acid dialdehyde (DEDA); (c) a polypeptide encoded by the nucleic acid molecule of SEQ ID NO: 5 or 6; (d) a polypeptide encoded by a nucleic acid molecule which is at least 69 % identical to the nucleic acid sequence of SEQ ID NO: 5 or 6 wherein said polypeptide is characterized by having an esterase activity to convert (i) an oleuropein aglycone into oleacein, (ii) a ligstroside aglycone into oleocanthal, or (iii) elenolic acid to decarboxymethyl elenolic acid dialdehyde (DEDA); and (e) a polypeptide encoded by the complementary sequence of a nucleic acid molecule that is able to hybridize with the nucleic acid molecule of SEQ ID NO: 5 or 6. The present invention also relates to an organism, preferably a plant cell, genetically engineered with a nucleic acid molecule encoding a polypeptide characterized by having an esterase activity or the vector of the present invention. The present invention also relates to a method for the production of oleacein, oleocanthal, and/or decarboxymethyl elenolic acid dialdehyde (DEDA) comprising the use of the esterase of the present invention.

The olive *(Olea europaea)* has been a highly bred crop plant since several thousand years. Today, almost 2500 varieties are known, which provide fruits for oil production as well as to serve as table olives. Beside the highly valuable fatty oil, olive produces a number of secondary compounds, which belong to different classes of metabolites. As many other genera of the *Oleaceae* family, olive has a high content in phenolic compounds, which are formally seco-iridoid glycosides that could be esterified with a phenolic moiety.

Chromatographic analysis of extra virgin olive oil (EVOO) showed that the total phenolic compound in EVOO ranged between 14.8 to 121.20 mg/100g, mainly attributable to deacetoxyligstroside aglycone, deacetoxyoleuropein aglycone, oleuropein aglycone and ligstroside aglycone (Galvano et al., Journal of Medicinal Food 10(4) (2007), 650-656).

Further phenolic compounds which may be detected in virgin olive oils are hydroxytyrosol, hydroxytyrosol acetate, 3,4-dihydroxyphenylethyl-[(2,6-dimethoxy-3-ethylidene) tetrahydropyran-4-yl]acetate, 10-hydroxy oleuropein aglycone, 10-hydroxy oleuropein aglycone decarboxymethyl, 10-hydroxy-10-methyl oleuropein aglycone, elenolic acid, dialdehydic elenolic acid decarboxymethyl, dialdehydic elenolic ester decarboxymethyl, vanillic acid, coumaric acid, and (+)-pinoresinol (Savarese et al., Food Chem. 105 (2007), 761-770; Gariboldi et al., Phytochemistry 25 (1986), 865-869; Perez-Trujillo et al., J. Agric. Food Chem. 58 (2010), 9129-9136; Montedoro et al., J. Agric. Food Chem. 41 (1993), 2228-2234; and Brenes et al., J. Am. Oil Chem. Soc. 77 (2000), 715-720).

Further, Christophoridou et al. (J Agric Food Chem. 53(12) (2005), 4667-4679) identified various phenolic compounds in olive oil by coupling HPLC with post column solid-phase extraction to nuclear magnetic resonance spectroscopy (LC-SPE-NMR). These include tyrosol acetate, vanillin, homovanillyl alcohol, (+)-1-acetoxypinoresinol, berchemol, 3-acetyloxy berchemol, hemiacetal of dialdehydic oleuropein aglycone decarboxymethyl, hemiacetal of dialdehydic ligstroside aglycone decarboxymethyl, and verucosin. Flavonoids such as apigenin and luteolin, other phenolics including 3,4-dihydroxyphenylethyl-4-formyl-3-formylmethyl-4-hexenoate and 4-hydroxyphenylethyl-4-formyl-3-formylmethyl-4-hexenoate (Scalzo and Scarpati, J. Nat. Prod. 56 (1993), 621-623; Perez-Trujillo et al., J. Agric. Food Chem. 58 (2010), 9129-9136), and isochromans, that is, 1-phenyl-6,7-dihydroxy-isochroman and 1-(3'-methoxy-4'-hydroxy)phenyl-6,7-dihydroxy-isochroman (Bianco et al., Food Chem. 77 (2002), 405-411) have also been reported from olive leaves, oil, and EVOO.

The basic feature of most phenolic compounds in *Oleaceae* is the iridoid moiety, whose biosynthesis can in principle be accomplished by at least two different pathways (Inouye and Uesato, Prog. Chem. Org. Nat. Prod. 50 (1986), 169-236). However, in *Oleaceae* plants only one pathway is present. Starting from the monoterpenoid precursor geranyl pyrophosphate (GPP), deoxyloganic acid is formed as a central intermediate to *Oleaceae-iridoids* in all genera. From this common intermediate, there are five basic options of rearranging and decorating the iridoid skeleton and these alternatives can be found in various genera (Jensen et al., Phytochemistry 60 (2002), 213-231). In *Olea* as well as in *Fraxinus* and *Syringa,* oleosides occur (or oleosidic seco-iridoids) which are characterized by an exocyclic 8,9-olefinic function.

While the biosynthesis of the oleosides is well established, all subsequent steps leading to the formation as well as degradation and rearrangement of the phenolic conjugates of oleosides are elusive. It has been proposed that tyrosol (derived from the amino acid tyrosine) is linked via an ester to oleoside 11-methylester, forming ligstroside. In a subsequent hydroxylation step at the 3-position of the tyrosol moiety the major component oleuropein is formed (Ryan et al., J Agri Food Chem 50 (2002), 6716-6724).

Oleuropein exhibits several pharmacological properties and, therefore, represents the best known component. Oleuropein is considered to act as antioxidant (Miles et al., Nutrition 21 (2005), 389-394), anti-inflammatory (Visioli et al., Biochem Biophys Res Commun. 247 (1998), 60-64), anti-cancer (Owen et al., Eur J Cancer. 36 (2000), 1235-1247), anti-atherogenic (Carkuccio et al., Arterioscler Thromb Vasc Biol. 23 (2003), 622-629.) and antimicrobial (Tripoli et al., 2005 Nutr Res Rev. Jun;18 (2005), 98-112.) agent. Further, oleuropein can contribute to the nutritional prevention of osteoporosis (Puel et al., Clin Nutr. 25 (2006), 859-68). Due to these favourable effects, it is commercially available as a food supplement (Omar, Sci Pharm. 78 (2010), 133-54).

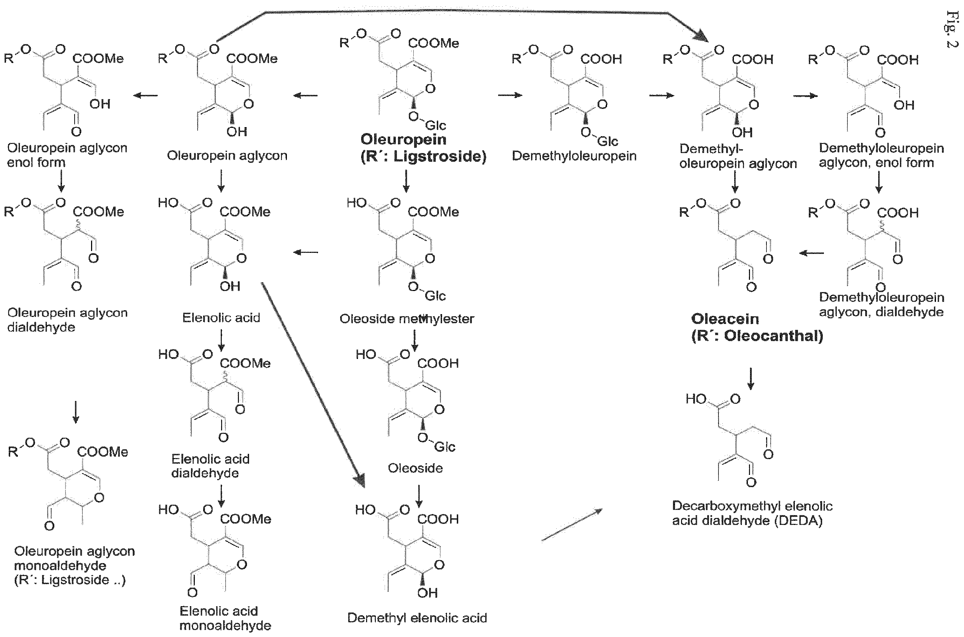

Oleuropein is the precursor for a number of degradation or rearrangement products (see Figure 2), wherein especially the glycosidic bond of the seco-iridoid moiety as well as the methyl ester and the hydroxytyrosol ester are prone to hydrolytic cleavage. It is the same with the precursor ligstroside which differs only in the replacement of hydroxytyrosol by tyrosol. However, no data about the sequence (or sequences) of the hydrolytic ester cleavage reactions are available. Moreover, analysis of the final products is often hampered by the strong reactivity of the oleosid aglycone, which is highly reactive in the dialdehyde-form and forms many side products. For the cleavage of the beta-glucosidic bond within oleuropein, an enzyme has been described recently (Koudounas et al., J Exp Bot 66(7) (2015), 2093-2106). Unfortunately, no thorough analysis of substrate acceptance or structure elucidation of the formed products has been undertaken. No esterase acting of oleuropein or related derivatives has been described so far. Although, the biochemical reaction and particularly the enzymes involved in these reactions are not known, many of these compounds are of high interest due to their inherent pharmacological effects. For the dialdehyde oleocanthal, for example, its *in vitro* anti-inflammatory (Beauchamp et al., Nature. 437 (2005), 45-46) and anti-neurodegenerative (Li et al., J Neurochem. 110 (2009), 1339-1351) properties were confirmed. Furthermore, anti-proliferative (Fabiani et al., J. Nutr. 136 (2006), 614-619) and anti-bacterial effects against *Helicobacter pylori* were reported (Romero et al., J Agric Food Chem. 55 (2007), 680-686. Among the many reported effects of the related oleacein are dose-dependent inhibition of CC-chemokine ligand 2 (CCL2) secretion and diminished monocyte adhesion (Sindona et al., Curr Med Chem. 19 (2012), 4006-4013.), increased NO formation in endothelial cells (Segade et al., J. Functional Foods 23 (2016), 637-646) and inhibition of the angiotensin converting enzyme (ACE) and lipoxygenases (Vougogiannopoulou et al., 2014 J Nat Prod. 77 (2014), 441-445). Moreover, oleocanthal has been shown to exhibit the same pharmacological activities as the nonsteroidal antiinflamatory drug (NSAID) ibuprofen, presumably due to the fact that both molecules inhibit the same cyclooxygenase in the prostaglandin biosynthetic pathway (Beauchamp et al., Nature. 437 (2005), 45-46). These characteristics lead to the acknowledgement of a health claim for olive polyphenols (EFSA J., 9 (2011), 2033).

However, isolation of oleocanthal and oleacein from olives is not only inefficient due to its very low content in the plant and in Extra Virgin Olive Oil (EVOO) (0.004-0.021 % in EVOO, Vulcano et al., Food Chem. 169 (2015), 381-386, but also unsustainable, as the chemical extraction of the beneficial compound is accompanied by the loss of a valuable resource and the accumulation of solvents (Garcia et al., Food Chem. 197 (2016), 554-561). Olive extracts have been used for example for the production of antioxidants by using microorganisms (ES 2 233 208 A1), for the conversion of oleuropein by enzyme preparations (WO 2017/032722 A1) and for hydrolysis by hyperthermophilic, immobilized beta-glucosidases (Briante et al., J Biotechn. 111 (2004), 67-77). However, unraveling the specific enzymes converting for example oleuropein aglycone, ligstroside aglycone or elenolic acid is crucial for the cost efficient and simple production of *Oleaceae-iridoids* and seco-iridoids. EMBL accession no. GO245644 (EBI accession no. FM_EST:GO245644; SEQ ID NO: 69) is a database entry that discloses an EST sequence that is 97% identical to SEQ ID NOs: 5 and 6 of the present invention. However, the EST sequence has not been biochemically characterized as having an esterase activity. Further, WO 2013/101339 A1 discloses esterases that are up to 55% identical to SEQ ID NO: 7 (SEQ ID NOs: 70 and 71 of the present application). Likewise, its alternative chemical synthesis for oleocanthal is disadvantageous in terms of costs, yield (7 %) and complex production processes (Smith et al., Org Lett. 7 (2005), 5075-5078).

Accordingly, given the numerous therapeutic applications of *Oleaceae-iridoids* and seco-iridoids, such as decarboxymethyl elenolic acid dialdehyde (DEDA), oleacein and oleocanthal there is an increasing demand for this compound. Thus, there is a need to provide alternatively methods for the environmentally friendly, cost efficient and simple production of *Oleaceae-*iridoids and seco-iridoids.

Therefore, the present invention meets this demand for an alternative process for the production of *Oleaceae-iridoids* and seco-iridoids, such as decarboxymethyl elenolic acid dialdehyde (DEDA), oleocanthal and oleacein and their precursor compounds, i.e. ligstroside aglycone and oleuropein aglycone which allows producing of oleocanthal and oleacein in vitro or in vivo in an organism, microorganism, preferably in a plant cell, by providing the subject-matter as recited in the claims.

The present invention not only relates to a method for the production of decarboxymethyl elenolic acid dialdehyde (DEDA), oleacein and/or oleocanthal. Rather the present invention relates to a polypeptide selected from the group consisting of:
(a) a polypeptide comprising the amino acid sequence of SEQ ID NO: 7;
(b) a polypeptide that is at least 69%, 69.3%, 70%, 71%, 71.4%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to the amino acid sequence of SEQ ID NO: 7 and which is characterized by having an esterase activity to convert (i) an oleuropein aglycone into oleacein, (ii) a ligstroside aglycone into oleocanthal, or (iii) elenolic acid to decarboxymethyl elenolic acid dialdehyde (DEDA);
(c) a polypeptide encoded by the nucleic acid molecule of SEQ ID NO: 5 or 6;
(d) a polypeptide encoded by a nucleic acid molecule which is at least 69%, 69.3%, 70%, 71%, 71.4%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to the nucleic acid sequence of SEQ ID NO: 5 or 6 and wherein said polypeptide is characterized by having an esterase activity to convert (i) an oleuropein aglycone into oleacein, (ii) a ligstroside aglycone into oleocanthal, or (iii) elenolic acid to decarboxymethyl elenolic acid dialdehyde (DEDA); and
(e) a polypeptide encoded by the complementary sequence of a nucleic acid molecule that is able to hybridize with the nucleic acid molecule of SEQ ID NO: 5 or 6 and wherein said polypeptide is characterized by having an esterase activity to convert (i) an oleuropein aglycone into oleacein, (ii) a ligstroside aglycone into oleocanthal, or (iii) elenolic acid to decarboxymethyl elenolic acid dialdehyde (DEDA).

Moreover, the present invention relates to a polypeptide selected from the group consisting of:
(a) a polypeptide comprising the amino acid sequence of SEQ ID NO: 7;
(b) a polypeptide that is at least 69%, 69.3%, 70%, 71%, 71.4%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to the amino acid sequence of SEQ ID NO: 7 and which is characterized by having an esterase activity to convert (i) an oleuropein aglycone into oleacein, (ii) a ligstroside aglycone into oleocanthal, or (iii) elenolic acid to decarboxymethyl elenolic acid dialdehyde (DEDA);
(c) a polypeptide encoded by the nucleic acid molecule of SEQ ID NO: 5 or 6;
(d) a polypeptide encoded by a nucleic acid molecule which is at least 69%, 69.3%, 70%, 71%, 71.4%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to the nucleic acid sequence of SEQ ID NO: 5 or 6 and wherein said polypeptide is characterized by having an esterase activity to convert (i) an oleuropein aglycone into oleacein, (ii) a ligstroside aglycone into oleocanthal, or (iii) elenolic acid to decarboxymethyl elenolic acid dialdehyde (DEDA); and
(e) a polypeptide encoded by the complementary sequence of a nucleic acid molecule that is able to hybridize with the nucleic acid molecule of SEQ ID NO: 5 or 6 and wherein said polypeptide is characterized by having an esterase activity to convert (i) an oleuropein aglycone into oleacein, (ii) a ligstroside aglycone into oleocanthal, or (iii) elenolic acid to decarboxymethyl elenolic acid dialdehyde (DEDA);
wherein a polypeptide selected from the group consisting of:
(i) a polypeptide comprising the amino acid sequence of SEQ ID NO: 49 or 60;
(ii) a polypeptide encoded by the nucleic acid molecule of SEQ ID NO: 48, 58 or 59 is excluded.

It may be preferred that any polypeptide selected from the group consisting of:
(iii) a polypeptide comprising the amino acid sequence of SEQ ID NO: 70 or 71;
(iv) a polypeptide encoded by the nucleic acid molecule of SEQ ID NO: 69 is excluded.

The esterase of the present invention is characterized in that the substrate of the enzyme is selected from aglycones of iridoids and seco-iridoids.

Thus, in the context of the present invention, but not part of the invention a polypeptide is described which has esterase activity and which is characterized in that the substrate of the polypeptide having esterase activity is preferably selected from iridoids and seco-iridoids and the aglycones of iridoids and seco-iridoids, more preferably a compound of Formula (I) 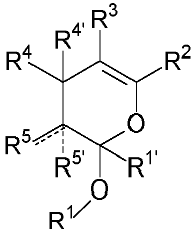 wherein
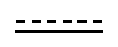 is either a single bond or a double bond, and
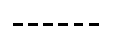 indicates that R^{5'} is either present or absent, depending on the absence or presence of a double bond in 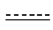 . More specifically, R^{5'} is absent if the 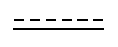 bonded to the same carbon atom as R^{5'} 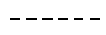 is a double bond. However, R^{5'} is present if 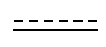 is a single bond.

R¹ is hydrogen or a group comprising at least one carbohydrate group. In the substrate of the polypeptide having esterase activity, R¹ is preferably hydrogen. If a glycosidase is used in combination with the esterase, the R¹ in the substrate of the glycosidase is preferably a group comprising at least one carbohydrate group. However, R¹ in the substrate of the polypeptide having esterase activity may also be a group comprising at least one carbohydrate group, even if no glycosidase is used in combination.

R^{1'}, R², R³, R⁴, R^{4'}, R⁵ and R^{5'} are each independently -H, -halogen, -CF₃, -CN, -OH, -CHO, -COOH, -COO-C₁₋₆ alkyl, or a hydrocarbon group containing from 1 to 25 carbon atoms, wherein the hydrocarbon group optionally contains 1 to 10 oxygen atoms, 1 to 5 nitrogen atoms, 1 to 3 sulfur atoms and/or 1 to 5 halogen atoms, and wherein one of the hydrocarbon groups of R^{1'}, R², R³, R⁴, R^{4'}, R⁵ and R^{5'} or the carbohydrate group in R¹ may be connected to one or two of the hydrocarbon groups of R^{1'}, R², R³, R⁴, R^{4'}, R⁵ and R^{5'}. The groups to be connected are preferably neighboring groups.

The connection between the one of the hydrocarbon groups of R^{1'}, R², R³, R⁴, R^{4'}, R⁵ and R^{5'} or the carbohydrate group in R¹ and the one or two of the hydrocarbon groups of R^{1'}, R², R³, R⁴, R^{4'}, R⁵ and R^{5'} is preferably one which results from removing a hydrogen radical (H·) from each hydrocarbon group or carbohydrate group which is to form a connection and connecting the resulting radicals of the hydrocarbon group(s) or carbohydrate group with each other in the positions in which the radicals have been formed.

The carbohydrate group in R¹ is preferably a carbohydrate group wherein one hydroxy group has been removed from a carbohydrate to form a bond between the carbon atom from which the hydroxy group has been removed and the O atom to which R¹ is connected. More preferably R¹ is glucosyl or rhamnosyl. Even more preferably, R¹ is a β-D-glucosyl residue or a β-D-rhamnosyl residue. Most preferably, R¹ is a β-D-glucosyl residue.

The carbohydrate group may be any substituted or unsubstituted carbohydrate group comprising from 1 to 10 sugar units, preferably 1 to 5 sugar units, more preferably one or two sugar units, each of which may independently be substituted with one or more hydrocarbon groups containing from 1 to 25 carbon atoms and optionally 1 to 10 oxygen atoms, 1 to 5 nitrogen atoms, 1 to 3 sulfur atoms and/or 1 to 5 halogen atoms. The sugar units, which may also be referred to as monosaccharide units, as used herein include sugar alcohol units and amino sugar units. The carbohydrate group is preferably glucose. Typical substituents of the carbohydrate group include acyl, isovaleroyl, isovanilloyl, benzoyl, p-hydroxybenzoyl, p-MeO-benzoyl, vanilloyl, veratroyl, syringyl, cinnamoyl, p-MeO-cinnamoyl, p-HO-cinnamoyl, o-HO-cinnamoyl, p-coumaroyl, feruloyl, isoferuloyl, caffeoyl, foliamenthoyl, dihydro-foliamenthoyl, menthiafoloyl, tigloyl, p-hydroxyphenethyl, dihyrocaffeoyl, apiofuranosyl, bis-foliamentoyl and sinapoyl, each of which may optionally be further substituted at one of its hydroxyl groups, if present, by glucosyl.

Preferably, R^{1'}, R², R³, R⁴, R^{4'}, R⁵ and R^{5'} are each independently selected from hydrogen, C₁₋₆ alkyl, C₂₋₆ alkenyl, C₂₋₆ alkynyl, heteroalkyl, cycloalkyl, heterocycloalkyl, aryl, heteroaryl, - R²¹-R²², -R²¹-OR²², -R²¹-OR²⁴, -R²¹-OR²¹-OR²², -R²¹-OR²¹-OR²⁴, -R²¹-SR²² -R²¹-SR²¹-SR²², -R²¹-NR²²R²², -R²¹-halogen, -R²¹-(C₁₋₆ haloalkyl), -R²¹-CN, -R²¹-CO-R²², -R²¹-CO-O-R²² , -R²¹-O-CO-R²², -R²¹-CO-NR²²R²², -R²¹-NR²²-CO-R²², -R²¹-SO₂-NR²²R²² and -R²¹-NR²²-SO₂-R²²; wherein said alkyl, said alkenyl, said alkynyl, said heteroalkyl, said cycloalkyl, said heterocycloalkyl, said aryl and said heteroaryl are each optionally substituted with one or more groups R²³.

Each R²¹ is independently selected from a single bond, C₁₋₆ alkylene, C₂₋₆ alkenylene, arylene and heteroarylene; wherein said alkylene, said alkenylene, said arylene and said heteroarylene are each optionally substituted with one or more groups R²³.

Each R²² is independently selected from hydrogen, C₁₋₆ alkyl, C₂₋₆ alkenyl, C₂₋₆ alkynyl, heteroalkyl, cycloalkyl, heterocycloalkyl, aryl and heteroaryl; wherein said alkyl, said alkenyl, said alkynyl, said heteroalkyl, said cycloalkyl, said heterocycloalkyl, said aryl and said heteroaryl are each optionally substituted with one or more groups R²³.

Each R²³.is independently selected from C₁₋₆ alkyl, C₂₋₆ alkenyl, C₂₋₆ alkynyl, -(C₀₋₃ alkylene)-OH, -(C₀₋₃ alkylene)-O-R²⁴, -(C₀₋₃ alkylene)-O(C₁₋₆ alkyl), -(C₀₋₃ alkylene)-O-aryl, -(C₀₋₃ alkylene)-O(C₁₋₆ alkylene)-OH, -(C₀₋₃ alkylene)-O(C₁₋₆ alkylene)-O-R²⁴, -(C₀₋₃ alkylene)-O(C₁₋₆ alkylene)-O(C₁₋₆ alkyl), -(C₀₋₃ alkylene)-SH, -(C₀₋₃ alkylene)-S(C₁₋₆ alkyl), -(C₀₋₃ alkylene)-S-aryl, -(C₀₋₃ alkylene)-S(C₁₋₆ alkylene)-SH, -(C₀₋₃ alkylene)-S(C₁₋₆ alkylene)-S(C₁₋₆ alkyl), -(C₀₋₃ alkylene)-NH₂, -(C₀₋₃ alkylene)-NH(C₁₋₆ alkyl), -(C₀₋₃ alkylene)-N(C₁₋₆ alkyl)(C₁₋₆ alkyl), -(C₀₋₃ alkylene)-halogen, -(C₀₋₃ alkylene)-(C₁₋₆ haloalkyl), -(C₀₋₃ alkylene)-CN, -(C₀₋₃ alkylene)-CHO, -(C₀₋₃ alkylene)-CO-(C₁₋₆ alkyl), - (C₀₋₃ alkylene)-COOH, -(C₀₋₃ alkylene)-CO-O-(C₁₋₆ alkyl), -(C₀₋₃ alkylene)-O-CO-(C₁₋₆ alkyl), -(C₀₋₃ alkylene)-CO-NH₂, -(C₀₋₃ alkylene)-CO-NH(C₁₋₆ alkyl), -(C₀₋₃ alkylene)-CON(C₁₋₆ alkyl)(C₁₋₆ alkyl), -(C₀₋₃ alkylene)-NH-CO-(C₁₋₆ alkyl), -(C₀₋₃ alkylene)-N(C₁₋₆ alkyl)-CO-(C₁₋₆ alkyl), -(C₀₋₃ alkylene)-SO₂-NH₂, -(C₀₋₃ alkylene)-SO₂-NH(C₁₋₆ alkyl), - (C₀₋₃ alkylene)-SO₂-N(C₁₋₆ alkyl)(C₁₋₆ alkyl), -(C₀₋₃ alkylene)-NH-SO₂-(C₁₋₆ alkyl), and - (C₀₋₃ alkylene)-N(C₁₋₆ alkyl)-SO₂-(C₁₋₆ alkyl); wherein said alkyl, said alkenyl, said alkynyl and the alkyl or alkylene moieties comprised in any of the aforementioned groups R²³.are each optionally substituted with one or more groups independently selected from halogen, - CF₃, -CN, -OH, -O-R²⁴, -O-C₁₋₄ alkyl and -S-C₁₋₄ alkyl;

Each R²⁴ is a monosaccharide, such as glucosyl and rhamnosyl, each of which can optionally be substituted with one or two of acyl, isovaleroyl, isovanilloyl, benzoyl, p-hydroxybenzoyl, p-MeO-benzoyl, vanilloyl, veratroyl, syringyl, cinnamoyl, p-MeO-cinnamoyl, p-HO-cinnamoyl, o-HO-cinnamoyl, p-coumaroyl, feruloyl, isoferuloyl, caffeoyl, foliamenthoyl, dihydro-foliamenthoyl, menthiafoloyl, tigloyl, p-hydroxyphenethyl, dihyrocaffeoyl, apiofuranosyl, bis-foliamentoyl and sinapoyl.

It is preferred that both R^{1'} and R² each be hydrogen.

Most preferably R^{1'}, R², R³, R⁴, R^{4'}, R⁵ and R^{5'} are independently as follows:
R^{1'} is preferably hydrogen.
R² is preferably hydrogen.
R³ is preferably hydrogen, CH₂OH, CH₂O-R^{x}, CHO, C₁₋₆ alkyl, COOH, C(O)O-C₁₋₆ alkyl, or C(O)O-C₁₋₆ alkylene-(phenyl which may be substituted with one or two or three of hydroxy, methyl and halogen), more preferably hydrogen, COOH or C(O)O-C₁₋₆ alkyl, even more preferably COOH or C(O)OMe.
R⁴ is preferably CH₂CH₂OH, CH₂CHO, CH₂COOH, CH₂COO-C₁₋₆ alkyl, CH_{2C}(O)-C₁₋₆ alkyl or CH₂COO-(C₁₋₆ alkylene)-(phenyl which may be substituted with one or two or three independently selected from -OH, -C₁₋₆-alkyl, -O-R^{x} and -halogen).
R^{4'} is preferably hydrogen.
R⁵ is preferably selected from hydrogen, -C₂₋₆ alkenyl and =CH-C₁₋₅ alkyl, wherein C₂₋₆ alkenyl and C₁₋₆ alkyl are optionally substituted with one or more selected from -OH, O-Ac, O-R^{x}. Most preferably, R^{5'} is absent and R⁵ is =CHCH₃. If 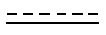 is a double bond, R⁵ is preferably optionally substituted =CH-C₁₋₆-alkyl, wherein the optional substituent of the optionally substituted C₁₋₆-alkyl is one or more independently selected from -OH and halogen. If 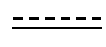 is a single bond, R⁵ is preferably optionally substituted C₂₋₆-alkenyl, wherein the optional substituent of the optionally substituted C₂₋₆-alkenyl is one or more, independently selected from -OH and halogen.
R^{5'} is preferably absent or hydrogen.
R^{x} may be any hydrocarbon group containing from 1 to 25 carbon atoms and optionally 1 to 10 oxygen atoms, 1 to 5 nitrogen atoms, 1 to 3 sulfur atoms and/or 1 to 5 halogen atoms. Preferred examples thereof include acyl, isovaleroyl, isovanilloyl, benzoyl, p-hydroxybenzoyl, p-MeO-benzoyl, vanilloyl, veratroyl, syringyl, cinnamoyl, p-MeO-cinnamoyl, p-HO-cinnamoyl, o-HO-cinnamoyl, p-coumaroyl, feruloyl, isoferuloyl, caffeoyl, foliamenthoyl, dihydro-foliamenthoyl, menthiafoloyl, tigloyl, p-hydroxyphenethyl, dihyrocaffeoyl, apiofuranosyl, bis-foliamentoyl and sinapoyl, each of which may optionally be further substituted at one of its hydroxyl groups, if present, by glucosyl. Further examples of R^{x} are glucosyl and rhamnosyl, each of which can optionally be substituted with one or two of acyl, isovaleroyl, isovanilloyl, benzoyl, p-hydroxybenzoyl, p-MeO-benzoyl, vanilloyl, veratroyl, syringyl, cinnamoyl, p-MeO-cinnamoyl, p-HO-cinnamoyl, o-HO-cinnamoyl, p-coumaroyl, feruloyl, isoferuloyl, caffeoyl, foliamenthoyl, dihydro-foliamenthoyl, menthiafoloyl, tigloyl, p-hydroxyphenethyl, dihyrocaffeoyl, apiofuranosyl, bis-foliamentoyl and sinapoyl.

When any of the hydrocarbon groups in any two or three of R^{1'}, R², R³, R⁴, R^{4'}, R⁵ and R^{5'} and/or the carbohydrate group in R¹ are connected to each other, they preferably form, together with the carbon atom(s) that they are attached to, a carbocyclic or heterocyclic ring, or more than one carbocyclic or heterocyclic ring, being optionally independently substituted with one or more substituents R²⁵. R²⁵ is independently selected from C₁₋₆ alkyl, C₂₋₆ alkenyl, C₂₋₆ alkynyl, heteroalkyl, cycloalkyl, heterocycloalkyl, aryl, heteroaryl, -R²¹-R²², -R²¹-OR²², -R²¹-OR²⁴, -R²¹-OR²¹-OR²², -R²¹-OR²¹-OR²⁴ ,-R²¹-SR²² -R²¹-SR²¹-SR²² -R²¹-NR²²R²², -R²¹-halogen, -R²¹-(C₁₋₆ haloalkyl), -R²¹-CN, -R²¹-CO-R²², -R²¹-CO-O-R²², -R²¹-O-CO-R²² , -R²¹-CO-NR²²R²² , -R²¹-NR²²-CO-R²², -R²¹-SO₂-NR²²R²² and -R²¹-NR²²-SO₂-R²²; wherein said alkyl, said alkenyl, said alkynyl, said heteroalkyl, said cycloalkyl, said heterocycloalkyl, said aryl and said heteroaryl are each optionally substituted with one or more groups R²³; The definitions of R²¹, R²², R²³.and R²⁴ are as set out above.

More preferably, the optional substituent of the carbocyclic or heterocyclic ring(s) is one or more groups independently selected from -C₁₋₆ alkyl, -C₂₋₆ alkenyl, -C₂₋₆ alkynyl, -C(O)-C₁₋₆ alkyl, -halogen, -CF₃, -CN, -OH, oxo, -CH₂C(O)OH, -CH₂C(O)OC₁₋₆ alkyl, -O-C₁₋₆ alkyl, -C₃₋₇ cycloalkyl, -COOR^{∗}, -(CH₂)_{q}-OR^{∗}, -(CH₂)_{q}-CN, -S(O)R^{∗}, -S(O)₂R^{∗}, - (CH₂)_{q}NR^{∗}R^{∗}, -C(O)-NR^{∗}R^{∗}, -NR^{∗}-C(O)-C₁₋₆ alkyl, -aryl, -heterocycloalkyl and - heteroaryl; wherein R^{∗} is selected from -H, -C₁₋₆ alkyl which is optionally substituted with one or more halogen atoms or -OH groups, -C(O)-C₁₋₆ alkyl which is optionally substituted with one or more halogen atoms or -OH groups, and phenyl which is optionally substituted with one or more halogen atoms or -OH groups and wherein q is an integer of from 1 to 6.

A preferred example of the compound of Formula (I) is the following formula 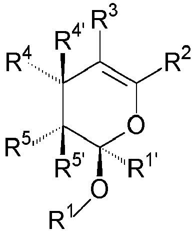 wherein the definition and preferred embodiments of R¹, R^{1'}, R², R³, R⁴, R^{4'}, R⁵ and R^{5'} as set out with respect to the compound of Formula (I) apply.

Further preferred examples of the compounds of formula (I) may have a structure of the following formula (Ia): 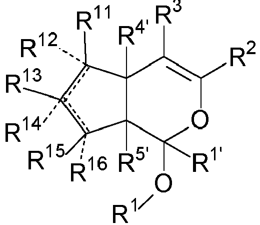 wherein
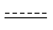 is either a single bond or a double bond, e.g., as shown in the following: 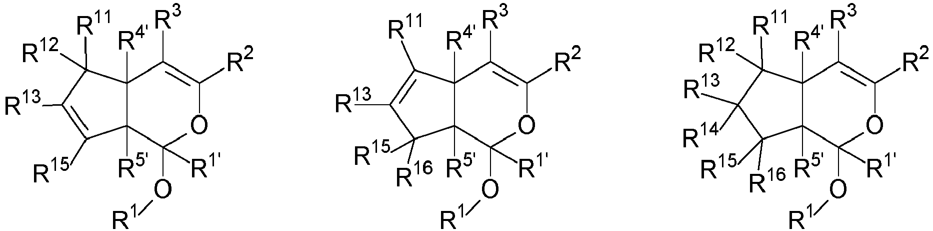
and 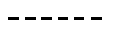 indicates that the respective substituents, in particular R¹², R¹⁴ and R¹⁶ are either present or absent, depending on the absence or presence of a double bond in 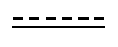 . More specifically, if a substituent is shown as bonded to a carbon atom via 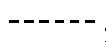 , this substituent is typically absent if 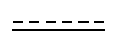 bonded to the same carbon atom is a double bond.

The following stereochemical orientation of the substituents is preferred in the compounds of formula (Ia): 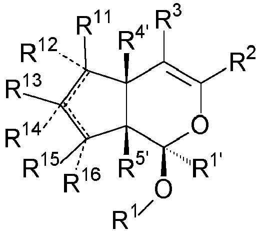 and correspondingly in the examples thereof: 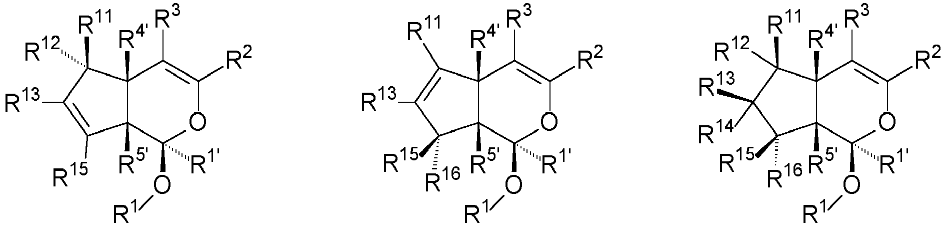 R¹, R^{1'}, R², R³, R^{4'} and R^{5'} are as defined in above with respect to formula (I), and R¹¹, R¹², R¹³, R¹⁴, R¹⁵ and R¹⁶ may be the same groups as the groups which are defined as any one of R^{1'}, R², R³, R⁴, R^{4'}, R⁵ and R^{5'} with respect to formula (I), including the preferred definitions of these groups.

Accordingly, R¹¹, R¹², R¹³, R¹⁴, R¹⁵ and R¹⁶ are preferably each independently -H, -halogen, -CF₃, -CN, -OH, -CHO, -COOH, or a hydrocarbon group containing from 1 to 25 carbon atoms and optionally 1 to 10 oxygen atoms, 1 to 5 nitrogen atoms, 1 to 3 sulfur atoms and/or 1 to 5 halogen atoms, and one of the hydrocarbon groups of R^{1'}, R² , R³, R^{4'}, R¹¹, R¹², R¹³, R¹⁴, R¹⁵ or R¹⁶ or the carbohydrate group in R¹ may be connected to one or two of the hydrocarbon groups of R^{1'}, R², R³, R^{4'}, R¹¹, R¹², R¹³, R¹⁴, R¹⁵ or R¹⁶.

Furthermore, any two of R¹¹ to R¹⁶, which are present on neighboring carbon atoms may form a group of the formula -O-. In particular, either R¹¹ and R¹³, or R¹³ and R¹⁵ may form a group of the formula -O-, thus resulting, with the carbon atoms to which each of these are attached, in an epoxy group.

It is preferred that one or more of R^{1'}, R^{4'}, R¹¹, R¹³ and R¹⁵ be hydrogen. Even more preferably, R^{1'} and R^{4'} are hydrogen and/or any two of R¹¹, R¹³ and R¹⁵ are also hydrogen.

Furthermore, it is preferred that one of R¹¹ and R¹², one of R¹³ and R¹⁴, and one of R¹⁵ and R¹⁶ be hydrogen.

It is preferred that not both of R¹¹ and R¹², both of R¹³ and R¹⁴, and/or both of R¹⁵ and R¹⁶ be -OH.

In the compound of formula (1a) and each of the examples thereof as described above, the following preferred definitions apply, either independently or in any combination thereof:
R^{1'} is preferably hydrogen.
R² is preferably hydrogen.
R³ is preferably selected from hydrogen, CH₂OH, CH₂-O-C(O)-C₁₋₆ alkyl, CH₂-O-R^{x}, CHO, C₁₋₆ alkyl, COOH, COO-C₁₋₆ alkyl, or COO-C₁₋₆ alkylene-(phenyl which is optionally substituted with one or two or three selected from OH and halogen). Most preferably, R³ is selected from hydrogen, CH₂OH and COO-C₁₋₆ alkyl.
R^{4'} is preferably hydrogen, -OH or -O-R^{x}, more preferably H.
R^{5'} is preferably hydrogen.
R¹¹ is preferably hydrogen, halogen, C₁₋₆ methyl, -OH, -O-C₁₋₆ methyl, -O-R^{x}, -COOH, - CHO, -CH₂-OH, -CH₂-halogen or -CH₂-O-R^{x}, more preferably H or OH.
R¹² , if present, is preferably hydrogen, halogen, C₁₋₆ methyl, -OH, -O-C₁₋₆ methyl, -O-R^{x}, - COOH, -CHO, -CH₂-OH, -CH₂-halogen or -CH₂-O-R^{x}, more preferably H or OH.
R¹³ is preferably hydrogen, halogen, C₁₋₆ methyl, -OH, -O-C₁₋₆ methyl, -O-R^{x}, -COOH, - CHO, -CH₂-OH, -CH₂-halogen or -CH₂-O-R^{x}, more preferably H or OH.
R¹⁴, if present, is preferably hydrogen, halogen, C₁₋₆ methyl, -OH, -O-C₁₋₆ methyl, -O-R^{x}, - COOH, -CHO, -CH₂-OH, -CH₂-halogen or -CH₂-O-R^{x}, more preferably H or OH.
R¹⁵ is preferably hydrogen, halogen, C₁₋₆ methyl, -OH, -O-C₁₋₆ methyl, -O-R^{x}, -COOH, - CHO, -CH₂-OH, -CH₂-halogen or -CH₂-O-R^{x}, more preferably H or OH.
R¹⁶, if present, is preferably hydrogen, halogen, C₁₋₆ methyl, -OH, -O-C₁₋₆ methyl, -O-R^{x}, - COOH, -CHO, -CH₂-OH, -CH₂-halogen or -CH₂-O-R^{x}, more preferably H or OH.

These preferred definitions are with the proviso that any two of R¹¹ to R¹⁶, which are present on neighboring carbon atoms may form a group of the formula -O-. In particular, either R¹¹ and R¹³ or R¹³ and R¹⁵ may form a group of the formula -O-, thus resulting, with the carbon atoms to which each of these are bound, in an epoxy group.

R^{x} may be any hydrocarbon group containing from 1 to 25 carbon atoms and optionally 1 to 10 oxygen atoms, 1 to 5 nitrogen atoms, 1 to 3 sulfur atoms and/or 1 to 5 halogen atoms. Preferred examples thereof include acyl, isovaleroyl, isovanilloyl, benzoyl, p-hydroxybenzoyl, p-MeO-benzoyl, vanilloyl, veratroyl, syringyl, cinnamoyl, p-MeO-cinnamoyl, p-HO-cinnamoyl, o-HO-cinnamoyl, p-coumaroyl, feruloyl, isoferuloyl, caffeoyl, foliamenthoyl, dihydro-foliamenthoyl, menthiafoloyl, tigloyl, p-hydroxyphenethyl, dihyrocaffeoyl, apiofuranosyl, bis-foliamentoyl and sinapoyl, each of which may optionally be further substituted at one of its hydroxyl groups, if present, by glucosyl. Further examples of R^{x} are glucosyl and rhamnosyl, each of which can optionally be substituted with one or two of acyl, isovaleroyl, isovanilloyl, benzoyl, p-hydroxybenzoyl, p-MeO-benzoyl, vanilloyl, veratroyl, syringyl, cinnamoyl, p-MeO-cinnamoyl, p-HO-cinnamoyl, o-HO-cinnamoyl, p-coumaroyl, feruloyl, isoferuloyl, caffeoyl, foliamenthoyl, dihydro-foliamenthoyl, menthiafoloyl, tigloyl, p-hydroxyphenethyl, dihyrocaffeoyl, apiofuranosyl, bis-foliamentoyl and sinapoyl.

The present disclosure also encompasses dimers, trimers, tetramers, pentamers and hexamers of the compounds described herein which may be homomers or heteromers and which are preferably formed by an ester linkage such as resulting from the reaction of hydroxyl groups and carboxylic ester groups present in one or more of the compounds described herein.

Accordingly, as shown in the appended Examples, esterases (SEQ ID NOs: 7, 24 or 37) from *Oleaceae* with activity against the ester moiety within some olive polyphenols was identified. Further, other than expected from the literature data, it was proven that the enzyme does not accept oleuropein, ligstroside or oleoside 11-methylester as substrates but shows strong activity against the aglyca of the aforementioned compounds. Moreover, it specifically cleaves the methylester while not releasing the tyrosol or hydroxytyrosol moiety.

The product which is obtained when using the polypeptide having esterase activity in the context of the present invention has preferably a structure of the following formula (II) 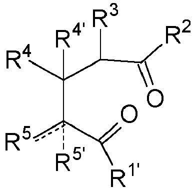 wherein the same definitions, including preferred definitions, as set out with respect to the compound of Formula (I) apply. In particular, R^{1'}, R², R³, R⁴, R⁴, R⁵ and R^{5'} are as defined with respect to the compound of Formula (I). In formula (II), R³ is most preferably hydrogen.

Other preferred examples thereof include compounds of the following formula (IIa) 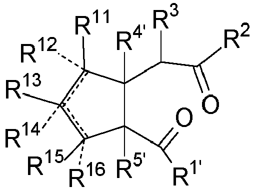 wherein the same definitions, including preferred definitions, as set out with respect to the compound of Formula (Ia) apply. In particular, R^{1'}, R², R³, R^{4'}, R^{5'}, R¹¹, R¹², R¹³, R¹⁴, R¹⁵ and R¹⁶ are as defined with respect to the compound of Formula (Ia). Furthermore, the options concerning the position of the double bond and the stereochemistry as set out with respect to the compounds of formula (Ia) apply correspondingly. In formula (IIa), R³ is most preferably hydrogen.

In the context of the present invention, the term "hydrocarbon group" refers to a group consisting of carbon atoms and hydrogen atoms. Examples of this group are alkyl, alkenyl, alkynyl, alkylene, carbocyl and aryl. Both monovalent and divalent groups are encompassed. If the "hydrocarbon group" is described as optionally including heteroatoms, such as "hydrocarbon group containing from 1 to 25 carbon atoms and optionally 1 to 10 oxygen atoms, 1 to 5 nitrogen atoms, 1 to 3 sulfur atoms and/or 1 to 5 halogen atoms", the hydrocarbon group may be bound via a carbon atom or one of its heteroatoms such as an oxygen atom. It is to be understood that the term "hydrocarbon group" optionally including heteroatoms also encompasses -COO-C₁₋₆ alkyl.

In the context of the present invention, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₆ alkyl" denotes an alkyl group having 1 to 6 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₄ alkyl, more preferably to methyl or ethyl, and even more preferably to methyl.

Furthermore, in the context of the present invention, the term "alkylene" refers to an alkanediyl group, i.e. a divalent saturated acyclic hydrocarbon group which may be linear or branched. A "C₁₋₆ alkylene" denotes an alkylene group having 1 to 6 carbon atoms, and the term "C₀₋₃ alkylene" indicates that a covalent bond (corresponding to the option "C₀ alkylene") or a C₁₋₃ alkylene is present. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene, and even more preferably to methylene.

In the context of the present invention, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₆ alkenyl" denotes an alkenyl group having 2 to 6 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to C₂₋₄ alkenyl. The term "alkenylene" corresponds to the definition of "alkenyl" with the only difference being that the group is not monovalent but divalent.

As used herein, the term "alkynyl" refers to a monovalent unsaturated acyclic hydrocarbon group which may be linear or branched and comprises one or more (e.g., one or two) carbon-to-carbon triple bonds and optionally one or more carbon-to-carbon double bonds. The term "C₂₋₆ alkynyl" denotes an alkynyl group having 2 to 6 carbon atoms. Preferred exemplary alkynyl groups are ethynyl, propynyl, or butynyl. Unless defined otherwise, the term "alkynyl" preferably refers to C₂₋₄ alkynyl.

In the context of the present invention, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Aryl" may, e.g., refer to phenyl, naphthyl, dialinyl (i.e., 1,2-dihydronaphthyl), tetralinyl (i.e., 1,2,3,4-tetrahydronaphthyl), anthracenyl, or phenanthrenyl. Unless defined otherwise, an "aryl" preferably has 6 to 14 ring atoms, more preferably 6 to 10 ring atoms, and most preferably refers to phenyl. The term "arylene" corresponds to the definition of "aryl" with the only difference being that the group is not monovalent but divalent.

In the context of the present invention, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromenyl, xanthenyl, phenoxathiinyl, pyrrolyl (e.g., 2H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolizinyl, isoindolyl, indolyl (e.g., 3H-indolyl), indazolyl, purinyl, isoquinolyl, quinolyl, phthalazinyl, naphthyridinyl, quinoxalinyl, cinnolinyl, pteridinyl, carbazolyl, beta-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, furazanyl, phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzoxazolyl, benzisoxazolyl, benzimidazolyl, 1H-tetrazolyl, 2H-tetrazolyl, coumarinyl, or chromonyl. Unless defined otherwise, a "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized. The term "heteroarylene" corresponds to the definition of "heteroarylene" with the only difference being that the group is not monovalent but divalent.

The term "heteroalkyl" refers to saturated linear or branched-chain monovalent hydrocarbon radical of one to twelve carbon atoms, including from one to six carbon atoms and from one to four carbon atoms, wherein at least one of the carbon atoms is replaced with a heteroatom selected from N, O, or S, and wherein the radical may be a carbon radical or heteroatom radical (i.e., the heteroatom may appear in the middle or at the end of the radical). The heteroalkyl radical may be optionally substituted independently with one or more substituents described herein. The term "heteroalkyl" encompasses alkoxy and heteroalkoxy radicals. The term "heteroalkylene" corresponds to the definition of "heteroalkyl" with the only difference being that the group is not monovalent but divalent.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, or adamantyl. Unless defined otherwise, "cycloalkyl" preferably refers to a C₃₋₁₁ cycloalkyl, and more preferably refers to a C₃₋₇ cycloalkyl. A particularly preferred "cycloalkyl" is a monocyclic saturated hydrocarbon ring having 3 to 7 ring members. The term "cycloalkylene" corresponds to the definition of "cycloalkyl" with the only difference being that the group is not monovalent but divalent.

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). "Heterocycloalkyl" may, e.g., refer to oxetanyl, tetrahydrofuranyl, piperidinyl, piperazinyl, aziridinyl, azetidinyl, pyrrolidinyl, imidazolidinyl, morpholinyl (e.g., morpholin-4-yl), pyrazolidinyl, tetrahydrothienyl, octahydroquinolinyl, octahydroisoquinolinyl, oxazolidinyl, isoxazolidinyl, azepanyl, diazepanyl, oxazepanyl or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized. The term "heterocycloalkylene" corresponds to the definition of "heterocycloalkyl" with the only difference being that the group is not monovalent but divalent.

As used herein, the term "halogen" refers to fluoro (-F), chloro (-Cl), bromo (-Br), or iodo (-I).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. Unless defined otherwise, "heterocyclyl" preferably refers to heteroaryl, heterocycloalkyl or heterocycloalkenyl.

As used herein, the term "heterocyclic ring" refers to saturated or unsaturated rings containing one or more heteroatoms, preferably selected from oxygen, nitrogen and sulfur. Preferably, the "heterocyclic ring" contains from 2 to 20 carbon atoms and from 1 to 6 heteroatoms selected from N, O and S. Examples include heteroaryl and heterocycloalkyl as defined herein. Preferred examples contain 5 or 6 atoms, particular examples, are 1,4-dioxane, pyrrole and pyridine. It is to be understood that each of the heterocyclic rings may independently be saturated or contain one or two or three unsaturated carbon-carbon bonds.

The term "carbocyclic ring" means saturated or unsaturated carbon rings such as aryl or cycloalkyl, preferably containing 5 or 6 carbon atoms. Examples include aryl and cycloalkyl as defined herein. It is to be understood that each of the carbocyclic may independently be saturated or contain one or two or three unsaturated carbon-carbon bonds.

As used herein, the term "carbohydrate group" preferably refers to groups comprising from 1 to 10 monosaccharide units and includes monosaccharides, disaccharides and oligosaccharides.

The term "monosaccharide" or "sugar" as used herein refers to sugars which consist of only a single sugar unit. These include all compounds which are commonly referred to as sugars and include sugar alcohols and amino sugars. Examples include tetroses, pentoses, hexoses and heptoses, in particular aldotetroses, aldopentoses, aldohexoses and aldoheptoses.

As used herein, the term "disaccharide" refers to a group which consists of two monosaccharide units. Disaccharides may be formed by reacting two monosaccharides in a condensation reaction which involves the elimination of a small molecule, such as water.

Examples of disaccharides are maltose, isomaltose, lactose, nigerose, sambubiose, sophorose, trehalose, saccharose, rutinose, and neohesperidose.

As used herein, the term "oligosaccharide" refers to a group which consists of three to eight monosaccharide units. Oligosaccharide may be formed by reacting three to eight monosaccharides in a condensation reaction which involves the elimination of a small molecule, such as water. The oligosaccharides may be linear or branched.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

In the context of the present invention, compounds to be converted by the provided polypeptide include the following compounds which have been disclosed by Dinda et al., Chem. Pharm. Bull. 55(2) (2007), 159-222 including any aglycons, in particular those where at least one glycosyl residue has been removed, thereof:
7β-Acetoxy-10-*O*-acetyl-8α-hydroxydecapetaloside , 10-Acetoxymajoroside, 7-*O*-Acetyl-10-O-acetoxyloganin, 6-*O*-Acetylajugol, 6-*O*-(2"-*O*-Acetyl-3"-*O*-cinnamoyl-4"-*O-p*-methoxy cinnamoyl-α-Lrhamnopyranosyl) catalpol, 6-*O*-(3"-*O*-Acetyl-2"-*O-trans*-cinnamoyl)-α-L-rhamnopyranosyl catalpol, 8-*O*-Acetylclandonoside, 8-*O*-Acetyl-6'-*O*-(p-coumaroyl)harpagide, 8-*O*-Acetyl-6-*O*-*trans-p*-coumaroylshanzhiside, 6'-Acetyl deacetylasperuloside, 8-*O*-Acetyl-1-*epi*-shanzhigenin methyl ester, Acetylgaertneroside, 10-O-Acetylgeniposidic acid, 10-*O*-Acetyl-8α-hydroxydecapetaloside, 8-*O*-Acetyl-6β-hydroxyipolamiide, 2'-*O*-Acetyllamiridoside, 3'-*O*-Acetylloganic acid, 4'-*O*-Acetylloganic acid, 6'-*O*-Acetylloganic acid, 6β-Acetyl-7β-(*E*)-*p*-methoxycinnamoyl-myxopyroside, 6β-Acetyl-7β-(*Z*)-*p*-methoxycinnamoyl-myxopyroside, 10-*O*-Acetylmonotropein, 8*-O-*Acetylmussaenoside, 10-*O*-Acetylpatrinoside, 3'-*O*-Acetylpatrinoside, 6'-*O*-Acetylplumieride-*p-E*-coumarate, 6'-*O*-Acetylplumieride-*p-Z*-coumarate, 6-*O*-Acetylscandoside, 8-*O-*Acetylshanzhigenin methyl ester, 8-*O*-Acetylshanzhiside, Acuminatuside, Agnucastoside A (6'-*O*-Foliamenthoylmussaenosidic acid), Agnucastoside B (6'-*O*-(6,7-Dihydrofoliamenthoyl)-mussaenosidic acid), Agnucastoside C (*7-O-trans-p-*Coumaroyl*-6'-O-trans-*caffeoyl*-8-epi-*loganic acid), Alatoside, Alboside I, Alboside II, Alboside III, Alpinoside, Angeloside, 6-*O*-β-D-Apiofuranosylmussaenosidic acid, 2'-*O*-Apiosylgardoside, Aquaticoside A (6'-*O*-Benzoyl-8-*epi*-loganic acid), Aquaticoside B (6'-*O-p*-Hydroxybenzoyl-8-*epi*-loganic acid), Aquaticoside C (6'-*O*-Benzoylgardoside), Arborescoside, Arborescosidic acid, Arborside D, Arcusangeloside, Artselaenin A, Artselaenin C, Artselaenin B, Asperuloide A, Asperuloide B, Asperuloide C, Asperulosidic acid ethyl ester, 6-*O*-α-L-(2"-*O*-Benzoyl,3"-*O-trans-p-*coumaroyl)rhamnopyranosylcatalpol, 10-*O*-Benzoyldeacetylasperulosidic acid, 6'-*O*-Benzoyl-8-*epi*-loganic acid, 6'-*O*-Benzoylgardoside, 10-*O*-Benzoylglobularigenin, 10-Bisfoliamenthoylcatalpol, Blumeoside A, Blumeoside B, Blumeoside C, Blumeoside D, Boucheoside, Brunneogaleatoside, 3β-Butoxy-3,4-dihydroaucubin, 6-*O*-Butylaucubin, 6-*O-*Butyl-*epi*-aucubin, 6-*O*-Caffeoylajugol, 10-*O*-Caffeoylaucubin, *6'-O-trans-*Caffeoylcaryoptosidic acid, 10-*O*-*trans-p*-Caffeoylcatalpol, 10-*O*-*E*-Caffeoylgeniposidic acid, 2'-Caffeoylmussaenosidic acid, 6'-*O*-*trans*-Caffeoylnegundoside, Caryoptosidic acid, Caudatoside A, Caudatoside B, Caudatoside C, Caudatoside D, Caudatoside E, Caudatoside F, Chlorotuberoside, 10-*O*-(Cinnamoyl)-6'-(desacetyl-alpinosidyl)catalpol, 10*-O-E-*Cinnamoylgeniposidic acid, 8-*O*-Cinnamoylmussaenosidic acid, 8-Cinnamoylmyoporoside, 7β-Cinnamoyloxyugandoside (Serratoside A), 7-*O*-*trans-p*-Coumaroyl-6'-*O*-*trans-*caffeoyl-8-*epi*-loganic acid, 6-*O*-α-L-(2"-*O*-*trans-*Cinnamoyl)-rhamnopyranosylcatalpol, 6-*O*-α-L-(3"-*O-trans*-Cinnamoyl)-rhamnopyranosylcatalpol, 6-*O*-α-L-(4"-*O*-*trans*-Cinnamoyl)-rhamnopyranosylcatalpol, Citrifolinin A, Citrifolinoside A, Clandonensine, Clandonoside, Clandonoside II, Coelobillardin, 6-*O*-*trans-p*-Coumaroyl-8-*O*-acetylshanzhiside methyl ester, 6-*O*-*cis-p*-Coumaroyl-8-*O*-acetylshanzhiside methyl ester, 6'-*O*-(*p*-Coumaroyl)antirrinoside, 10-*O*-*cis-p*-Coumaroylasystasioside E, 10-*O*-*trans-p*-Coumaroylasystasioside E, 6-*O*-*p-*Coumaroylaucubin, 6'-*O-p-trans*-Coumaroylcaryoptosidic acid, 6*-O-cis-p-*Coumaroylcatalpol, 10-*O*-*cis-p*-Coumaroylcatalpol, 6*-O-trans-p-*Coumaroyl-7-deoxyrehmaglutin A, 6-*O*-c*is-p*-Coumaroyl-7-deoxyrehmaglutin A, 2"*-trans-p-*Coumaroyldihydropenstemide, 2'-*O*-Coumaroyl-8-*epi-*tecomoside, 10*-O-trans-*Coumaroyleranthemoside, 10-*O*-*E-p-*Coumaroylgeniposidic acid, 7-*O*-Coumaroylloganic acid, Crescentin I, Crescentin II, Crescentin III, Crescentin IV, Crescentin V, 6'*-O-trans-p-*Coumaroylloganin, 6'*-O-cis-p*-Coumaroylloganin, 7-*O-p*-Coumaroylpatrinoside, 2'-*O-*Coumaroylplantarenaloside, 6-*O*-(4"-*O*-*p*-Coumaroyl-β-D-xylopyranosyl)-aucubin, 7β-Coumaroyloxyugandoside, Crescentoside A, Crescentoside B, Crescentoside C, Cyanogenic glycoside of geniposidic acid, Daphcalycinosidine A, Daphcalycinosidine B, Davisioside, Deacetylalpinoside (Arborescosidic acid), Dehydrogaertneroside, Dehydromethoxygaertneroside, 5-Deoxyantirrhinoside, 4"-Deoxykanokoside A, 4"-Deoxykanokoside C, 6-Deoxymelittoside, 5-Deoxysesamoside, Desacetylhookerioside, Des-*p*-hydroxybenzoylkisasagenol B, 2",3"-Diacetylisovalerosidate, 2",3"-Diacetylvalerosidate, 6-*O*-α-L-(2"-*O*-,3"-*O*-Dibenzoyl,4"-*O*-*cis-p*-coumaroyl)rhamnopyranosylcatalpol, 6-*O*-α-L-(2"-*O*-,3"-*O*-Dibenzoyl,4"-*O*-*trans-p*-coumaroyl)rhamnopyranosylcatalpol, 6-*O*-α-L-(2"-*O*-,3"-*O-*Dibenzoyl)rhamnopyranosylcatalpol, 6α-Dihydrocornic acid, 6β-Dihydrocornic acid, 6'-*O-*(6,7-Dihydrofoliamenthoyl)-mussaenosidic acid, 3,4-Dihydro-3α-methoxypaederoside, 3,4-Dihydro-3β-methoxypaederoside, 3,4-Dihydro-6-*O*-methylcatalpol, 5,6β-Dihydroxyadoxoside, 2'-(2,3-Dihydroxybenzoyloxy)-7-ketologanin, 5β ,6β-Dihydroxyboschnaloside, Dimer of paederosidic acid, Dimer of paederosidic acid and paederoside, Dimer of paederosidic acid and paederosidic acid methyl ester, 6-*O*-(3,4-Dimethoxybenzoyl)crescentin IV 3-*O*-β-D-glucopyranoside, 10-*O*-(3,4-Dimethoxy-(*E*)-cinnamoyl)-aucubin, 10-*O*-(3,4-Dimethoxy-(*Z*)-cinnamoyl)-catalpol, 10-*O*-(3,4-Dimethoxy-(E)-cinnamoyl)-catalpol, 6-*O*-[3-*O*-(*trans*-3,4-Dimethoxycinnamoyl)-α-L-rhamnopyranosyl]-aucubin, Dumuloside, Dunnisinin, Dunnisinoside, Duranterectoside A, Duranterectoside B, Duranterectoside C, Duranterectoside D, *6-epi-* 8-*O*-Acetylharpagide, *6-O-epi-*Acetylscandoside, 6,9-*epi*-8-*O*-Acetylshanzhiside methyl ester, 8-*epi*-Apodantheroside, 1,5,9-*epi*-Deoxyloganic acid glucosyl ester, 5,9-*epi*-7,8-Didehydropenstemoside, (5α-H)-6α-8-*epi-*Dihydrocornin, 8-*epi*-Grandifloric acid, 7-*epi*-Loganin, 8-*epi*-Muralioside, *5,9-epi-*Penstemoside, 3-*epi*-Phlomurin, 1-*epi*-Shanzhigenin methyl ester, 8-*epi*-Tecomoside (7β-Hydroxyplantarenaloside), 7β ,8β-Epoxy-8α-dihydrogeniposide, 7,8-Epoxy-8-*epi*-loganic acid, 6β ,7β-Epoxy-8-*epi*-splendoside, Epoxygaertneroside, Epoxymethoxygaertneroside, Erinoside, 8-*O*-Feruloylharpagide, 7-*O*-*E*-Feruloylloganic acid, 7-*O*-*Z*-Feruloylloganic acid, 6'-*O*-*E*-Feruloylmonotropein, 10-*O*-*E*-Feruloylmonotropein, 6'-*O*-*trans*-Feruloylnegundoside, 6-*O*-α-L-(4"-*O*-*cis*-Feruloyl)-rhamnopyranosylcatalpol, 6'-*O*-Foliamenthoylmussaenosidic acid, 2'-*O*-Foliamenthoylplantarenaloside, Formosinoside, 10-*O*-β-D-Fructofuranosyltheviridoside, Gaertneric acid, Gaertneroside, 6-*O*-α-D-Galctopyranosylharpagoside, 6'-*O*-α-D-Galactopyranosylsyringopicroside, Gelsemiol-6'-*trans*-caffeoyl-1-glucoside, Globuloside A, Globuloside B, Globuloside C, 3'-*O*-β-D-Glucopyranosylcatalpol, 6-*O*-(4"-*O*-β-Glucopyranosyl)-*trans-p*-coumaroyl-8-*O-*acetylshanzhiside methyl ester, 6'-*O*-α-D-Glucopyranosylloganic acid, 3'-*O*-β-Glucopyranosylstilbericoside, 6'-*O*-α-D-Glucopyranosylsyringopicroside, 3'-*O*-β-D-Glucopyranosylsyringopicroside, 4'-*O*-β-D-Glucopyranosylsyringopicroside, 3'*-O*-β-D-Glucopyranosyltheviridoside, 6'-*O*-β-D-Glucopyranosyltheviridoside, 10-*O*-β-D-Glucopyranosyltheviridoside, 4"-*O*-Glucoside of linearoside (7-*O*-(4"-*O*-Glucosyl)-coumaroylloganic acid), Glucosylmentzefoliol, Gmelinoside A, Gmelinoside B, Gmelinoside C, Gmelinoside D, Gmelinoside E, Gmelinoside F, Gmelinoside G, Gmelinoside H, Gmelinoside I, Gmelinoside J, Gmelinoside K, Gmelinoside L, Gmephiloside *(1-O-*(8*-epi-*Loganoyl)-β-D-glucopyranose), Grandifloric acid, GSIR-1, Hookerioside, 6α-Hydroxyadoxoside, 6-*O*-*p*-Hydroxybenzoylasystasioside, 2'-O-p-Hydroxybenzoyl-6'-O-trans-caffeoyl-8-*epi*-loganic acid, 2'-*O-p*-Hydroxybenzoyl-6'-*O*-*trans*-caffeoylgardoside, 6'*-O-p-*Hydroxybenzoylcatalposide, 3-*O*-(4-Hydroxybenzoyl)-10-deoxyeucommiol-6-*O*-*p*-D-glucopyranoside, 2'-*O-p*-Hydroxybenzoyl-8-*epi*-loganic acid, 6'-*O-p*-Hydroxybenzoyl-8-*epi-*loganic acid, 2'-*O-p*-Hydroxybenzoylgardoside, 6-*O-p*-Hydroxybenzoylglntinoside, *7-O-p-*Hydroxybenzoylovatol-1-*O*-(6'-*O-p*-hydroxybenzoyl)-β-D-glucopyranoside, 8-*O*(-2-Hydroxycinnamoyl)harpagide, 5-Hydroxydavisioside, 10-Hydroxy-(5α-H)-6-*epi-*dihydrocornin, 1β-Hydroxy-4-*epi*-gardendiol, 6β-Hydroxy-7-*epi*-loganin, (5α-H)-6a-Hydroxy-8-*epi*-loganin, 7β-Hydroxy-11-methylforsythide, 6β-Hydroxygardoside methyl ester, 6α-Hydroxygeniposide, 4"-Hydroxy-E-globularinin, 7β-Hydroxyharpagide, 5-Hydroxyloganin, 7β-Hydroxyplantarenaloside, Humifusin A, Humifusin B, Inerminoside A, Inerminoside A1, Inerminoside B, Inerminoside C, Inerminoside D, Ipolamiidic acid, Iridoid dimer of asperuloside and asperulosidic acid, Iridolactone, Iridolinarin A, Iridolinarin B, Iridolinarin C, Iridolinaroside A, 6-*O*-Isoferuloyl ajugol, 10-*O*-*trans-*Isoferuloylcatalpol, Isosuspensolide E, Isosuspensolide F, Isounedoside, Isovibursinoside II, Isoviburtinoside III, Jashemsloside A, Jashemsloside B, Jashemsloside C, Jashemsloside D, Jashemsloside E (6"S-7-*O*-{6-*O*-[β-D-apiofuranosyl-(1→6)-β-D-glucopyranosyl]-menthiafolioyl}-loganin, Kansuenin, Kansuenoside, 7-Ketologanic acid, Kickxin, Lamiidic acid, Lantanoside, Linearoside (7-*O*-Coumaroylloganic acid), Lippioside I (6'*-O-p-trans-*Coumaroylcaryoptosidic acid), Lippioside II (6'-*O*-*trans-*Caffeoylcaryoptosidic acid), Loganic acid-6'-*O*-β-D-glucoside, Lupulinoside, Luzonoid A, Luzonoid B, Luzonoid C, Luzonoid D, Luzonoid E, Luzonoid F, Luzonoid G, Luzonoside A, Luzonoside B, Luzonoside C, Luzonoside D, Macedonine, Macrophylloside, 7-*O*-(6'-*O*-Malonyl)-cachinesidic acid (Malonic ester of 8-hydroxy-8-*epi*-loganic acid), Melittoside 3"-*O*-*p*-glucopyranoside, 5-*O-*Menthiafoloylkickxioside, 6'-*O*-Menthiafoloylmussaenosidic acid, Mentzefoliol, 6-*O*-(4-Methoxybenzoyl)-5,7-bisdeoxycynanchoside, 6-*m*-Methoxybenzoylcatalpol, 6-*O*-(4-Methoxybenzoyl)crescentin IV (3-*O*-β-D-glucopyranoside), 10-*O*-(4-Methoxybenzoyl)impetiginoside A, 7-*O*-(*p*-Methoxybenzoyl)-tecomoside, 6-*O-p*-Methoxy-*trans*-cinnamoyl-8-*O*-acetylshanzhiside methyl ester, 6-*O-p*-Methoxy-*cis*-cinnamoyl-8-*O-*acetylshanzhiside methyl ester, 10-*O*-*trans*-*p*-Methoxycinnamoylasystasioside E, 10*-O-cis-p-*Methoxycinnamoyl asystasioside E, 10-*O*-*cis*-*p*-Methoxycinnamoylcatalpol, 10-*O-trans-p-*Methoxycinnamoylcatalpol, 8-*O*-Z-*p*-Methoxycinnamoylharpagide, *6'-O-Z-p-*Methoxycinnamoylharpagide, 8-*O*-*E-p*-Methoxycinnamoylharpagide, *6'-O-Ep-*Methoxycinnamoylharpagide, 1β-Methoxy-4-*epi*-gardendiol, 1 β-Methoxy-4-*epi*-mussaenin A, 1α-Methoxy-4-*epi*-mussaenin A, Methoxygaertneroside, 1β-Methoxygardendiol, 4"-Methoxy-Z-globularimin, 4"-Methoxy-Z-globularinin, 4"-Methoxy-*E*-globularimin, 4"-Methoxy-*E*-globularinin, 6-*O*-[3-*O*-(*trans-p*-Methoxycinnamoyl)-α-L-rhamnopyranosyl]-aucubin, 1β-Methoxylmussaenin A, 6-*O*-Methyl-*epi*-aucubin, Muralioside (7β-Hydroxyharpagide), Myxopyroside, Nepetacilicioside, Nepetanudoside, Nepetanudoside B, Nepetanudoside C, Nepetanudoside D, Nepetaracemoside A, Nepetaracemoside B, (-) Ningpogenin (revision of 1-dehydroxy-3,4-dihydroaucubingenin), Officinosidic acid (5-Hydroxy-10-*O*-(*p*-methoxycinnamoyl)-adoxosidic acid), Ovatic acid methyl ester-7-*O*-(6'-*O*p-Hydroxybenzoyl)-β-D-glucopyranoside, Ovatolactone-7-*O*-(6'-*O-p*-hydroxybenzoyl)-β-D-glucopyranoside, 7-Oxocarpensioside, Paederoscandoside, Paederosidic acid methyl ester, Patrinioside, Pedicularis-lactone, Phlomiside, Phlomoidoside (6-*O*-(4"-*O-p*-Coumaroyl-β-D-xylopyranosyl)-aucubin), Phlomurin, Phlorigidoside A (2'-*O*-Acetyllamiridoside), Phlorigidoside B (8-*O*-Acetyl-6b-hydroxyipolamiide), Phlorigidoside C (5-Deoxysesamoside), Picconioside 1, Picroside IV, Picroside V (6-m-Methoxybenzoylcatalpol), Pikuroside, Plicatoside A, Plicatoside B, Premnaodoroside D, Premnaodoroside E, Premnaodoroside F [isomeric mixture of A and B in ratio (1 : 1)], Premnaodoroside G (isomeric mixture of (C) and (D)), Premnosidic acid, Proceroside (7-Oxocarpensioside), Randinoside, Saletpangponoside A [6-*O*-(4"-*O*-β-Glucopyranosyl)-*trans-p-*coumaroyl-8-*O-*acetylshanzhiside methyl ester], Saletpangponoside B, Saletpangponoside C, Sammangaoside C (Melittoside 3"-*O*-β-glucopyranoside), Saprosmoside A, Saprosmoside B, Saprosmoside C, Saprosmoside D, Saprosmoside E, Saprosmoside F, Saprosmoside G, Saprosmoside H, Scorodioside (6-*O*-(3"-*O*-Acetyl-2"-*O*-*trans*-cinnamoyl)-α-L-rhamnopyranosyl catalpol), Scrolepidoside, Scrophuloside A1, Scrophuloside A2, Scrophuloside A3, Scrophuloside A4, Scrophuloside A5, Scrophuloside A6, Scrophuloside A7, Scrophuloside A8, Scrophuloside B4 [6-*O*-(2"-*O*-Acetyl-3"-*O*-cinnamoyl-4"-*O-p*-methoxy cinnamoyl-α-L-rhamnopyranosyl)catalpol], Scrovalentinoside, Senburiside III, Senburiside IV, Serratoside A, Serratoside B, Shanzhigenin methyl ester, 6-*O*-Sinapoyl scandoside methyl ester, Sintenoside, Stegioside I, Stegioside II, Stegioside III, Syringafghanoside, 7,10,2",6"-Tetra-*O-*acetylisosuspensolide F, 7,10,2",3"-Tetra-*O*-acetylisosuspensolide F, 7,10,2",3"-Tetra-*O-*acetylsuspensolide F, Thunaloside, 7,10,2"-Tri-*O*-acetylpatrinoside, 7,10, 2"-Tri-*O-*acetylsuspensolide F, 6-*O*-α-L-(2"-*O*-,3"-*O*-,4"-*O*-Tribenzoyl)-rhamnopyranosylcatalpol, 6-*O-*(3",4",5"-Trimethoxybenzoyl)ajugol, Unbuloside (6-*O*-[(2"-*O*-*trans-*Feruloyl)-α-L-rhamnopyranosyl]-aucubin), Urphoside A, Urphoside B, Verbaspinoside (*6-O-*[(*2"-*O*-trans-*Cinnamoyl)-α-L-rhamnopyranosyl]-catalpol), Viburtinoside I, Viburtinoside II, Viburtinoside III, Viburtinoside IV, Viburtinoside V, Viteoid I, Viteoid II, Wulfenoside [(10-*O-*(Cinnamoylalpinosidyl)-6'-(desacetyl-alpinosidyl)-catalpol)], Yopaaoside A, Yopaaoside B, Yopaaoside C and Zaluzioside (6β-Hydroxygardoside methyl ester).

Further examples of compounds to be convereted by the polypeptide in the context of the present invention include the following compounds which have been disclosed by Dinda et al., Chem. Pharm. Bull. 55(5) (2007), 689-728 including any aglycons, in particular those where at least one glycosyl residue has been removed, thereof:
Acerifolioside, 10-Acetoxyoleosidedimethyl ester, 7-O-Acetylabelioside, 6'-*O*-Acetyl-10-acetoxyoleoside dimethyl ester, 2'-*O*-Acetyl-4'-*O*-*cis-p*-coumaroylswertiamarin, 2'-*O*-Acetyl-4'-*O*-*cis*-feruloylswertiamarin, 6'-*O*-Acetyldiderroside, 7-*O*-Acetyllaciniatoside IV, 7-*O-*Acetyllaciniatoside V, 2'-*O*-Acetyl-4'-*O*-*trans-p*-coumaroylswertiamarin, 2'-*O*-Acetyl-4'-*O-trans*-feruloylswertiamarin, Adinoside A, Adinoside B, Adinoside C, Adinoside D, Adinoside E, Alboside IV, (Z)-Aldosecologanin, Angustifoliside C, 6'-*O*-β-Apiofuranosylsweroside, Asaolaside, Austrosmoside, Butyl isoligustrosidate, Caeruleoside A, Caeruleoside B, Caeruleoside C, 3'-*O*-Caffeoylsweroside, Cantleyoside-dimethyl-acetal, Chelonanthoside, 6'-*O*-*cis-p*-Coumaroyl-8-*epi*-kingiside, 10-*cis*-(*p*-Coumaroyloxy)oleoside dimethyl ester, 6'-*O-trans-p-*Coumaroyl-8-*epi*-kingiside, 10-*trans*-(*p*-Coumaroyloxy)oleoside dimethyl ester, 4'-*O-trans-p*-Coumaroylswertiamarin, Depressine, Depresteroside, Desrhamnosyloleoacteoside, Dihydrochelonanthoside, Diderroside methyl ester, 6'-*O*-(2,3-Dihydroxybenzoyl)sweroside, 6'-*O*-(2,3-Dihydroxybenzoyl)swertiamarin, 2"-*epi*-Frameroside, Fraxicarboside A, Fraxicarboside B, Fraxicarboside C, Fraximalacoside, Gentiascabraside A, Gentiotrifloroside, 6'-Gentisoyl-8-*epi*-kingiside, 2'-Gentisoylgelidoside, 7β-[(*E*)-4'*O*-(β-D-Glucopyranosyl)caffeoyloxy]sweroside, 3"-Glucosyldepresteroside, 1-*O*-β-D-Glucopyranosyl-4-*epi*-amplexine, 6'-*O*-β-D-Glucopyranosyl morroniside, 6"-*O*-β-D-Glucopyranosyloleuropein, 7-*O*-(4-β-D-Glucopyranosyloxy-3-methoxybenzoyl)-secologanolic acid, 6""-*O*-Glucosyltrifloroside, Gonocaryoside A, Gonocaryoside B, Gonocaryoside C, Gonocaryoside D, Gonocaryoside E, Grandifloroside-11-methyl ester, Hydrachoside A, Hydramacroside A, Hydramacroside B, Hydroxyframoside A, Hydroxyframoside B, 8-Hydroxy-10-hydrosweroside, 6β-Hydroxyswertiajaposide A, 6'-*O-*(7α-Hydroxyswerosyloxy)-loganin, Insularoside-3',4‴-di-*O*-β-D-glucoside, Insularoside-3'-*O-*β-D-glucoside, Insuloside, Isojasminoside, Isojaspolyoside A, Isojaspolyoside B, Isojaspolyoside C, Isomacrophylloside, Isooleoacteoside, Jaslanceoside A, Jaslanceoside B, Jaslanceoside C, Jaslanceoside D, Jaslanceoside E, Jasnudifloside A (Jasuroside A?), Jasnudifloside B (Jasuroside B?), Jasnudifloside C, Jasnudifloside D, Jasnudifloside E, Jasnudifloside H, Jasnudifloside L, Jasnudifloside F, Jasnudifloside G, Jasnudifloside I, Jasnudifloside J, Jasnudifloside K, Jaspofoliamoside A, Jaspofoliamoside B, Jaspolinaloside, Jaspolyanoside, Jaspolyanthoside, Jaspolyoleoside A, Jaspolyoleoside B, Jaspolyoleoside C, Jaspolyoside, Jaspolyside, Jasuroside A, Jasuroside B, Jasuroside C, Jasuroside D, Jasuroside E, Jasuroside F, Jasuroside G, Korolkoside, (8Z)-Ligstroside, Loasafolioside, Loniceracetalide A, Loniceracetalide B, Macrophylloside A, Macrophylloside B, Molihuaside A, Molihuaside B, Molihuaside C, Molihuaside D, Molihuaside E, Neopolyanoside, Nudifloside A, Nudifloside B, Nudifloside C, Nudifloside D, (8*Z*)-Nüzhenide, Oleopolyanthoside A, Oleopolyanthoside B, L-Phenylalaninosecologanin, Polyanoside, Rapulaside A, Rapulaside B, Rhodanthoside B, Rhodanthoside C, Safghanoside A, Safghanoside B, Safghanoside C, Safghanoside D, Safghanoside E, Safghanoside F, Safghanoside G, Safghanoside H, Scabran G3, Scabran G4, Scabran G5, Secologanoside-7-methyl ester, Secostrychnosin, Strychoside A, Strychoside B, Stryspinoside, 8-*O*-Tigloyldeacetyldiderroside and Tricoloroside methyl ester.

Other examples of compounds to be converted by the polypeptide in the context of the present invention include the following compounds which have been disclosed by Dinda et al., Chem. Pharm. Bull. 57(8) (2009), 765-796 including any aglycons, in particular those where at least one glycosyl residue has been removed, thereof:
6-*O*-Acetylantirrinoside, 8-*O*-Acetylharpagide-6-*O*-β-glucoside, Alstonoside, Axillaroside, 10-*O*-Benzoyl-10-*O*-deacetylasperuloside, 10-*O*-Benzoyl-10-*O*-deacetyldaphylloside, 10-*O-*Benzoyl-10-*O*-deacetyl-11-demethoxy-11-ethoxydaphylloside, 6-*O*-(3"-*O*-Benzoyl) α-L-rhamnopyranosylcatalpol, 6-*O*-Caffeoylharpagide, 6'-*O*-[(*E*)-Caffeoyl]-deacetylasperulosidic acid methyl ester, 6-*O*-*E*-Cinnamoyl-*E*-harpagoside, 6-*O*-(3"-*O*-*E*-Cinnamoyl)-α-L-rhamnopyranosylcatalpol, 6-*O*-(3"-*O*-*Z*-Cinnamoyl)-α-L-rhamnopyranosylcatalpol, Citrifoside, 5-Deoxy-8-deacetyl-3-*O*-methyl-6-*O-p*-hydroxybenzoyl-1,3-di-*epi-*clandonensine, 5-Deoxy-8-deacetyl-3-*O*-methyl-6-*O-p*-hydroxybenzoyl-1-*epi*-clandonensine, 10-*O*-(*E*)-*p*-Coumaroyl deacetylasperulosidic acid, 10-*O*-(*Z*)-*p*-Coumaroyl desacetylasperulosidic acid, 10-*O*-Coumaroyl-10-*O*-deacetylasperuloside, *10-O-p-*Coumaroyl-10-*O*-deacetyldaphylloside, 10-*O*-*p*-Coumaroyl-10-*O*-deacetyl-11-demethoxy-11-ethoxydaphylloside, 10-(*E*)-*p*-Coumaroyl-6,7-dihydromonotropein, 10-(*Z*)-*p*-Coumaroyl-6,7-dihydromonotropein, 6'-*O*-*E-p*-Coumaroyl-3,4-dihydrocatalpol (Piscroside B), 6'*-O-*(*E*)-*p-*Coumaroyl-8-*epi*-loganic acid, 10-*O-*(*E*)-*p*-Coumaroylscandoside, *10-O-*(*Z*)*-p-*Coumaroylscandoside, 6'-*O*-*p*-Coumaroyl-8-epikingiside, 6'-*O*-*E*-*p*-Coumaroylgardoside, 4"-*O*-[(*E*)-*p*-Coumaroyl]-gentiobiosylgenipin, 6'-*O*-Cyclopropanoyltheviridoside, Daphmacropodosidine A, Daphmacropodosidine B, 6,7-Dehydro-8-acetylharpagide, 5-Dehydro-*8*-*epi*-adoxosidic acid, 7,8-Dehydroharpagide, 5-Dehydro-8-*epi*-mussaenoside, 11-Demethoxy-11-ethoxydaphylloside, 7-Deoxyloganic acid-β-D-glucopyranosyl ester, 8-Deoxyshanzhiside, 6,8-Diacetylharpagide, 6,8-Diacetylharpagide-1-*O*-β-(2',3'-di-*O-*acetylglucoside), 1,5-Dihydroxy-3,8-epoxyvalechlorine A, 10-*O*-Dihydroferuloyl-10-*O-*deacetyldaphylloside, Dipsanoside A, Dipsanoside B, Dipsanoside C, Dipsanoside D, Dipsanoside E, Dipsanoside F, Dipsanoside G, Eucomoside A, Eucomoside B, Eucomoside C, 6-*epi*-Barlerin, 13(*R*)-*epi*-Epoxygaertneroside, 13(*R*)-*epi*-Gaertneroside, Eriobioside (Ipolamiide 6'-*O*-β-glucopyranoside), 6'-*O*-Feruloylharpagide, 6'-*O*-α-D-Galactopyranosylbarlerin, 6'-*O*-α-D-Galactopyranosylphlorigidoside C, 6-*O*-α-D-Galactopyranosylshanzhiside methyl ester, 6'-*O*-α-D-Galactopyranosylsesamoside, Gardaloside, Genameside A, Genameside B, Genameside C, Genameside D, Genestifolioside, Genipin-1-*O*-α-L-rhamnopyranosyl(1→6)-β-D-glucopyranoside, Globularioside, 6'-*O*-β-D-Glucopyranosylbarlerin, 6'-*O*-Glucopyranosylharpagoside, 5-*O*-β-Glucopyranosylharpagide, 6'-*O*-β-D-Glucopyranosylphlorigidoside C, 6'-*O*-β*-*Glucopyranosylsecologanoside, 6'-*O*-β-D-Glucopyranosylsecologanol, 6'-*O*-β-D-Glucopyranosylsesamoside, 3'-*O*-β-D-Glucopyranosylsweroside, Harpagide 6-*O*-β-glucoside, Harprocumbide A (6'-*O*-α-D-Galactopyranosyl-harpagoside), Harprocumbide B (6'-*O*-(*cis-p*-Coumaroylprocumbide)), Hedycoryside A, Hedycoryside B, Hedycoryside C, 6'-*O-p*-Hydroxybenzoylantirrinoside, 2'-*O*-(3"-Hydroxybenzoyl)-8-epikingiside, 2'-*O*-(3"-Hydroxybenzoyl)-kingiside, 10*-O-p-*Hydroxybenzoyltheveridoside, 6β-Hydroxy-7-epigardoside methyl ester, 6β-Hydroxy-2-oxabicyclo[4.3.0]non-8-en-1-one, Incaside, Isoplumericin, 10-Isovalerylkanokoside C, Kankanol, Kankanoside A, Kankanoside B, Kankanoside C, Kankanoside D, 6-Keto-8-acetylharpagide, Lamalbidic acid, Lamerioside, Loganic acid 11-*O*-β-glucopyranosyl ester, Marinoid A, Marinoid B, Marinoid C, Marinoid D, Marinoid E, 2'-*O*-(4-Methoxycinnamoyl)mussaenosidic acid, 8-Methylvalepotriate, Morindacin, Myobontioside A, Myobontioside B, Myopochlorin, Paederoside B, Pensteminoside, 1β ,6β ,7α ,8α ,10-Pentahydroxy-*cis*-2-oxabicyclo[4.3.0]nonane, 2'-*O*-[(2*E*,4*E*)-5-Phenylpenta-2,4-dienoyl]mussaenosidic acid, Picroroside A, Picroroside B, Picroroside C, Piscrocin D, Piscrocin E, Piscrocin F, Piscrocin G, Piscroside A (7-Deoxy-7-α-Cl-pikuroside), Piscroside B (6'-*O*-*E*-Cinnamoyl-3β-methoxy-3,4-dihydro-catalpol), Plumericin, Prismatomerin, Rupesin A, Rupesin B, Rupesin C, Rupesin D, Rupesin E, Scutelloside, Scyphiphorin A (10-*O-*Benzoylgeniposidic acid), Scyphiphorin B, Shanzhilactone, 6'-*O*-[(*E*)-Sinapoyl]gardoside, 6'-*O*-Sinapoylgeniposide, Stachysoside E (3"-*O-p*-Coumaroyl ester of melittoside), Stachysoside F, Stachysoside G, Stachysoside H, Stereospermoside (6-*O*-*trans*-*p*-Coumaroyl-decinnamoyl globularimin), Tarennin (artifact), Tarenninoside A, Tarenninoside B, Tarenninoside C, Tarenninoside D, Tarenninoside E, Tarenninoside F, Tarenninoside G, Teneoside A (10-*O*-(α-L-Rhamno) deacetylasperuloside), Teneoside B, 6β ,7β ,8α ,10-Tetrahydroxy-*cis*-2-oxabicyclo[4.3.0]nonan-3-one, 6β ,7β ,8α ,10-Tetra-*p*-hydroxybenzoyl-*cis*-2-oxabicyclo[4.3.0]nonan-3-one, Tudoside, (*E*)-Uenfoside, (*Z*)-Uenfoside, Valeriotetrate C, Valeriotriate A, Valeriotriate B, 10-*O*-Vanilloyltheviridoside, 6'-*O*-Vanilloyl-3,4-dihydrocatalpol (Piscroside A), Verbenabraside A, Verbenabraside B, Versibirioside, Viburtinoside A, Viburtinoside B and Wendoside.

Additional examples of the compound to be converted by the polypeptide in the context of the present invention include the following compounds which have been disclosed by Dinda et al., Chem. Pharm. Bull. 59(7) (2011), 803-833 including any aglycons, in particular those where at least one glycosyl residue has been removed, thereof:
8-*O*-Acetylharpagide, 6'-*O*-Acetylgeniposide, 6'-*O*-Acetyl-3"-methoxygaertneroside, 10-*O-*Acetylpatrinoside-aglycone-11-*O*-[4"-*O*-acetyl-α-L-rhamnopyranosyl-(1→2)-β-D-glucopyranoside], 7-*O*-Acetylpatrinoside-aglycone-11-*O*-[4"-*O*-acetyl-α-L-rhamnopyranosyl-(1→2)-β-D-ribohexo-3-ulopyranoside], 10-*O*-Acetylpatrinoside-aglycone-11-*O*-[4"-*O*-acetyl-α-L-rhamnopyranosyl-(1→2)-β-D-ribohexo-3-ulopyranoside], 10-*O*-Acetylpatrinoside-aglycone-11-*O*-[α-L-rhamnopyranosyl-(1→2)-β-Dribohexo-3-ulopyranoside], Acevaltrate, Adenosmoside, Alatenoside (C-7α-OH epimer), Alatinoside (C-7β-OH epimer), Allamanoid, Amphicoside, Asperulosidic acid, Aucubin, Buddlejosides B, A5 and A2, 2'-*O-*Caffeoylloganic acid, 7-*epi*-7-*O*-(*E*)-Caffeoylloganic acid, Catalpol, Catarractoside (=6-*O-*Rhamnopyranosylasystasioside *E*), 6"-*O*-*trans*-Cinnamoylgenipin gentiobioside, 6'-*O-*Cinnamoylharpagide, 6-*O*-[3"-(*E*)-Cinnamoyl-α-L-rhamnopyranosyl]-glutinoside, Cocculoside, 6-*O*-*cis-p*-Coumaroylcatalpol, 6"-*O*-*trans*-*p*-Coumaroylgenipin gentiobioside, 6'-*O*-*trans*-*p*-Coumaroylgeniposide, 6'-*O*-*trans*-*p*-Coumaroylgeniposidic acid, 2*'-O-E*-*p-*Coumaroylloganic acid, 2'-*O*-*Z*-*p*-Coumaroylloganic acid, 10-*O*-(*E*)-*p*-Coumaroylmelittoside, 15-Demethylisoplumieride, Derwentioside A (=6-*O*-Benzoylaucubin), Derwentioside B (=6-*O*-*p*-Hydroxybenzoylaucubin), Derwentioside C (=6-*O*-Vanilloylaucubin), Didrovaltrate acetoxyhydrin, Diffusoside A, Diffusoside B, (1*R*)-1,5-Dihydroxy-3,8-epoxyvalechlorine (revised structure), 6'-(3,4-Dimethoxycinnamoyl)-ajugol, 6'-(3,4-Dimethoxycinnamoyl)-harpagide, Dunalianoside I, 8-Epiloganic acid, (1*R*)-3,8-Epoxy-1-*O*-ethyl-5-hydroxyvalechlorine (artifact), (1*S*)-3,8-Epoxy-1-*O*-ethyl-5-hydroxyvalechlorine (artifact), Eurostoside, Excelside A, Excelside B, Fagraldehyde, Floribundane A, Floribundane B, GEIR-1, GEIR-2, GEIR-3, Genipin, Geniposidic acid, GI-3, GI-5, Glucologanin, 2'-*O*-β-D-Glucopyranosyl-6'-*O*-(*p*-methoxycinnamoyl)-harpagide, 6β-*O*-β-D-Glucosylpaederosidic acid, Griffithoside A, Griffithoside B, Griffithoside C, GRIR-1, 5-Hydroxydidrovaltrate, 2'-*O-p*-Hydroxylbenzoylloganic acid, (7"*R*)-10-Hydroxy-7"-methoxyoleuropein, (7"*S*)-10-Hydroxy-7"-methoxyoleuropein, Incarvillic acid, Isoplumericin, (IR)-1-Isopropyloxygenipin, Jatamanin A, Jatamanin B, Jatamanin C, Jatamanin D, Jatamanin E, Jatamanin F, Jatamanin G, Jatamanin H, Jatamanin I, Jatamanin J, Jatamanin K, Jatamanin L, Jatamanin M, Jatamanvaltrate A, Jatamanvaltrate B, Jatamanvaltrate C, Jatamanvaltrate D, Jatamanvaltrate E, Jatamanvaltrate F, Jatamanvaltrate G, Jatamanvaltrate H, Jatamanvaltrate I, Jatamanvaltrate J, Jatamanvaltrate K, Jatamanvaltrate L, Jatamanvaltrate M, Javanicoside A, Javanicoside B, Kankanoside A, Kankanoside L, Kankanoside M, Kankanoside N, Kutkoside, Lamalbid, Lamiridosin A, Lamiridosin B, Ligustrohemiacetal A (revised structure of jasmolactone E), Ligustrohemiacetal B (revised structure of jasmolactone A), Loganic acid 1, Loganin, Longifolioside A, Longifolioside B, Lonijaposide A, Lonijaposide B, Lonijaposide C, 6'-*O*-(*p*-Methoxycinnamoyl)-harpagide, *E*-*6*-*O*-*p*-Methoxycinnamoyl scandoside methyl ester, 9-*epi*-6α-Methoxygeniposidic acid, Minecoside, Morroniside, Mussaenosidic acid, Muzhenide, Naucledal, *cis*-Nepetalactone, *trans*-Nepetalactone, Oleoside dimethyl ester, Oreosolenoside, Patrinoside-aglycone-11-*O*-[4"-*O*-acetyl-α-L-rhamnopyranosyl-(1→2)-β-D-ribohexo-3-ulopyranoside], Patrinoside-aglycone-11-*O*-2'-deoxy-β-glucopyranoside, Patriscadoid I, Patriscadoid II, Phloyoside1, Phlomiol, Picogentioside I, Picroside1, Picroside II, Pirosecoside I, Pirosecoside II, Plumericin, Pulchelloside 1, Safghanoside D, Shanzhiside, Shanzhiside methyl ester, Sanshiside D, Scandoside methyl ester, Scyphiphin A1, Scyphiphin A2, Scyphiphin B1, Scyphiphin B2, 6"-*O*-*trans*-Sinapoylgenipin gentiobioside, 10-(6-*O-trans*-Sinapoylglucopyranosyl)gardendiol, 11-(6-*O*-*trans*-Sinapoylglucopyranosyl)-gardendiol, Specioside, 10-*O*-Succinoylgeniposide, Swerilactone A, Swerilactone B, Swerilactoside A, Swerilactoside B, Swerilactoside C, Swertiamarin, Syrveoside A (=6'-*O-*(6,7-Dihydrofoliamenthoyl)-8-*epi*-kingisidic acid), Syrveoside B (=6'-*O*-(Menthiafoloyl)-8-*epi*-kingisidic acid), 2',3',6'-Tri-*O*-acetyl-4'-*O*-*trans*-*p*-(*O*-β-D-glucopyranosyl)-coumaroyl-7-ketologanin, Triohima A, Triohima B, Triohima C, Valeriotetrate C, Valtrate, IVHD-valtrate 1, 10-*O*-Vanilloylgeniposidic acid, Verbenalin, Verminoside, Versibirioside (6-*O*-[3"-(*E*)-Cinnamoyl-α-L-rhamnopyranosyl]-glutinoside), Volvaltrate A and Volvaltrate B.

As shown in the Examples of the present disclosure, the present disclosure provides a polypeptide having esterase activity and which is characterized in that is capable of converting compounds corresponding to the general formula C₂₅H₃₂Oₙ, with n ≥ 12, into a compound corresponding to the general formula C₂₅H₃₂Oₙ₋₇. In particular, the present disclosure provides a corresponding esterase of wherein,
(i) the compound corresponding to the general formula C₂₅H₃₂Oₙ, with n ≥ 12, is ligstroside (C₂₅H₃₂O₁₂), preferably an aglycone thereof, and the compound corresponding to the general formula C₂₅H₃₂Oₙ₋₇ is oleocanthal (C₂₅H₃₂O₅); or
(ii) the compound corresponding to the general formula C₂₅H₃₂Oₙ, with n ≥ 12, is oleuropein (C₂₅H₃₂O₁₃), preferably an aglycone thereof, and the compound corresponding to the general formula C₂₅H₃₂Oₙ₋₇ is oleacein (C₂₅H₃₂O₆).

The polypeptide having an esterase activity disclosed herein relates to an enzyme which is chacaterized in that is capable of converting oleoside-11-methylester into decarboxymethyl elenolic acid dialdehyde (DEDA) (as demonstrated in the appended Examples by the conversion of elenolic acid, i.e. the aglycone of oleoside-11-methylester into decarboxymethyl elenolic acid dialdehyde (DEDA)).

Accordingly, in the context of the disclosure which is not part of the invention, a polypeptide having esterase activity is characterized in that is capable of converting
(i) ligstroside, preferably a ligstroside aglycone, into oleocanthal;
(ii) oleuropein, preferably an oleuropein aglycone, into oleacein; and/or
(iii) oleoside-11-methylester, preferably elenolic acid, into decarboxymethyl elenolic acid dialdehyde (DEDA).

Thus, compounds to be converted can in particular be ligstroside, and/or a ligstroside aglycone (leading to oleocanthal) and/or oleuropein, and/or a oleuropein aglycone (leading to oleacein), and/or oleoside-11-methylester, and/or elenolic acid (leading to decarboxymethyl elenolic acid dialdehyde (DEDA)). Most preferred compounds to be converted are ligstroside aglycone and/or oleuropein aglycone and/or elenolic acid.

The term "esterase" refers to an enzyme which can catalyze the cleavage of an ester from a compound/substrate as defined herein above and which is preferably selected from the iridoids and seco-iridoids, more preferably a compound of the above defined formula (I) or any preferred embodiments thereof. Even more preferably, in the context of the present invention, the term "esterase" refers to an enzyme which can convert the compound/substrate such as oleuropein, oleuropein aglycone, ligstroside, ligstroside aglycone, nüzhenide, oleoside-11-methylester, elenolic acid, safghanoside, oleuroside, secologanin, adinoside, geniposide and/or loganin. More precisely, as shown in the appended Examples, the provided polypeptide having esterase activity, i.e. the esterase in the context of the present invention, shows the ability to convert compounds corresponding to the general formula C₂₅H₃₂Oₙ, with n ≥ 12 into a compound corresponding to the general formula C₂₅H₃₂Oₙ₋₇, such as preferably ligstroside (C₂₅H₃₂O₁₂), more preferably a ligstroside aglycone, into oleocanthal (C₂₅H₃₂O₅), or oleuropein (C₂₅H₃₂O₁₃), more preferably an oleuropein aglycone, into oleacein (C₂₅H₃₂O₆). In addition, as shown in the appended Examples, the esterase in the context of the present invention shows the ability to convert oleoside-11-methylester, preferably elenolic acid (C₁₁H₁₄O₆), into decarboxymethyl elenolic acid dialdehylde (DEDA). This esterase activity can be determined by the skilled person by using methods well established in the art. Methods for determining the esterase activity are known in the art and described in the Examples in more detail and include, e.g., HPLC measurement.

Accordingly, in the context of the disclosure which is not part of the invention, the esterase relates to a polypeptide/enzyme selected from the group consisting of:
(a) a polypeptide comprising the amino acid sequence of SEQ ID NO: 7;
(b) a polypeptide that is at least 60%, 61%, 62%, 63%, 64%, 65%, 66%, 67%, 68%, 69%, 69.3%, 70%, 71%, 71.4%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to the amino acid sequence of SEQ ID NO: 7 and which is characterized by having an esterase activity;
(c) a polypeptide encoded by a nucleic acid molecule which is at least 60%, 61%, 62%, 63%, 64%, 65%, 66%, 67%, 68%, 69%, 69.3%, 70%, 71%, 71.4%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to the nucleic acid sequence of SEQ ID NO: 5 or 6 and wherein said polypeptide is characterized by having an esterase activity;
(d) a polypeptide encoded by the nucleic acid molecule of SEQ ID NO: 5 or 6; and
(e) a polypeptide encoded by the complementary sequence of a nucleic acid molecule that is able to hybridize with the nucleic acid molecule of SEQ ID NO: 5 or 6 and wherein said polypeptide is characterized by having an esterase activity.

Further, described as not part of the invention are polypeptides having an esterase activity which are at least 60% identical to the amino acid sequence of SEQ ID NO: 7 (OeEst1). Thus, the present disclosure also relates to polypeptides having esterase activity which comprises/consists of the amino acid sequence of SEQ ID NO: 24 or SEQ ID NO: 37. The polypeptide with the amino acid sequence of SEQ ID NO: 24 is a polypeptide having an esterase activity which is 69.3% identical to SEQ ID NO: 7. Further, the protein sequence of SEQ ID NO: 37 (as another example for a polypeptide having esterase activity which is at least 60% identical to the amino acid sequence of SEQ ID NO: 7) is 71.4% identical to the amino acid sequence of SEQ ID NO: 7. As shown in the appended Examples, both polypeptides (named OeEst030 (SEQ ID NO: 24) and OeEst679 (SEQ ID NO: 37)) are enzymes having an esterase activity. In particular, the polypeptides with the amino acid sequence of SEQ ID NO: 24 or 37 are also enzymes which preferably convert ligstroside, and/or a ligstroside aglycone (leading to oleocanthal) and/or oleuropein, and/or a oleuropein aglycone (leading to oleacein), and/or oleoside-11-methylester, and/or elenolic acid (leading to decarboxymethyl elenolic acid dialdehyde (DEDA)).

In the context of the disclosure which is not part of the invention, the polypeptide is characterized by having the amino acid sequence shown in SEQ ID NO: 7 or a related sequence (at least 60% identical) which show the ability to convert compounds as defined herein and which are preferably selected from the iridoids and seco-iridoids, more preferably (a) compound(s)/substrate(s) of the above defined formula (I) or any preferred embodiments thereof. Even more preferably in the context of the present invention, the polypeptide is characterized by having the amino acid sequence shown in SEQ ID NO: 7 or a related sequence (at least 60% identical) which show the ability to convert compounds corresponding to the general formula C₂₅H₃₂Oₙ, with n ≥ 12 into a compound corresponding to the general formula C₂₅H₃₂Oₙ₋₇, such as preferably ligstroside (C₂₅H₃₂O₁₂), more preferably a ligstroside aglycone, into oleocanthal (C₂₅H₃₂O₅), or oleuropein (C₂₅H₃₂O₁₃), more preferably an oleuropein aglycone, into oleacein (C₂₅H₃₂O₆), and/or oleoside-11-methylester, preferably eleonic acid (C₁₁H₁₄O₆), into decarboxymethyl elenolic acid dialdehyde (DEDA). In the context of the present invention the polypeptide of the present invention may be derived from a sequence which shows at least 60% sequence identity and in which one or more substitutions and/or deletions and/or insertions in the amino acid sequences shown in SEQ ID NO: 7 have been effected. However, the teaching of the present invention is not restricted to the polypeptide shown in SEQ ID NO: 7 which had been used in the appended Examples as a model enzyme but can be extended to polypeptides from other organisms, in particular to other esterases, or to enzymes which are structurally related to SEQ ID NO: 7 such as, e.g., truncated variants of the polypeptide that has the amino acid sequence shown in SEQ ID NO: 7. Thus, the present disclosure also relates to variants of the polypeptide that comprises/consists of the amino acid sequence of SEQ ID NO: 7 which are characterized by having esterase activity. In the context of the present invention, the term "structurally related" refers to polypeptides, preferably esterases, which show a sequence identity of at least n% to the sequence shown in SEQ ID NO: 7 with n being an integer between 69 and 100, preferably 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99. In the context of the present invention, the structurally related polypeptides having esterase activity are of eukaryotic origin, even more preferably they stems from a plant, most preferably of a plant belonging to the family *Oleacea* such as a plant belonging to the genus *Olea* or *Liguster.*

In the context of the present invention, "substituted" or "substitution" means that the respective amino acid sequence can be substituted within the amino acid sequence as shown in SEQ ID NO: 7 with any other possible amino acid residues, e.g. naturally occurring amino acids or non-naturally occurring amino acids (Brustad and Arnold, Gurr. Opin. Chem. Biol. 15 (2011), 201-210), preferably with an amino acid residues selected from the group consisting of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine. Moreover, the term "subsituted" or "substitution" also means that one or more respective amino acid residue(s) within the amino acid sequence of SEQ ID NO: 7 is modified. Such modifications include naturally occurring modifications and non-naturally occurring modifications. Naturally occurring modifications include but are not limited to eukaryotic post-translational modifications, such as attachment of functional groups (e.g. acetate, phosphate, hydroxyl, lipids (myristoylation of glycine residues) and carbohydrates (e.g. glycosylation of arginine, asparagines etc.). Naturally occurring modifications also encompass the change in the chemical structure by citrullination, carbamylation and disulphide bond formation between cysteine residues; attachment of co-factors (FMN or FAD that can be covalently attached) or the attachement of peptides (e.g. ubiquitination or sumoylation). Non-naturally occurring modifications include, e.g., in vitro modifications such as biotinylation of lysine residue or the inclusion of non-canonical amino acids (see Liu and Schultz, Annu. Rev. Biochem. 79 (2010), 413-44 and Wang et al., Chem. Bio. 2009 March 27; 16 (3), 323-336; doi:101016/jchembiol.2009.03.001).

In the context of the present invention, "deleted" or "deletion" means that the amino acid at the corresponding position is deleted.

In the context of the present invention, "inserted" or "insertion" means that at the respective position one or two, preferably one amino acid residue is inserted in the amino acid sequence as shown in SEQ ID NO: 7.

Thus, in the context of the present invention the variant of the polypeptide of the present invention characterized by having an esterase activity has or is preferably derived from a sequence which is at least n% identical to SEQ ID NO: 7 which n being an integer between 69 and 100, preferably 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99. In the context of the present invention, the provided poypeptide is at least 60%, 61%, 62%, 63%, 64%, 65%, 66%, 67%, 68%, 69%, 69.3% 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to the amino acid sequence of SEQ ID NO: 7 and characterized by having an esterase activity. Further, in the context of the present invention, the provided polypeptide characterized by having an esterase activity has or is preferably derived from a sequence which is at least n% identical to SEQ ID NO: 7 which n being an integer between 69 and 100, preferably 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99, and it has, preferably, (a) substitution(s) and/or (a) deletion(s) and/or (an) insertion(s) at a position within the amino acid sequence shown in SEQ ID NO: 7.

In accordance with the present invention, the term at least n% identical to in connection with an amino acid/polypeptide sequence describes the number of matches ("hits") of identical nucleic acids/amino acid residues of two or more amino acid sequences as compared to the number of amino acid residues making up the overall length of the amino acid sequences (or the overall compared part thereof). In other terms, using an alignment, for two or more sequences or subsequences, the percentage of amino acid residues or nucleic acid residues that are the same (e.g. at least 69%, 69.3%, 70%, 71%, 71.4%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity) may be determined, when the (sub)sequences are compared and aligned for maximum correspondence over a window of comparison, or over a designated region as measured using a sequence comparison algorithm as known in the art, or when manually aligned and visually inspected.

As regards the determination of sequence identity, the following should apply: When the sequences which are compared do not have the same length, the degree of identity either refers to the percentage of amino acid residues in the shorter sequence which are identical to amino acid residues in the longer sequence which are identical to amino acid residues in the shorther sequence. Preferably, it refers to the percentage of amino acid residues in the shorter sequence which are identical to amino acid residues in the longer sequence. The degree of sequence identity can be determined according to methods well known in the art using preferably suitable computer algorithms such as CLUSTAL. When using the Clustal analysis method to determine whether a particular sequence is, for instance, at least 60% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0. In the context of the present invention, ClustalW2 is preferably used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix: BLOSUM 62; gap open: 10; gap extension: 0.2; gap distance: 5; no end gap. Preferably, the degree of identity is calculated over the complete length of the sequence.

An esterase of the present invention can be fused to a homologous or heterologous polypeptide or protein, an enzyme, a substrate or a tag to form a fusion protein. Fusion proteins in accordance with the present invention will have the same improved activity as the esterase of the present invention. Polypeptides, enzymes, substrates or tags that can be added to another protein are known in the art. They may useful for purifying or detecting the proteins of the invention. For instance, tags that can be used for detection and/or purification are e.g. thiorededoxin-tag, FLAG-tag, His6-tag or a Strep-tag. In the context of the present invention the thioredoxin-tag can be pet-32a(+) thioredoxin fusion protein which comprise or consist of SEQ ID NO: 9 (as encoded by the nucleic acid molecule shown in SEQ ID NO: 8) or a truncated variant thereof. Illustratively, as shown in the appended Examples of the present invention the esterase which can be used according to the methods disclosed herein, which can be carried out in vitro and in vivo, comprises or consists of the amino acid sequence shown in SEQ ID NO: 11 (as encoded by the nucleic acid molecule shown in SEQ ID NO: 10), SEQ ID NO: 26 (as encoded by the nucleic acid molecule shown in SEQ ID NO: 25), or SEQ ID NO: 39 (as encoded by the nucleic acid molecule shown in SEQ ID NO: 38). Alternatively, the protein of the invention can be fused to an enzyme e.g. luciferase, for the detection or localisation of said protein. Other fusion partners include, but are not limited to, bacterial β-galactosidase, trpE, Protein A, β-lactamase, alpha amylase, alcohol dehydrogenase or yeast alpha mating factor. It is also conceivable that the polypeptide, enzyme, substrate or tag is removed from the protein of the invention after e.g. purification. Fusion proteins can typically be made by either recombinant nucleic acid methods or by synthetic polypeptide methods known in art.

Excluded from the present invention is any polypeptide selected from the group consisting of:
(i) a polypeptide comprising the amino acid sequence of SEQ ID NO: 49 or 60;
(ii) a polypeptide encoded by the nucleic acid molecule of SEQ ID NO: 48, 58 or 59. Said polypeptides do convert for example para-nitrophenyl acetate, para-nitrophenyl butyrate, methyl jasmonate, methyl salicylate, and methyl indole-3-acetate, but do not convert ligstroside aglycone and/or oleuropein aglycone and/or elenolic acid.

Furthermore, excluded is any polypeptide selected from the group consisting of:
(iii) a polypeptide comprising the amino acid sequence of SEQ ID NO: 70, or 71;
(iv) a polypeptide encoded by the nucleic acid molecule of SEQ ID NO: 69.

In the context of the present invention a polypeptide is disclosed that comprises the amino acid sequence of SEQ ID NO: 24 or 37. Such polypeptide can also be encoded by any of the nucleic acid molecules of SEQ ID NO: 22, 23, 35 or 36.

Thus, in the context of the present invention, a polypeptide is disclosed that comprises the amino acid sequence of SEQ ID NO: 7, 24 or 37 and/or is encoded by any of the nucleic acid molecules of SEQ ID NO: 5, 6, 22, 23, 35 or 36 and that converts ligstroside aglycone and/or oleuropein aglycone and/or elenolic acid (as described herein).

The present invention further relates to a nucleic acid molecule encoding a polypeptide having esterase activity of the present invention and to a vector comprising said nucleic acid molecules. Vectors that can be used in accordance with the present invention are known in the art. The vectors can further comprise expression control sequences operably linked to the nucleic acid molecules of the present invention contained in the vectors. These expression control sequences may be suited to ensure transcription and synthesis of a translatable RNA in bacteria or fungi. Expression control sequences can for instance be promoters. Promoters for use in connection with the nucleic acid molecules of the present invention may be homologous or heterologous with regard to its origin and/or with regard to the gene to be expressed. Suitable promoters are for instance promoters which lend themselves to constitutive expression. However, promoters which are only activated at a point in time determined by external influences can also be used. Artificial and/or chemically inducible promoters may be used in this context. Preferably, the vector of the present invention is an expression vector. Expression vectors have been widely described in the literature. As a rule, they contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence, if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E. coli, S. cerevisiae*) are sufficiently described in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), lp1, rac (Boros et al., Gene 42 (1986), 97-100). Inducible promoters are preferably used for the synthesis of polypeptides. These promoters often lead to higher polypeptide yields than do constitutive promoters. In order to obtain an optimum amount of polypeptide, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-β-D-thiogalactopyranoside) (deBoer et al., Proc. Natl. Acad. Sci. USA 80 (1983), 21-25). Termination signals for transcription are also described in the literature.

Furthermore, the present invention relates to (a) nucleic acid molecule(s) which express the polypeptide(s) of the present invention. Accordingly, the present invention relates to a polypeptide as encoded by nucleic acid molecule of SEQ ID NO: 5 or 6. Furthermore, in the context of the present inventiona polypeptide encoded by a nucleic acid molecule is disclosed, which is at least n% identical to the nucleic acid sequence of SEQ ID NO: 5 or 6 which n being an integer between 69 and 100, preferably 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99 and which is characterized by having an esterase activity. In the context of the present invention, the nucleic acid molecule expressing or encoding a polypeptide of the present invention having an esterase activity may be a nucleic acid molecule which is mutated at one or more positions within the nucleic acid sequence of SEQ ID NO: 5 or 6. Accordingly, also disclosed is a nucleic acid molecules which comprise a nucleic acid molecule which is at least 60% identical to SEQ ID NO: 5 or 6 and in which one or more substitutions and/or deletions and/or insertions in the nucleic acid molecule as shown in SEQ ID NO: 5 or 6 have been effected. For example, the present invention relates to a nucleic acid molecule as shown in SEQ ID NO: 6 which differs from SEQ ID NO: 5 in that the nucleotide at position 51 (thymine) of SEQ ID NO: 5 has been replaced by cytosine. Accordingly, the present invention, relates to a nucleic acid as shown in SEQ ID NO: 5 or 6 which encodes a polypeptide which comprise the amino acid sequence as shown in SEQ ID NO: 7. Examplary disclosed is also a nucleic acid molecule as shown in SEQ ID NO: 23 which differs from SEQ ID NO: 22 in that the nucleotide at position 51 (thymine) of SEQ ID NO: 22 has been replaced by cytosine. Accordingly, disclosed is a nucleic acid as shown in SEQ ID NO: 22 or 23 which encodes a polypeptide which comprise the amino acid sequence as shown in SEQ ID NO: 24. For example, further disclosed is a nucleic acid molecule as shown in SEQ ID NO: 36 which differs from SEQ ID NO: 35 in that the nucleotide at position 51 (thymine) of SEQ ID NO: 35 has been replaced by cytosine. Accordingly, disclosed is a nucleic acid as shown in SEQ ID NO: 35 or 36 which encodes a polypeptide which comprise the amino acid sequence as shown in SEQ ID NO: 37.

The nucleic acid molecules/nucleic acid sequences of the invention may be of natural as well as of synthetic or semi-synthetic origin. Thus, the nucleic acid molecules may, for example, be nucleic acid molecules that have been synthesized according to conventional protocols of organic chemistry. The person skilled in the art is familiar with the preparation and the use of such nucleic acid molecules (see, e.g., Sambrook and Russel "Molecular Cloning, A Laboratory Manual", Cold Spring Harbor Laboratory, N.Y. (2001)).

The term comprising, as used herein, denotes that further sequences and/or method steps can be included in addition to the specifically recited sequences and/or method steps. However, this term also encompasses that the claimed subject-matter consists of exactly the recited sequences and/or method steps.

In those embodiments where the nucleic acid molecule comprises (rather than consists of) the recited sequence, additional nucleotides extend over the specific sequence either on the 5' end or the 3' end, or both. Those additional nucleotides may be of heterologous or homologous nature. In the case of homologous sequences, these sequences may comprise up to 1500 nucleotides at the 5' and/or the 3' end, such as e.g. up to 1000 nucleotides, such as up to 900 nucleotides, more preferably up to 800 nucleotides, such as up to 700 nucleotides, such as e.g. up to 600 nucleotides, such as up to 500 nucleotides, even more preferably up to 400 nucleotides, such as up to 300 nucleotides, such as e.g. up to 200 nucleotides, such as up to 100 nucleotides, more preferably up to 50 nucleotides, such as up to 40 nucleotides such as e.g. up to 30 nucleotides, such as up to 20 nucleotides, more preferably up to 10 nucleotides and most preferably up to 5 nucleotides at the 5' and/or the 3' end. The term "up to [...] nucleotides", as used herein, relates to a number of nucleotides that includes any integer below and including the specifically recited number. For example, the term "up to 5 nucleotides" refers to any of 1, 2, 3, 4 and 5 nucleotides. Furthermore, in the case of homologous sequences, those embodiments do not include complete genomes or complete chromosomes.

Additional heterologous sequences may, for example, include heterologous promoters, which are operatively linked to the coding sequences of the invention, as well as further regulatory nucleic acid sequences well known in the art and described in more detail herein below. Thus, in the context of the present invention, the nucleic acid sequences may be under the control of regulatory sequences. Accordingly, in the context of the present invention, the vector of the present invention further comprises a regulatory sequence, which is operably linked to the nucleic acid sequences described herein. For example, promoters, transcriptional enhancers and/or sequences, which allow for induced expression of the esterase described herein may be employed. In the context of the present invention, the nucleic acid molecules are expressed under the control of a constitutive or an inducible promoter.

The term "regulatory sequence" refers to DNA sequences, which are necessary to effect the expression of coding sequences to which they are ligated. The nature of such control sequences differs depending upon the host organism. In prokaryotes, control sequences generally include (a) promoter(s), (a) ribosomal binding site(s), and (a) terminator(s). In eukaryotes generally control sequences include (a) promoter(s), (a) terminator(s) and, in some instances, (an) enhancer(s), (a) transactivator(s) or (a) transcription factor(s). The term "control sequence" is intended to include, at a minimum, all components the presence of which are necessary for expression, and may also include additional advantageous components.

The term "hybridize/hybridizing" as used herein refers to a pairing of a polynucleotide to a (partially) complementary strand of this nucleic acid molecule which thereby form a hybrid. It is well known in the art how to perform hybridization experiments with nucleic acid molecules. Correspondingly, the skilled person knows what hybridization conditions s/he has to use to allow for a successful hybridization in accordance with item (d), above. The establishment of suitable hybridization conditions is referred to in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985). In the context of the present invention, the hybridization may be effected under stringent conditions. "Stringent hybridization conditions" refers to conditions which comprise, e.g. an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing the filters in 0.1 × SSC at about 65°C. Said conditions for hybridization are also known by a skilled person as "highly stringent conditions for hybridization". Also contemplated are nucleic acid molecules that hybridize to the polynucleotides of the invention at lower stringency hybridization conditions ("low stringency conditions for hybridization"). Changes in the stringency of hybridization and signal detection are primarily accomplished through the manipulation of formamide concentration (lower percentages of formamide result in lowered stringency), salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 mg/ml salmon sperm blocking DNA; followed by washes at 50°C with 1X SSPE, 0.1% SDS. In addition, to achieve an even lower stringency, washes performed following stringent hybridization can be done at higher salt concentrations (e.g. 5X SSC). It is of note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed). The embodiment recited herein above preferably refers to highly stringent conditions and alternatively to conditions of lower stringency.

The present disclosure also relates to the use of the polypeptides characterized by having an esterase activity or of a host cell comprising such (a) polypeptide(s) for the conversion of (a) compound(s)/substrate(s) as defined herein above and which are preferably selected from the iridoids and seco-iridoids, more preferably (a) compound(s)/substrate(s)of the above defined formula (I) or any preferred embodiments thereof. Even more preferably, in the context of the present invention, the provided polypeptide(s) can convert the substrate(s)/compound(s) such as oleuropein, oleuropein aglyone(s), ligstroside, ligstroside aglycone(s), elenolic acid, nüzhenide, oleoside-11-methylester, safghanoside, oleuroside, secologanin, adinoside, geniposide and/or loganin. The present dislosure relates to the use of the provided polypeptides which are characterized by having an esterase activity or of a host cell comprising such (a) polypeptide(s) for the conversion of (a) compound(s) corresponding the the general formula C₂₅H₃₂Oₙ, with n ≥ 12, such as, e.g., ligstroside or oleuropein, preferably (a) ligstroside or oleuropein aglycone(s), into a compound corresponding to the general formula C₂₅H₃₂Oₙ₋₇ such as, e.g., oleocanthal or oleacein. Further, disclosed is the use of the provided polypeptides which are characterized by having an esterase activity or of a host cell comprising such (a) polypeptide(s) for the conversion of oleoside-11-methylester, preferably elenolic acid (C₁₁H₁₄O₆), into decarboxymethyl elenolic acid dialdehyde (DEDA).

Therefore the present invention relates to a method for the production of oleocanthal comprising the enzymatic conversion of ligstroside into oleocanthal: (a) two enzymatic steps comprising (i) first the enzymatic conversion of ligstroside into a ligstroside aglycone; and (ii) then enzymatically converting the thus obtained ligstroside aglycone into said oleocanthal; or (b) a single enzymatic reaction in which the ligstroside aglycone is directly converted into oleocanthal by making use of the polypeptides of the present invention which are characterized by having an esterase activity.

Further, the present invention relates to a method for the production of oleacein comprising the enzymatic conversion of oleuropein into oleacein: (a) two enzymatic steps comprising (i) first the enzymatic conversion of oleuropein into an oleuropein aglycone; and (ii) then enzymatically converting the thus obtained oleuropein aglycone into said oleacein; or (b) a single enzymatic reaction in which the oleuropein aglycone is directly converted into oleacein by making use of the polypeptides of the present invention which are characterized by having an esterase activity.

The present invention also realts to a method for the production of decarboxymethyl elenolic acid dialdehyde (DEDA) comprising the conversion of oleoside-11-methylester into decarboxymethyl elenolic acid dialdehyde (DEDA): (a) two enzymatic steps comprising (i) first the enzymatic conversion of oleoside-11-methylester into elenolic acid; and (ii) then enzymatically converting the thus obtained elenolic acid into decarboxymethyl elenolic acid dialdehyde (DEDA) by making use of the polypeptides of the present invention which are characterized by having an esterase activity.

The amino acid sequence of the polypeptide having esterase acivtiy of the present invention is shown in SEQ ID NO: 7. In the context of the present disclosure which is not part of the invention, any protein/polypeptide showing an amino acid sequence which is at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to SEQ ID NO: 7 and having an esterase activity wherein such an enzyme is capale of catalyzing the enzymatic conversion of (a) compound(s)/substrate(s) as defined herein above and which are preferably selected from the iridoids and seco-iridoids, more preferably (a) compound(s)/substrate(s)of the above defined formula (I) or any preferred embodiments thereof can be employed in a method according to the present invention. Even more preferably, in the context of the present invention, the provided polypeptide(s) can convert the substrate(s)/compound(s) such as oleuropein, oleuropein aglcone(s), ligstroside, ligstroside aglycone(s), elenolic acid, nüzhenide, oleoside-11-methylester, safghanoside, oleuroside, secologanin, adinoside, geniposide and/or loganin can be employed in a method according to the present invention. In the context of the present invention, the disclosure relates to the use of the provided polypeptides which are characterized by having an esterase activity or of a host cell comprising such (a) polypeptide(s) for the converstion of (a) compound(s) corresponding the the general formula C₂₅H₃₂Oₙ, with n ≥ 12, such as, e.g., ligstroside or oleuropein, preferably a ligstroside aglycone or oleuropein aglycone, into a compound corresponding to the general formula C₂₅H₃₂Oₙ₋₇ such as, e.g., oleocanthal or oleacein which can be employed in a method according to the present invention. Further, the present disclosure relates to the use of the provided polypeptides which are characterized by having an esterase activity or of a host cell comprising such (a) polypeptide(s) for the conversion of oleoside-11-methylester, preferably elenolic acid (C₁₁H₁₄O₆), into decarboxymethyl elenolic acid dialdehyde (DEDA) which can be employed in a method according to the present invention.

A method according to the present invention may be carried out in vitro or in vivo. An in vitro reaction is understood to be a reaction in which no cells are employed, i.e. an acellular reaction. Thus, in vitro preferably means in a cell-free system. The term "in vitro" in the context of the present invention means in the presence of isolated polypeptides which are characterized by having an esterase activity (or systems optionally comprising possibly required cofactors). In one embodiment, the polypeptides of the present invention employed in the method of the present invention are used in purified form. In the context of the present invention, it is preferred that in an in vitro reaction the polypeptides of the present invention and an enzyme categorized under the EC number 3.2.1 (glucosidase) are employed in the method of the present invention. In the context of the present invention, in the in vitro reaction an enzyme is used which is classified as EC 3.2.1 and which has glucosidase activity. Preferably the enzyme used in the context of the present invention, e.g., for converting oleuropein into an oleuropein aglycone and/or ligstroside into a ligstroside aglycone as described herein refers to a glucosidase which is classified as EC 3.2.1.21 (beta-glucosidase). In the context of the present invention, enzyme preparations with glucosidase activity, more preferably beta-glucosidase activity can be used. Exemplarily enzyme preparations with beta-glucosidase activity which may be used in the context of the present invention relate to Lallzyme C-MaxTM (Lallemand Inc. (Austria)), Beerzym Combi, Beerzym Penta, Beerzym BG-Super or Beerzym Fructozyme NAR (Erbslöh Geisenheim AG (Germany)), the enzyme preparation Brewers Compass^{®}, Brewers FiltraseTM, Rapidase^{®} (DSM, Düsseldorf (Germany)), Cellulase TXL (ASA GmbH, Wolfenbüttel (Germany)), Rohlase^{®}, Rohament^{®} (AB Enzymes GmbH, Darmstadt (Germany)), Panzym^{®} Arome G, or Panzym^{®} Extract G (Eaton Technologies GmbH, Langenlonsheim (Germany)).

Accordingly, the present invention relates to a method for the production of oleacein comprising the enzymatic conversion of an oleuropein aglycone into oleacein, wherein said method comprises the use of a polypeptide having esterase activity as described herein. In the context of the present invention, the method for the production of oleacein comprises the following steps:
(i) enzymatic conversion of oleuropein into an oleuropein aglycone as described herein; and
(ii) enzymatic conversion of the oleuropein aglycone into oleacein.

Further, the present invention relates to a method for the production of decarboxymethyl elenolic acid dialdehyde (DEDA) comprising the enzymatic conversion of an elenolic acid into decarboxymethyl elenolic acid dialdehyde (DEDA), wherein said method comprises the use of a polypeptide having esterase activity as described herein.

In the context of the present invention, the method for the production of decarboxymethyl elenolic acid dialdehyde (DEDA) comprises the following steps:
(i) enzymatic conversion of oleoside-11-methylester into elenolic acid; and
(ii) enzymatic conversion of the elenolic acid into decarboxymethyl elenolic acid dialdehyde (DEDA).

Furthermore, the present invention relates to a method for the production of oleocanthal comprising the enzymatic conversion of a ligstroside aglycone into oleocanthal, wherein said method comprises the use of a polypeptide having an esterase activity as described herein. In the context of the present invention, the method for the production of oleocanthal comprises the following steps:
(i) enzymatic conversion of ligstroside into a ligstroside aglycone as described herein; and
(ii) enzymatic conversion of the ligstroside aglycone into oleocanthal.

In the context of the present invention, for carrying out the above reaction step (i) it is preferred that an enzyme categorized under the EC number 3.2.1 (glucosidase) is employed in the method of the present invention. Preferably the enzyme used in the context of the present invention for converting oleuropein into an oleuropein aglycone and/or ligstroside into a ligstroside aglycone as described herein refers to a glucosidase which is classified as EC 3.2.1.21 (beta-glucosidase). In the context of the present invention, enzyme preparations with glucosidase activity, more preferably beta-glucosidase activity, can be used. Exemplarily enzyme preparations with beta-glucosidase activity which may be used in the context of the present invention relate to Lallzyme C-MaxTM (Lallemand Inc. (Austria)), Beerzym Combi, Beerzym Penta, Beerzym BG-Super or Beerzym Fructozyme NAR (Erbslöh Geisenheim AG (Germany)), the enzyme preparation Brewers Compass^{®}, Brewers FiltraseTM, Rapidase^{®} (DSM, Düsseldorf (Germany)), Cellulase TXL (ASA GmbH, Wolfenbüttel (Germany)), Rohlase^{®}, Rohament^{®} (AB Enzymes GmbH, Darmstadt (Germany)), Panzym^{®} Arome G, or Panzym^{®} Extract G (Eaton Technologies GmbH, Langenlonsheim (Germany)).

For carrying out the method in vitro the substrates for the reaction and the polypeptides which are characterized by having an esterase activity are incubated under conditions (buffer, temperature, co-substrates, co-factors etc.) allowing the esterase and/or the enzyme categorized under the EC number 3.2.1 as described herein to be active and the enzymatic conversion to occur. The reaction is allowed to proceed for a time sufficient to produce the respective product(s). In the context of the present invention, the in vitro method is carried out at pH range between 1.0 to 10.0, preferably at a pH range between 2.5 to 5.5, even more preferably at a pH range between 4.0 to 5.0. In the context of the present invention the in vitro method is carried out at a pH range between 4.0 to 5.0 and/or at an incubation temperature between 15°C to 50°C and/or a sodium buffer with a salt concentration of 100, preferably of 50 mM or less. The production of the respective product(s) can be measured by methods known in the art, such as gas chromatography possibly linked to mass spectrometry detection.

The polypeptides of the present invention having an esterase activity may be in any suitable form allowing the enzymatic reaction to take place. They may be purified or partially purified or in the form of crude cellular extracts or partially purified extracts. It is also possible that the enzymes are immobilized on a suitable carrier.

In the context of the present invention the method according to the invention is carried out in culture, in the presence of an organism, a microorganism, preferably a plant cell, producing the polypeptide having an esterase activity described herein for the conversions of the methods according to the present invention.

In the context of the present invention an organism, a microorganism, or preferably a plant cell is used which expresses in addition to the polypeptide(s) having esterase activity an enzyme which is categorized under the EC number 3.2.1 (glucosidase). Accordingly, in the context of the present invention the organism, microorganism, or preferably plant cell expresses (a) polypeptide(s) having esterase activity of the present invention and an enzyme which is categorized under the EC number 3.2.1 (glucosidase) for the conversions of the methods according the present invention.

In the context of the present invention, the method employs an organism, a microorganism, preferably a plant cell, for carrying out a method according to the invention is referred to as an "in vivo" method. It is possible to use a organism, microorganism, preferably a plant cell, which naturally produces the polypeptide having an esterase activity described above and optionally an enzyme which is categorized under the EC number 3.2.1 (glucosidase) for the conversions of the methods according to the present invention or an organism, a microorganism, preferably a plant cell, which had been genetically modified so that it expresses (including overexpression) one or more of such polypeptides having an esterase activity and optionally an enzyme which is categorized under the EC number 3.2.1 (glucosidase). Thus, the organism, microorganism, preferably plant cell, can be an engineered organism, microorganism, preferably plant cell, which expresses polypeptides having an esterase activity described herein and optionally further expresses an enzyme which is categorized under the EC number 3.2.1 (glucosidase) for the conversion of the methods according to the present invention, i.e. which has in its genome a nucleic acid sequence or a vector expressing such enzymes, i.e. polypeptide(s) having an esterase activity as described herein and an enzyme which is categorized under the EC number 3.2.1 (glucosidase) as described herein and which has been modified to express/overexpress them. The expression may occur constitutively or in an induced or regulated manner.

In the context of the present invention, the organism, microorganism or preferably plant cell can be an organism, a microorganism or preferably a plant cell which has been genetically modified by the introduction of one or more nucleic acid molecules containing nucleotide sequences encoding one or more polypeptide(s) having an esterase activity described herein for the conversion of the methods according to the present invention. The nucleic acid molecule can be stably integrated into the genome or the organism, microorganism or preferably plant cell or may be present in an extrachromosomal manner, e.g., on a plasmid if a microorganism is used.

Such a genetically modified organism, microorganism or preferably plant cell can, e.g., be an organism, a microorganism or preferably a plant cell that does not naturally express (a) polypeptide(s) having esterase activity as described herein and/or an enzyme which is categorized under the EC number 3.2.1 (glucosidase) as described herein for the conversions of the methods according to the present invention and which has been genetically modified to express such polypeptides or an organism, a microorganism or preferably a plant cell which naturally express such polypeptides having esterase activity and/or enzymes which are categorized under the EC number 3.2.1 (glucosidase) which has been genetically modified, e.g., transformed with a nucleic acid molecule, e.g., a vector encoding the respective polypeptide(s) having esterase activity and/or glucosidase activity, and/or insertion of a promoter in front of the endogenous nucleotide sequence encoding the polypeptide(s) of the present invention in order to increase the respective esterase activity and/or glucosidase activity in said organism, microorganism or preferably plant cell.

However, the invention preferably excludes naturally occurring organisms, microorganisms or preferably plant cells as found in nature expressing a polypeptide characterized by having an esterase activity in accordance with the present invention and/or an enzyme which is categorized under the EC number 3.2.1 (glucosidase) and employed in a method of the present invention is preferably a non-naturally occurring organism, microorganism or preferably plant cell, whether it has been genetically modified to express (including overexpression) an exogenous polypeptide of the present invention and/or an enzyme which is categorized under the EC number 3.2.1 (glucosidase) not normally existing in its genome or whether it has been engineered to overexpress an exogenous polypeptide having an esterase activity and/or an enzyme which is categorized under the EC number 3.2.1 (glucosidase) of the present invention.

Thus, the organisms, microorganisms or preferably plant cells employed in connection with the present invention are preferably non-naturally occurring polypeptides/enzymes having esterase activity or organisms, microorganisms or plant cells, i.e. they are enzymes or organisms, microorganisms or preferably plant cells which differ significantly from naturally occurring enzymes or organisms, microorganisms or preferably plant cells and which do not occur in nature. As regards the polypeptides having esterase activity, i.e. the enzymes, they may e.g. be variants of the polypeptide/enzyme having the amino acid sequence shown in SEQ ID NO: 7. Such variants include, e.g., mutants, in particular prepared by molecular biological methods, which show improved properties, such as a higher enzyme activity, higher substrate specificity, higher temperature resistance and the like. As regards the organisms, microorganisms or preferably plant cells, they are preferably genetically modified organisms, microorganisms or plant cells as described herein which differ from naturally occurring organisms, microorganisms or plant cells due to a genetic modification. Genetically modified organisms, microorganisms or plant cells are organisms, microorganisms or plant cells which do not naturally occur, i.e., which cannot be found in nature, and which differ substantially from naturally occurring organisms, microorganisms or plant cells due to the introduction of a foreign nucleic acid molecule.

By overexpressing an exogenous or endogenous polypeptide having esterase activity and/or an enzyme having glucosidase activity, i.e. an enzyme which is categorized under the EC number 3.2.1 (glucosidase) as described herein, the concentration of the polypeptide having esterase activity and/or glucosidase activity, i.e. the esterase activity and/or glucosidase activity is substantially higher than what is found in a non-overexpressed nature, which can then unexpectedly force the reaction of the present invention which uses a non-overexpressed form of the respective esterase. Preferably, the concentration of the overexpressed esterase and/or glucosidase is at least 5%, 10%, 20%, 30% or 40% of the total host cell protein.

Thus, it is also possible in the context of the present invention that the organism, microorganism, or preferably plant cell is an organism, microorganism, or preferably plant cell which naturally does not have the respective esterase enzyme activity and/or glucosidase enzyme activity but which is genetically modified so as to comprise a nucleotide sequence allowing the expression of (a) corresponding enzyme(s). Similarly, the organism, microorganism, or preferably plant cell may also be an organism, a microorganism, or preferably a plant cell which naturally has the respective esterase enzyme activity and/or glucosidase enzyme activity but which is genetically modified so as to enhance such an esterase enzyme activity and/or glucosidase enzyme activity, e.g. by the introduction of an exogenous nucleotide sequence encoding (a) corresponding enzyme(s) or by the introduction of a promoter for the endogenous gene(s) encoding the enzyme(s) to increase endogenous production to overexpressed (non-natural) levels.

If an organism, microorganism, or preferably plant cell is used which naturally expresses a corresponding esterase and/or glucosidase, it is possible to modify such an organism, microorganism, or preferably plant cell so that the respective esterase activity and/or esterase activity is overexpressed in the organism, microorganism or preferably plant cell. This can, e.g., be achieved by effecting mutations in the promoter region of the corresponding gene(s) or introduction of a high expressing promoter so as to lead to a promoter which ensures a higher expression of the gene(s). Alternatively, it is also possible to mutate the gene(s) as such so as to lead to (an) enzyme(s) showing a higher activity.

By using organisms, microorganisms, or preferably plant cells which express polypeptides having an esterase activity and/or which further expresses an enzyme which is categorized under the EC number 3.2.1 (glucosidase) for the conversions of the methods according to the present invention, it is possible to carry out the methods according to the invention directly in the culture medium, without the need to separate or purify the enzyme(s). In the context of the present invention, the organism, microorganism, or preferably plant cell employed in a method according to the invention is an organism, microorganism or plant cells which has been genetically modified to express (a) polypeptide(s) having esterase activity and/or further express an enzyme which is categorized under the EC number 3.2.1 (glucosidase). In the context of the present invention, the

The term "organism" in the context of the present invention refers to any eukaryotic and prokaryotic organisms. The term "microorganism" in the context of the present invention refers to bacteria, as well as to fungi, such as yeasts, and also to algae and archaea. In the context of the, the microorganism is preferably a bacterium or a yeast. In principle any bacterium can be used. Preferred bacteria to be employed in the process according to the invention are bacteria of the genus *Bacillus, Clostridium, Corynebacterium, Pseudomonas, Zymomonas* or *Escherichia.* In a particularly preferred embodiment the bacterium belongs to the genus *Escherichia* and even more preferred to the species *Escherichia coli.* In another preferred embodiment the bacterium belongs to the species *Pseudomonas putida* or to the species *Zymomonas mobilis* or to the species *Corynebacterium glutamicum* or to the species *Bacillus subtilis.*In another preferred embodiment the microorganism is a fungus, more preferably a fungus of the genus *Saccharomyces, Schizosaccharomyces, Aspergillus, Trichoderma, Kluyveromyces* or *Pichia* and even more preferably of the species *Saccharomyces cerevisiae, Schizosaccharomyces pombe, Aspergillus niger, Trichoderma reesei, Kluyveromyces marxianus, Kluyveromyces lactis, Pichia pastoris, Pichia torula* or *Pichia utilis.*

In the context of the invention, the method according to the invention makes use of a photosynthetic microorganism expressing at least one enzyme for the conversion according to the invention as described above. Preferably, the microorganism is a photosynthetic bacterium, or a microalgae. Alternatively, the microorganism is an algae, more preferably an algae belonging to the diatomeae.

In the context of the present invention, a "plant cell" is preferred. In principle any plat can be used. Preferred plants to be employed in the method of present invention are plants which belong to the family *Oleaceae.*

In the context of the present invention the term "plant" refers to any various photosynthetic, eukaryotic multicellular organisms of the kingdom Plantae, characteristically producing embryos, containing chloroplasts, having cellulose cell walls and lacking locomotion. As used herein, a "plant" includes any plant or part of a plant at any stage of development, including seeds, suspension cultures, plant cells, embryos, meristematic regions, callus tissue, leaves, roots, shoots, gametophytes, sporophytes, pollen, microspores, and progeny thereof. Also included are cuttings, and cell or tissue cultures. As used in conjunction with the present disclosure, plant tissue includes, without limitation, whole plants, plant cells, plant organs, e.g., leafs, stems, roots, meristems, plant seeds, protoplasts, callus, cell cultures, and any groups of plant cells organized into structural and/or functional units. Accordingly, the polypeptides of the present invention having an esterase activity and/or the enzyme which is categorized under the EC number 3.2.1 (glucosidase) may be introduced into plant cells via any suitable methods known in the art.

In another embodiment, the method of the invention comprises the step of providing the organism, microorganism, preferably the plant cell, carrying the respective esterase enzyme activity or esterase and glucosidase enzyme activities in the form of a (cell) culture, preferably in the form of a liquid cell culture, a subsequent step of cultivating the organism, microorganism, preferably the plant cell in a fermenter (often also referred to a bioreactor) under suitable conditions allowing the expression of the respective enzyme(s) and further comprising the step of effecting an enzymatic conversion of a method of the invention as described herein above. Suitable fermenter or bioreactor devices and fermentation conditions are known to the person skilled in the art. A bioreactor or a fermenter refers to any manufactured or engineered device or system known in the art that supports a biologically active environment. Thus, a bioreactor or a fermenter may be a vessel in which a chemical/biochemical like the method of the present invention is carried out which involves organisms, microorganisms, preferably plant cells and/or biochemically active substance(s), i.e., the enzyme(s) described above or organisms, microorganisms or plant cells harbouring the herein described enzyme(s). In a bioreactor or a fermenter, this process can either be aerobic or anaerobic. These bioreactors are commonly cylindrical, and may range in size from litres to cubic metres, and are often made of stainless steel. In this respect, without being bound by theory, the fermenter or bioreactor may be designed in a way that it is suitable to cultivate the organisms, preferably microorganisms, in, e.g., a batch-culture, feed-batch-culture, perfusion culture or chemostate-culture, all of which are generally known in the art. The culture medium can be any culture medium suitable for cultivating the respective organism or microorganism.

In the context of the present invention the method according to the present invention also comprises the step of recovering the oleocanthal (or the ligstroside aglycone), oleacein (or the oleacein aglycone), or decarboxymethyl elenolic acid dialdehyde (DEDA) (or elenolic acid) produced by the method. For example, if the method according to the present invention is carried out in vivo by fermenting a corresponding organism, microorganism, or preferably a plant cell expressing the necessary enzymes, the oleocanthal (or the ligstroside aglycone) can be recovered by solvent liquid/liquid extraction.

In the context of the present invention the method as described herein preferably relates to a method in which an organism, microorganism, or preferably plant cell as described herein is employed, wherein the organism, microorganism, or preferably plant cell is capable of enzymatically converting ligstroside into ligstroside aglycone and preferably further into oleocanthal, wherein said method comprises culturing the organism, microorganism, or preferably plant cell in a culture medium. The invention also relates to a method in which an organism, microorganism, or preferably plant cell as described herein is employed, wherein the organism, microorganism, or preferably plant cell is capable of enzymatically converting oleuropein into oleuropein aglycone and preferably further into oleacein, wherein said method comprises culturing the organism, microorganism, or preferably plant cell in a culture medium.

The enzymes used in the method according to the invention can be the esterase as shown in the amino acid sequence of SEQ ID NO: 7 and/or an enzyme which is categorized under the EC number 3.2.1 (glucosidase) or enzymes which are derived from (a) naturally occurring enzyme(s), e.g., by the introduction of mutations or other alterations which, e.g., alter or improve the enzymatic activity, the stability, etc.

Methods for modifying and/or improving the desired enzymatic activities of proteins are well-known to the person skilled in the art and include, e.g., random mutagenesis or site-directed mutagenesis and subsequent selection of enzymes having the desired properties or approaches of the so-called "directed evolution". For example, for genetic modification in prokaryotic cells, a nucleic acid molecule encoding a corresponding enzyme can be introduced into plasmids which permit mutagenesis or sequence modification by recombination of DNA sequences. Standard methods (see Sambrook and Russell (2001), Molecular Cloning: A Laboratory Manual, CSH Press, Cold Spring Harbor, NY, USA) allow base exchanges to be performed or natural or synthetic sequences to be added. DNA fragments can be ligated by using adapters and linkers complementary to the fragments. Moreover, engineering measures which provide suitable restriction sites or remove surplus DNA or restriction sites can be used. In those cases, in which insertions, deletions or substitutions are possible, *in vitro* mutagenesis, "primer repair", restriction or ligation can be used. In general, a sequence analysis, restriction analysis and other methods of biochemistry and molecular biology are carried out as analysis methods. The resulting enzyme variants are then tested for the desired activity, e.g., enzymatic activity, with an assay as described herein and in particular for their increased enzyme activity.

As described herein, the organism, microorganism, or preferably plant cell employed in a method of the invention may be an organism, microorganism, or preferably plant cell which has been genetically modified by the introduction of a nucleic acid molecule encoding a polypeptide as described herein having an esterase activity and/or an enzyme which is categorized under the EC number 3.2.1 (glucosidase).

Thus, in a the context of the present invention the organism, microorganism, or preferably plant cell is preferably is a recombinant organism, microorganism or plant cell which has been genetically modified to have an increased activity of at least one enzyme described herein for the conversions of the method according to the present invention. This can be achieved e.g. by transforming the organism, microorganism, or plant cell with a nucleic acid encoding a corresponding enzyme. A detailed description of genetic modification of microorganisms will be given further below. Preferably, the nucleic acid molecule introduced into the organism, microorganism or plant cell is a nucleic acid molecule which is heterologous with respect to the organism, microorganism or plant cell, i.e. it does not naturally occur in said organism, microorganism or plant cell.

In the context of the present invention, an "increased activity" means that the expression and/or the activity of an enzyme in the genetically modified organism, microorganism, or plant cell is at least 10%, preferably at least 20%, more preferably at least 30% or 50%, even more preferably at least 70% or 80% and particularly preferred at least 90% or 100% higher than in the corresponding non-modified organism, microorganism or plant cell. In even more preferred embodiments the increase in expression and/or activity may be at least 150%, at least 200% or at least 500%. In particularly preferred embodiments the expression is at least 10-fold, more preferably at least 100-fold and even more preferred at least 1000-fold higher than in the corresponding non-modified organism, microorganism, or plant cell.

The term "increased" expression/activity also covers the situation in which the corresponding non-modified organism, microorganism, or plant cell does not express a corresponding enzyme so that the corresponding expression/activity in the non-modified organism, microorganism, or plant cell is zero. Preferably, the concentration of the overexpressed enzyme is at least 5%, 10%, 20%, 30%, or 40% of the total protein content of genetically modified organism, microorganism, or plant cell.

Methods for measuring the level of expression of a given protein in a cell are well known to the person skilled in the art. In one embodiment, the measurement of the level of expression is done by measuring the amount of the corresponding protein. Corresponding methods are well known to the person skilled in the art and include Western Blot, ELISA etc. In another embodiment the measurement of the level of expression is done by measuring the amount of the corresponding RNA. Corresponding methods are well known to the person skilled in the art and include, e.g., Northern Blot.

In the context of the present invention the term "recombinant" means that the organism, microorganism or plant cell is genetically modified so as to contain a nucleic acid molecule encoding an enzyme as defined above as compared to a wild-type or non-modified organism, microorganism, or plant cell. A nucleic acid molecule encoding an polypeptide having esterase activity and/or an enzyme which is categorized under the EC number 3.2.1 (glucosidase) as described herein can be used alone or as part of a vector.

The nucleic acid molecules can further comprise expression control sequences operably linked to the polynucleotide comprised in the nucleic acid molecule. The term "operatively linked" or "operably linked", as used throughout the present description, refers to a linkage between one or more expression control sequences and the coding region in the polynucleotide to be expressed in such a way that expression is achieved under conditions compatible with the expression control sequence.

Expression comprises transcription of the heterologous DNA sequence, preferably into a translatable mRNA. Regulatory elements ensuring expression in fungi as well as in bacteria, are well known to those skilled in the art. They encompass promoters, enhancers, termination signals, targeting signals and the like. Examples are given further below in connection with explanations concerning vectors.

Promoters for use in connection with the nucleic acid molecule may be homologous or heterologous with regard to its origin and/or with regard to the gene to be expressed. Suitable promoters are for instance promoters which lend themselves to constitutive expression. However, promoters which are only activated at a point in time determined by external influences can also be used. Artificial and/or chemically inducible promoters may be used in this context.

The vectors can further comprise expression control sequences operably linked to said polynucleotides contained in the vectors. These expression control sequences may be suited to ensure transcription and synthesis of a translatable RNA in bacteria or fungi.

In addition, it is possible to insert different mutations into the nucleic acid molecules by methods usual in molecular biology (see for instance Sambrook and Russell (2001), Molecular Cloning: A Laboratory Manual, CSH Press, Cold Spring Harbor, NY, USA), leading to the synthesis of polypeptides possibly having modified biological properties. The introduction of point mutations is conceivable at positions at which a modification of the amino acid sequence for instance influences the biological activity or the regulation of the polypeptide. Moreover, mutants possessing a modified substrate or product specificity can be prepared. Preferably, such mutants show an increased esterase and/or glucosidase activity. Alternatively, mutants can be prepared the catalytic activity of which is abolished without losing substrate binding activity.

Furthermore, the introduction of mutations into the nucleic acid molecules encoding an enzyme as described herein, i.e. the polypeptides having an esterase activity and/or enzymes which are categorized under the EC number 3.2.1 (glucosidase) allows the gene expression rate and/or the activity of the enzymes encoded by said nucleic acid molecules to be reduced or increased.

For genetically modifying bacteria or fungi, the polynucleotides encoding an enzyme as defined above or parts of these molecules can be introduced into plasmids which permit mutagenesis or sequence modification by recombination of DNA sequences. Standard methods (see Sambrook and Russell (2001), Molecular Cloning: A Laboratory Manual, CSH Press, Cold Spring Harbor, NY, USA) allow base exchanges to be performed or natural or synthetic sequences to be added. DNA fragments can be connected to each other by applying adapters and linkers to the fragments. Moreover, engineering measures which provide suitable restriction sites or remove surplus DNA or restriction sites can be used. In those cases, in which insertions, deletions or substitutions are possible, *in vitro* mutagenesis, "primer repair", restriction or ligation can be used. In general, a sequence analysis, restriction analysis and other methods of biochemistry and molecular biology are carried out as analysis methods.

Thus, in accordance with the present invention a recombinant microorganism can be produced by genetically modifying fungi or bacteria comprising introducing the above-described polynucleotides, nucleic acid molecules or vectors into a fungus or bacterium.

The polynucleotide encoding the respective enzyme is expressed so as to lead to the production of a polypeptide having any of the activities described above. An overview of different expression systems is for instance contained in Methods in Enzymology 153 (1987), 385-516, in Bitter et al. (Methods in Enzymology 153 (1987), 516-544) and in Sawers et al. (Applied Microbiology and Biotechnology 46 (1996), 1-9), Billman-Jacobe (Current Opinion in Biotechnology 7 (1996), 500-4), Hockney (Trends in Biotechnology 12 (1994), 456-463), Griffiths et al., (Methods in Molecular Biology 75 (1997), 427-440). An overview of yeast expression systems is for instance given by Hensing et al. (Antonie van Leuwenhoek 67 (1995), 261-279), Bussineau et al. (Developments in Biological Standardization 83 (1994), 13-19), Gellissen et al. (Antonie van Leuwenhoek 62 (1992), 79-93, Fleer (Current Opinion in Biotechnology 3 (1992), 486-496), Vedvick (Current Opinion in Biotechnology 2 (1991), 742-745) and Buckholz (Bio/Technology 9 (1991), 1067-1072).

Expression vectors have been widely described in the literature. As a rule, they contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence, if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E. coli, S. cerevisiae)* are sufficiently described in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), lp1, rac (Boros et al., Gene 42 (1986), 97-100). Inducible promoters are preferably used for the synthesis of polypeptides. These promoters often lead to higher polypeptide yields than do constitutive promoters. In order to obtain an optimum amount of polypeptide, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-β-D-thiogalactopyranoside) (deBoer et al., Proc. Natl. Acad. Sci. USA 80 (1983), 21-25). Termination signals for transcription are also described in the literature.

The transformation of the host cell with a polynucleotide or vector as described above can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990. The host cell is cultured in nutrient media meeting the requirements of the particular host cell used, in particular in respect of the pH value, temperature, salt concentration, aeration, antibiotics, vitamins, trace elements etc.

The present invention also relates to a (recombinant) organism, microorganism or plant cell which is able to express (a) polypeptides having an esterase activity as described herein and/or an enzyme which is categorized under the EC number 3.2.1 (glucosidase). Thus, in the context of the present invention, a (recombinant) organism, microorganism or plant cell expresses preferably both of the above described enzymes required for the enzymatic conversion of (i) ligstroside into oleocanthal or (ii) oleuropein into oleacein.

Thus, the present invention relates to a recombinant organism, microorganism, or plant cell which expresses
(i) an enzyme capable of converting ligstroside into a ligstroside aglycone as described herein; and
(ii) a polypeptide having an esterase activity as described herein which is capable of converting the ligstroside aglycone into oleocanthal.

The present invention relates to the recombinant organism, microorganism, or plant cell which expresses (i) an enzyme capable of converting ligstroside into a ligstroside aglycone as described herein; and (ii) a polypeptide having an esterase activity as described herein which is capable of converting the ligstroside aglycone into oleocanthal is a recombinant organism, microorganism, or plant cell, wherein the enzyme capable of enzyme capable of enzymatically converting said ligstroside into said ligstroside aglycone is an enzyme which is classified as EC 3.2.1 (glucosidase) and the enzyme which is capable of converting said ligstroside aglycone into oleocanthal is the polypeptide of the present invention, wherein said polypeptide is preferably an esterase having the amino acid sequence as shown in SEQ ID NO: 7.

The present invention also relates to a recombinant organism, microorganism, or plant cell which expresses
(i) an enzyme capable of converting oleuropein into an oleuropein aglycone as described herein; and
(ii) a polypeptide having an esterase activity as described herein which is capable of converting the oleuropein aglycone into oleacein.

The present invention relates to the recombinant organism, microorganism, or plant cell which expresses (i) an enzyme capable of converting oleuropein into a oleuropein aglycone as described herein; and (ii) a polypeptide having an esterase activity as described herein which is capable of converting the oleuropein aglycone into oleacein is a recombinant organism, microorganism, or plant cell, wherein the enzyme capable of enzyme capable of enzymatically converting said oleuropein into said oleuropein aglycone is an enzyme which is classified as EC 3.2.1 (glucosidase) and the enzyme which is capable of converting said oleuropein aglycone into oleacein is the polypeptide of the present invention, wherein said polypeptide is preferably an esterase having the amino acid sequence as shown in SEQ ID NO: 7.

Further, the present invention also relates to a recombinant organism, microorganism, or plant cell which expresses
(i) an enzyme capable of converting oleoside-11-methylester into elenolic acid as described herein; and
(ii) a polypeptide having an esterase activity as described herein which is capable of converting elenolic acid into decarboxymethyl elenolic acid dialdehyde (DEDA).

The present invention relates to the recombinant organism, microorganism, or plant cell which expresses (i) an enzyme capable of converting oleoside-11-methylester into elenolic acid as described herein; and (ii) a polypeptide having an esterase activity as described herein which is capable of converting elenolic acid into decarboxymethyl elenolic acid dialdehyde (DEDA) is a recombinant organism, microorganism, or plant cell, wherein the enzyme capable of enzyme capable of enzymatically converting said oleoside-11-methylester into elenolic acid is an enzyme which is classified as EC 3.2.1 (glucosidase) and the enzyme which is capable of converting said elenolic acid into decarboxymethyl elenolic acid dialdehyde (DEDA) is the polypeptide of the present invention, wherein said polypeptide is preferably an esterase having the amino acid sequence as shown in SEQ ID NO: 7.

In the context of the present invention, the recombinant organism, microorganism, or plant cell is an organism, microorganism, or plant cell wherein the enzyme classified as EC 3.2.1 is selected from the group consisting of OeGLU from *Olea europeae* (Koudounas et al., J. Exp. Bot., 66(7) (2015), 2093-2106; UniProt accession number: Q8GVD0 (second accession number: V5RMD5) (entry version number: 60; sequence version number: 1)) and EcSβgly from *Sulfolobus solfataricus* (Briante et al., J. Biotechnol., 77(2-3) (2000), 275-286). Further, in the context of the present invention the enzyme classified as EC 3.2.1 can be β-glucosidase(s) from almonds (CAS 9001-22-3; Jemai et al., Chem. Biol. Interact., 176(2-3) (2008), 88-98). Exemplarily β-glucosidase(s) from almonds which may be used in the context of the present invention are known to the skilled person and are, e.g., commercially available from Sigma-Aldrich, St. Louis, MO, USA, under the catalogue number G0395. Further, in the context of the present invention the enzyme classified as EC 3.2.1 can also be an enzyme mixture such, e.g., a filtrate from *Aspergillus niger* culture broth as described in Khoufi et al., Bioresour. Technol., 102(19) (2011), 9050-9058.

The microorganism is preferably a bacterium, a yeast or a fungus. Further, in the context of the present invention the organism is a plant or a non-human animal. As regards other preferred embodiments of the organism, microorganism, or plant cell, the same applies as has been set forth above in connection with the methods according to the present invention.

The present disclosure furthermore relates to the use of a polypeptide having an esterase activity as described herein and/or of an enzyme which is categorized under the EC number 3.2.1 (glucosidase), or an organism, microorganism, or plant cell which expresses such (an) enzyme(s) for the conversion of ligstroside into oleocanthal. In the context of the present invention, the polypeptide having an esterase activity is preferably an enzyme having the amino acid sequence as shown in SEQ ID NO: 7. Further, examples of a polypeptide having an esterase activity is preferably an enzyme having the amino acid sequence as shown in SEQ ID NO: 24 or 37.

Correspondingly, the present disclosure furthermore relates to the use of a polypeptide having an esterase activity as described herein and/or of an enzyme which is categorized under the EC number 3.2.1 (glucosidase), or an organism, microorganism, or plant cell which expresses such (an) enzyme(s) for the conversion of oleuropein into oleacein. In the context of the present invention, the polypeptide having an esterase activity is preferably an enzyme having the amino acid sequence as shown in SEQ ID NO: 7. Further, examples of a polypeptide having an esterase activity in the context of the present invention is an enzyme having the amino acid sequence as shown in SEQ ID NO: 24 or 37.

As regards the enzyme which is categorized under the EC number 3.2.1 (glucosidase) and the organism, microorganism, or plant cell the same applies as has been set forth above in connection with the methods and the organisms, microorganisms, or plant cells according to the present invention.

Furthermore, disclosed is a composition comprising:
(a) oleocanthal; and
(b) a polypeptide having esterase activity as described herein and/or an enzyme which is categorized under the EC number 3.2.1 (glucosidase) or a (recombinant) organism, microorganism, or plant cell which expresses such (an) enzyme(s). In the context of the present invention, said polypeptide having an esterase activity is preferably an enzyme having an amino acid sequence as shown in SEQ ID NO: 7. Another example of a polypeptide having an esterase activity in the context of the present invention is an enzyme having the amino acid sequence as shown in SEQ ID NO: 24 or 37.

As regards the enzyme which is categorized under the EC number 3.2.1 (glucosidase) and the organism, microorganism, or plant cell the same applies as has been set forth above in connection with the methods and the organisms, microorganisms, or plant cells according to the present invention.

Correspondingly, further disclosed is a composition comprising:
(a) oleacein; and
(b) a polypeptide having esterase activity as described herein and/or an enzyme which is categorized under the EC number 3.2.1 (glucosidase) or a (recombinant) organism, microorganism, or plant cell which expresses such (an) enzyme(s). In the context of the present invention, said polypeptide having an esterase activity is preferably an enzyme having an amino acid sequence as shown in SEQ ID NO: 7. Another example of a polypeptide having an esterase activity in the context of the present invention is an enzyme having the amino acid sequence as shown in SEQ ID NO: 24 or 37.

Further, in the context of the present invention, a composition is provided which comprises:
(a) decarboxymethyl elenolic acid dialdehyde (DEDA); and
(b) a polypeptide having esterase activity as described herein and/or an enzyme which is categorized under the EC number 3.2.1 (glucosidase) or a (recombinant) organism, microorganism, or plant cell which expresses such (an) enzyme(s). In the context of the present invention, said polypeptide having an esterase activity is preferably an enzyme having an amino acid sequence as shown in SEQ ID NO: 7. Another example of a polypeptide having an esterase activity in the context of the present invention is an enzyme having the amino acid sequence as shown in SEQ ID NO: 24 or 37.

As regards the enzyme which is categorized under the EC number 3.2.1 (glucosidase) and the organism, microorganism, or plant cell the same applies as has been set forth above in connection with the methods and the organisms, microorganisms, or plant cells according to the present invention.

Accordingly, the present disclosure also relates to a composition comprising oleocanthal and a recombinant organism, microorganism or plant cell as defined in the context of the present invention; or oleocanthal and a polypeptide having an esterase activity as defined herein.

Further, the present disclosure also relates to a composition comprising oleacein and a recombinant organism, microorganism or plant cell as defined herein; or oleacein and a polypeptide having an esterase activity as defined herein.

Furthermore, the present disclosure also relates to a composition comprising decarboxymethyl elenolic acid dialdehyde (DEDA) and a recombinant organism, microorganism or plant cell as defined herein; or decarboxymethyl elenolic acid dialdehyde (DEDA) and a polypeptide having an esterase activity as defined herein.

### The Figures show

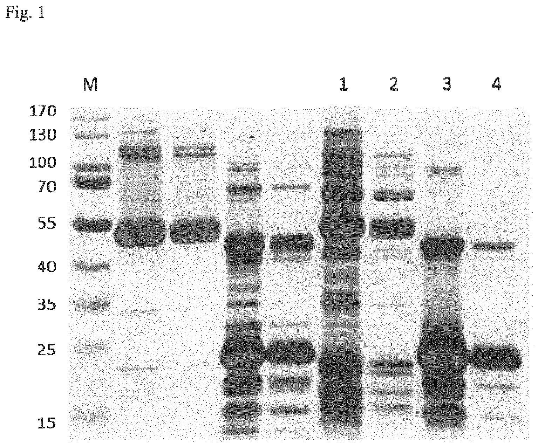

- **Figure 1:**: SDS-PAGE of both protein preparations visualized by silver nitrate staining. Preparation OeEst1 (SEQ ID NO: 11 as encoded by the nucleic acid molecule shown in SEQ ID NO: 10) (6.6 µg (1) and 1.3 µg (2)) exhibited the desired ~ 47 kDa protein while the negative control, the pET-32a(+) thioredoxin fusion protein (7.4 µg (3) and 1.5 µg (4); SEQ ID NO: 9 as encoded by the nucleic acid molecule shown in SEQ ID NO: 8), showed the expected 20 kDa protein band. Numbers to the left indicate molecular mass of marker proteins (M).
- **Figure 2:**: Schematic view of possible oleuropein or ligstroside degradation reactions occuring in olives. Names indicated are for R = hydroxytyrosol. If appropriate, names for substances with R = tyrosol (R') are given in brackets. Bold arrows show reaction catalyzed by the esterase Oest1 of the present invention.

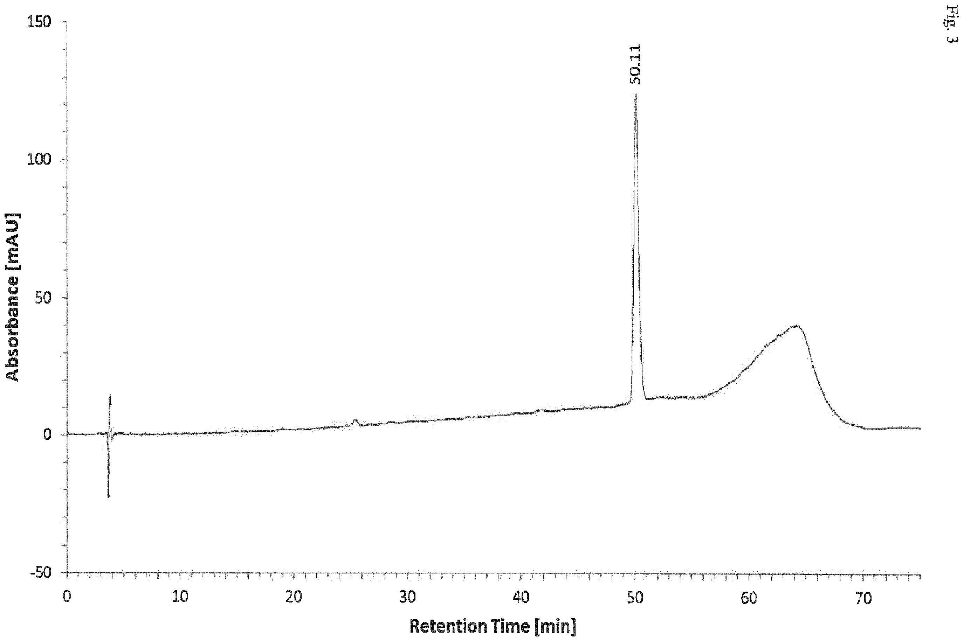

- **Figure 3:**: HPLC run of OeEst1 esterase (SEQ ID NO: 11 as encoded by SEQ ID NO: 10) assay with ligstroside (Rt, 50.11 min) as substrate.

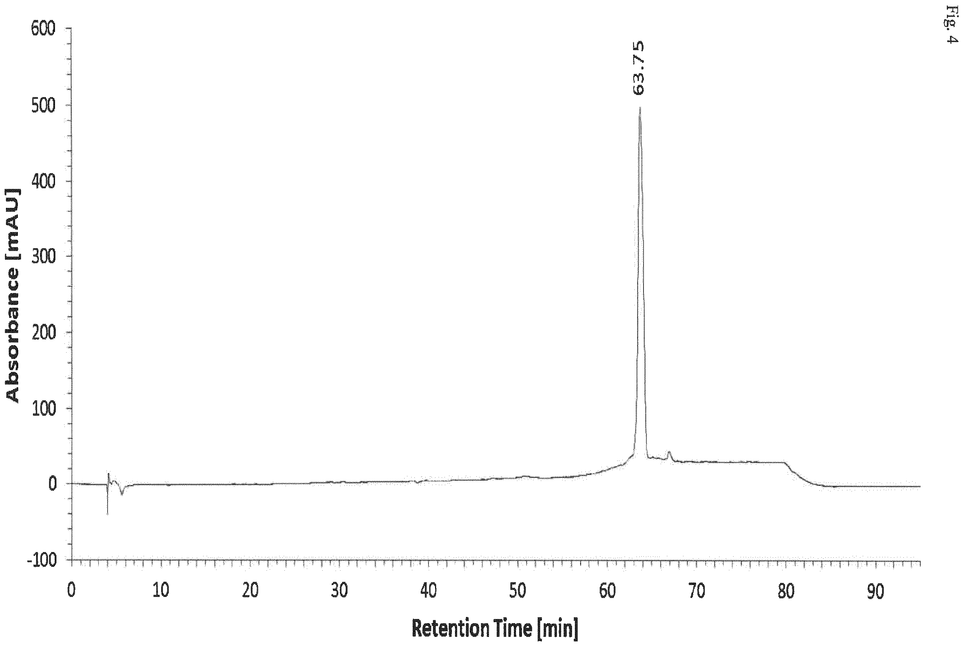

- **Figure 4:**: HPLC run of beta-Glucosidase (Sigma, catalogue number: G0395) assay with ligstroside as substrate. Ligstroside aglycone (Rt, 63.75 min) is formed (250-280 µg).

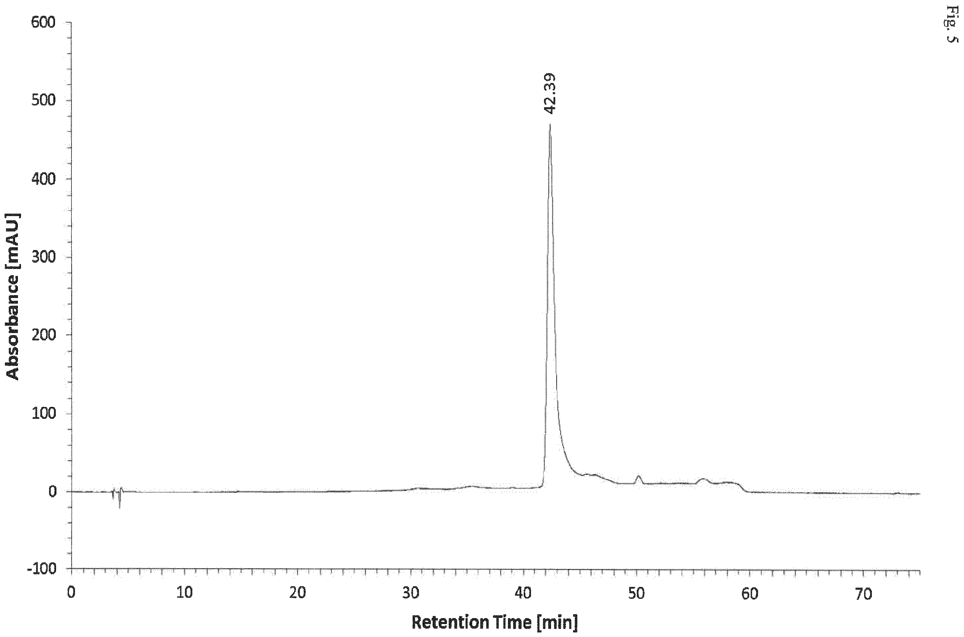

- **Figure 5:**: HPLC run of beta-Glucosidase (Sigma, catalogue number: G0395) + OeEst1 esterase (SEQ ID NO: 11 as encoded by SEQ ID NO: 10) assay with ligstroside as substrate. Oleocanthal (Rt, 42.39 min) is formed (yield 120 - 150 µg). Negative control experiments were performed with the thioredoxin fusion protein (SEQ ID NO 9 as encoded by SEQ ID NO 8) instead of OeEst1 fused to the thioredoxin tag (SEQ ID NO: 11 as encoded by SEQ ID NO: 10). No esterase activity was detectable in such a control assay.

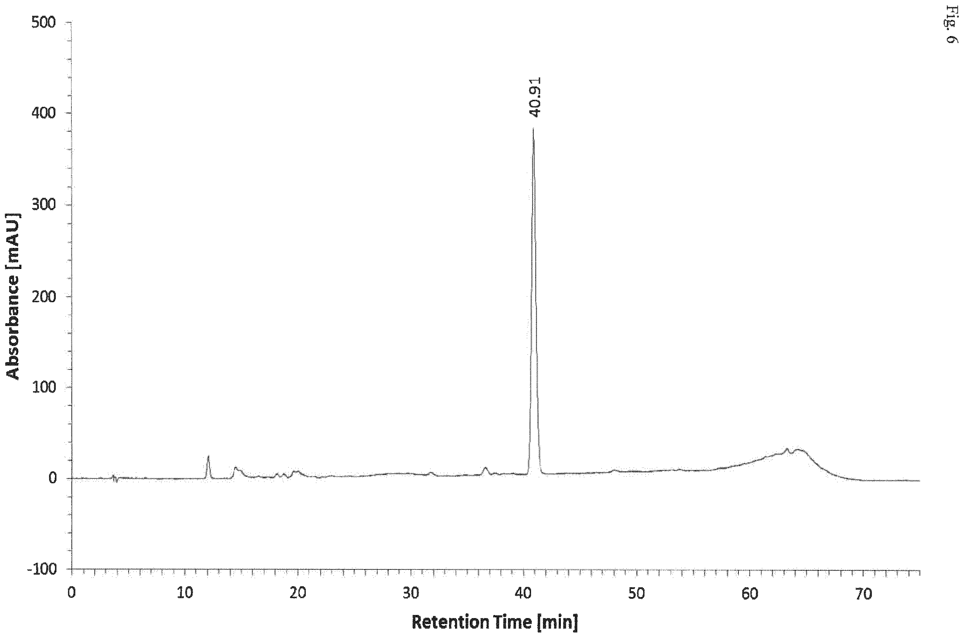

- **Figure 6:**: HPLC run of OeEst1 (SEQ ID NO: 11 as encoded by SEQ ID NO: 10) esterase assay with oleuropein (Rt, 40.91 min) as substrate. Dependent on the pH value, a hydrolysis to hydroxytyrosol and oleosid 11-methylester can be observed, independent of esterase presence.

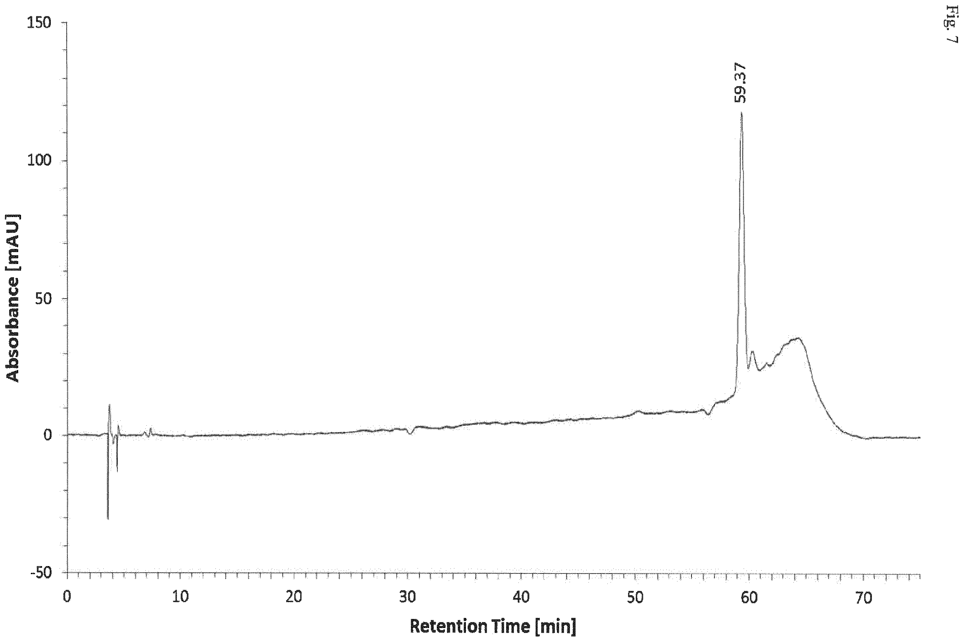

- **Figure 7:**: HPLC run of beta-Glucosidase (Sigma, catalogue number: G0395) assay with oleuropein as substrate. Oleuropein aglycone (Rt, 59.37 min) is formed (250-280 µg).

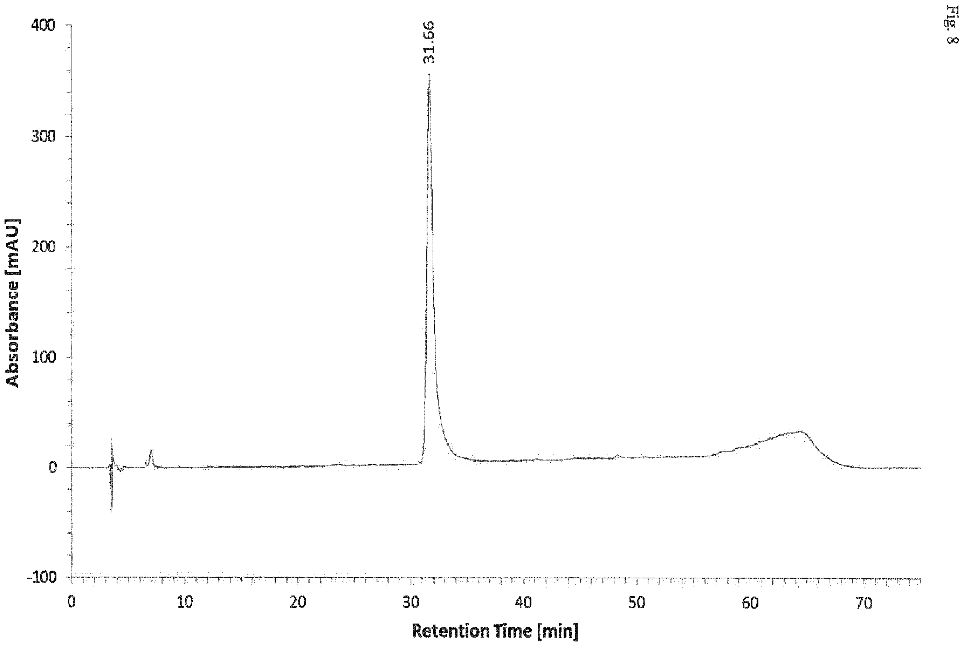

- **Figure 8:**: HPLC run of beta-Glucosidase (Sigma, catalogue number: G0395) + OeEst1 (SEQ ID NO: 11 as encoded by SEQ ID NO: 10) esterase assay with oleuropein as substrate. Oleacein (Rt, 31.66 min) is formed (120 - 150 µg). Negative control experiments were performed with the thioredoxin fusion protein (SEQ ID NO 9 as encoded by SEQ ID NO 8) instead of OeEst1 fused to the thioredoxin tag (SEQ ID NO: 11 as encoded by SEQ ID NO: 10). No esterase activity was detectable in such a control assay.

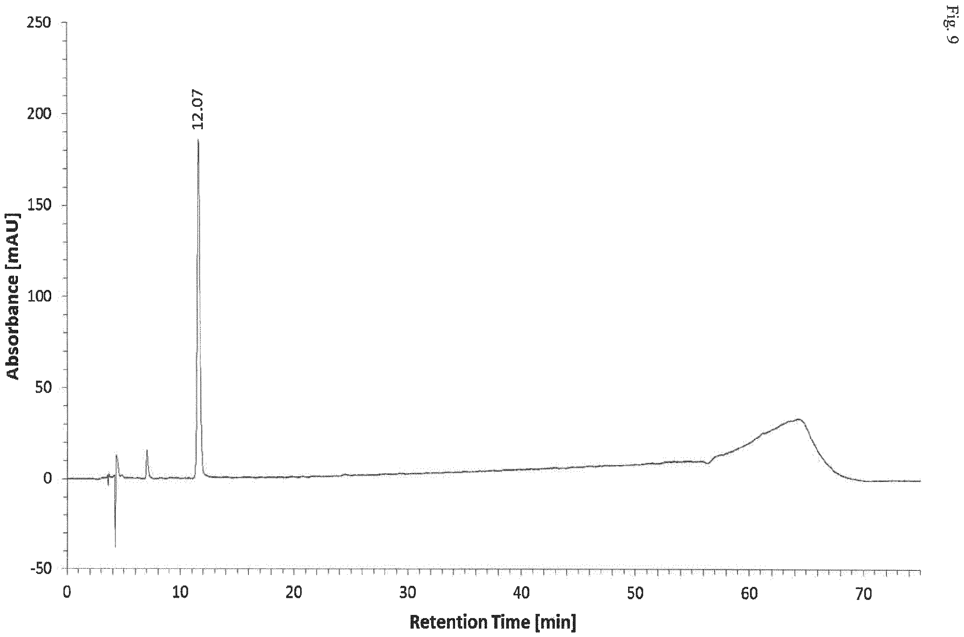

- **Figure 9:**: HPLC run of OeEst1 (SEQ ID NO: 11 as encoded by SEQ ID NO: 10) esterase assay with oleoside 11-methyl ester (Rt, 12.07 min) as substrate.

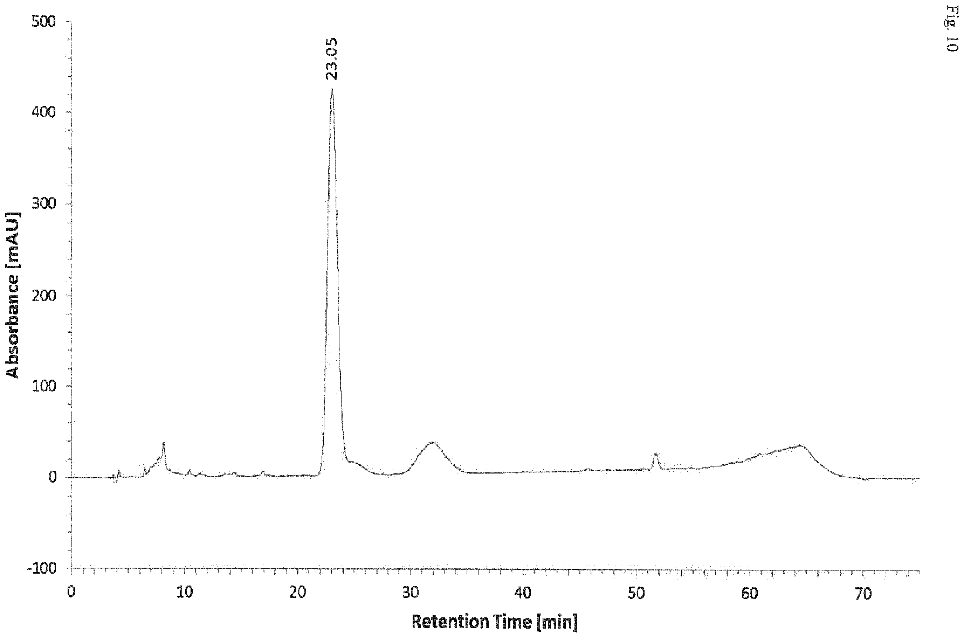

- **Figure 10:**: HPLC run of beta-Glucosidase assay with oleoside 11-methylester as substrate. Elenolic acid (Rt, 23.05 min) is formed (150 - 160 µg).

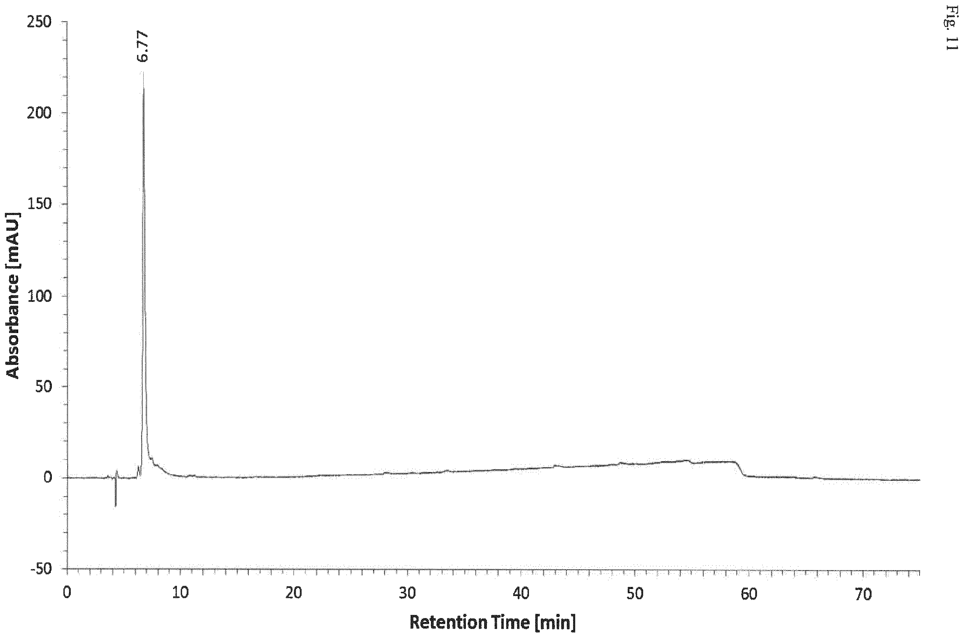

- **Figure 11:**: HPLC run of beta-Glucosidase (Sigma, catalogue number: G0395) + OeEst1 (SEQ ID NO: 11 as encoded by SEQ ID NO: 10) esterase assay with oleoside 11-methylester as substrate. Decarboxymethyl elenolic acid aldehyde (Rt, 6.77 min) is formed (70 - 90 µg).

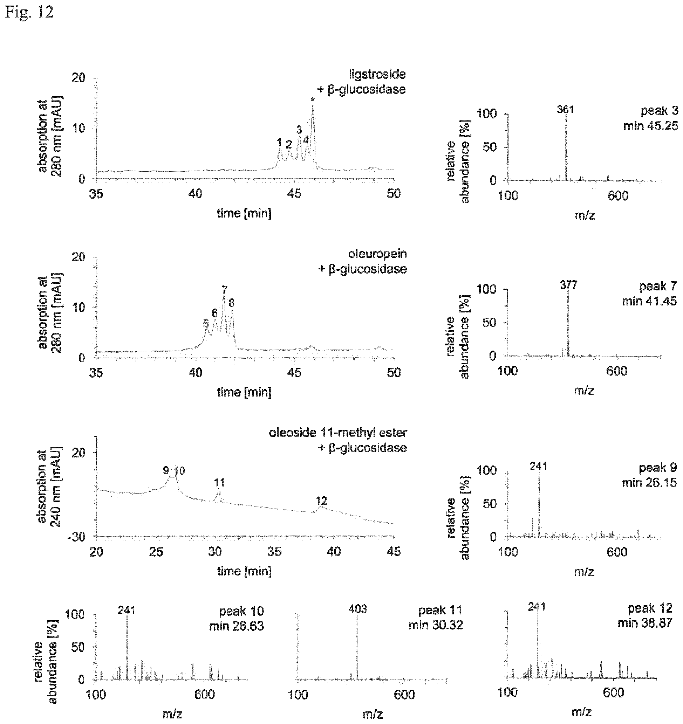

- **Figure 12:**: HPLC run of beta-Glucosidase (Sigma, catalogue number: G0395) assay with ligstroside, oleuropein and oleoside 11-methyl ester as substrate. Aglycone derivatives of ligstroside (peaks 1 to 4), oleuropein (peaks 5 to 8) and oleoside 11-methyl ester (peaks 9, 10, 12) are formed and analyzed by mass spectrometry by means of the negative ion mode. All aglycone derivatives derived from the same substrate featured highly similar mass spetra. Therefore, representative for peaks 1-4 and 5 - 8, only the mass spectra of peak 3 and 9 are shown. Oleoside 11-methyl ester, however, was not completely converted leading to the detection of remaining amounts of substrate (peak 11). The substance represented by the peak marked with an asterik is an impurity within the substrate preparation.

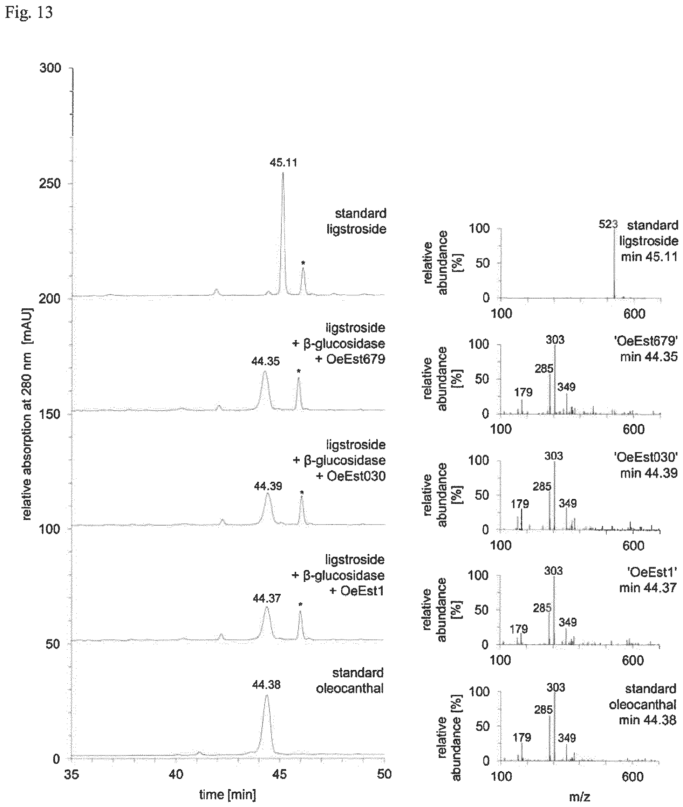

- **Figure 13:**: HPLC run of beta-Glucosidase (Sigma, catalogue number: G0395) + OeEst1 (SEQ ID NO: 11 as encoded by SEQ ID NO: 10), OeEst030 (SEQ ID NO: 26 as encoded by SEQ ID NO: 25), or OeEst679 (SEQ ID NO: 39 as encoded by SEQ ID NO: 38) esterase assay with ligstroside as substrate resulting in the formation of oleocanthal, which was confirmed by mass spectrometry. All mass spectra were generated using the negative ion mode. The substance represented by the peak marked with an asterik is an impurity within the substrate preparation.

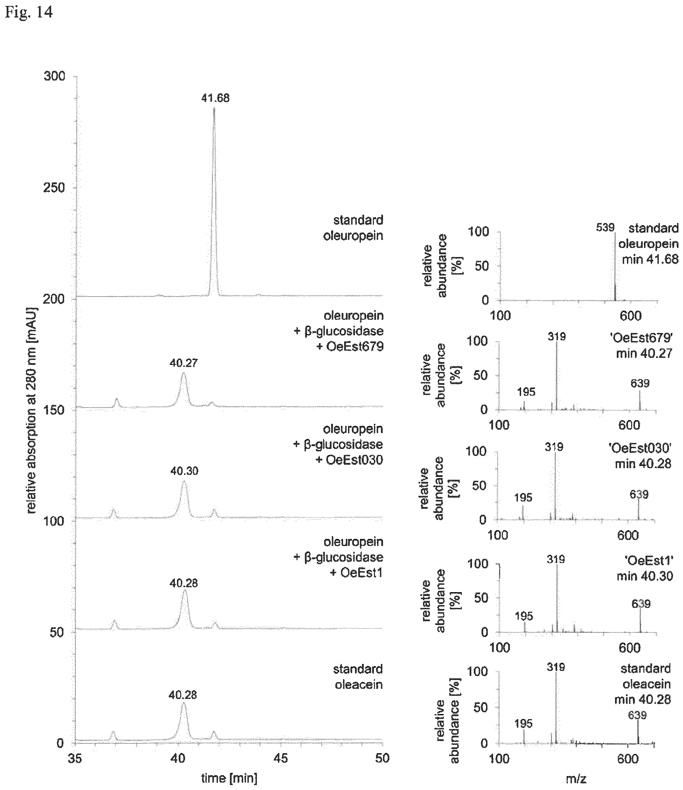

- **Figure 14:**: HPLC run of beta-Glucosidase (Sigma, catalogue number: G0395) + OeEst1 (SEQ ID NO: 11 as encoded by SEQ ID NO: 10), OeEst030 (SEQ ID NO: 26 as encoded by SEQ ID NO: 25), or OeEst679 (SEQ ID NO: 39 as encoded by SEQ ID NO: 38) esterase assay with oleuropein as substrate resulting in the formation of oleacein, which was confirmed by mass spectrometry. All mass spectra were generated using the negative ion mode.

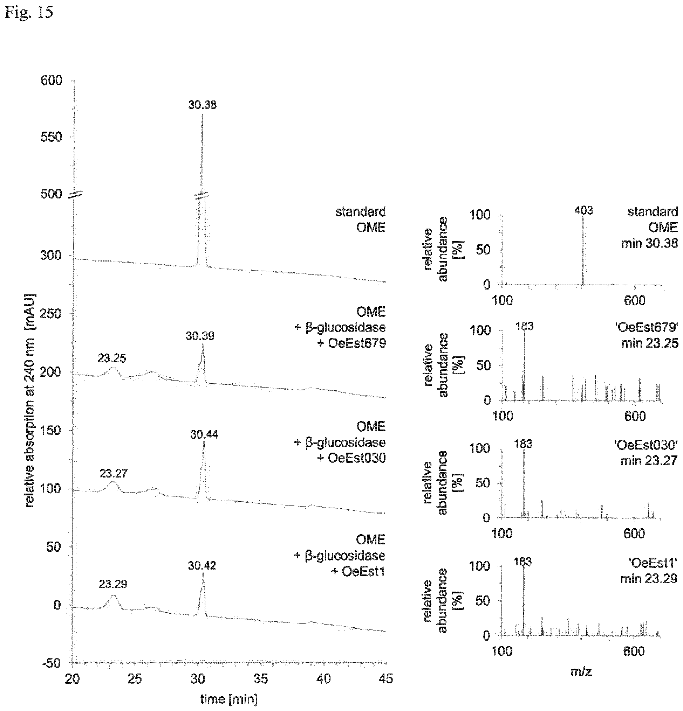

- **Figure 15:**: HPLC run of beta-Glucosidase (Sigma, catalogue number: G0395) + OeEst1 (SEQ ID NO: 11 as encoded by SEQ ID NO: 10), OeEst030 (SEQ ID NO: 26 as encoded by SEQ ID NO: 25), or OeEst679 (SEQ ID NO: 39 as encoded by SEQ ID NO: 38) esterase assay with oleoside 11-methyl ester as substrate resulting in the formation of a new product, which was analyzed by mass spectrometry. All mass spectra were generated using the negative ion mode.

### The following Examples illustrate the invention

Illustratively, in the following Examples, for the biochemical and enzymatic characterization of the newly identified OeEst1 esterase from *Olea europaea* a fusion protein which consists of the OeEst1 esterase (SEQ ID NO: 7 as encoded by SEQ ID NO: 5 or 6) and a thioredoxin tag was used. The amino acid sequence of the fusion protein is shown in SEQ ID NO: 11 (as encoded by the nucleic acid molecule shown in SEQ ID NO: 10). Further, Examples 3 and 4 of the Examples disclose isolation of and characterization of OeEst030 (SEQ ID NO: 24 as encoded by SEQ ID NO: 23) and OeEst679 (SEQ ID NO: 37 as encoded by SEQ ID NO: 36). The amino acid sequence of the fusion proteins are shown in SEQ ID NOs: 24 and 37. As explained herein below in the Examples, the polypeptide with the amino acid sequence of SEQ ID NO: 24 is a polypeptide having an esterase activity which is 69.3% identical to SEQ ID NO: 7. Further, the protein sequence of SEQ ID NO: 37 (as another example for a polypeptide having esterase activity which is at least 60% identical to the amino acid sequence of SEQ ID NO: 7) is 71.4% identical to the amino acid sequence of SEQ ID NO: 7. As will be shown in the appended Examples, both polypeptides (named OeEst030 (SEQ ID NO: 24) and OeEst679 (SEQ ID NO: 37)) are enzymes having an esterase activity which is comparable to OeEst1 (SEQ ID NO: 7).

### Example 1: Isolation and Purification of the OeEst1 Protein (SEQ ID NO: 7)

### 1.1 Plant material

Olive stones of an unknown variety of *Olea europaea* were purchased from 'Seedeo Samenraritäten' (Münster, Germany). The endocarp was cracked carefully and the embryo was sterilized for 15 min with 5 % sodium hypochlorite containing 0.05 % Tween-20. After several aseptic rinses with sterile distilled water seeds were placed on Rugini Olive solid medium (Duchefa Biochemie, Haarlem, The Netherlands, Cat. no. R0258; Rugini, Scientia Horticulturae, 24(2) (1984), 123-134 (doi:Doi 10.1016/0304-4238(84)90143-2) and incubated at 23°C under a 12 h photoperiod. After germination, seedlings were transferred to fresh Rugini Olive solid medium. Olive calli were induced from sterile seedlings using Gamborg B5 medium (Duchefa Biochemie, Haarlem, The Netherlands, Cat. no. G0210) supplemented with 2.26 µM 2,4-dichlorophenoxyacetic acid (Duchefa Biochemie, Haarlem, The Netherlands, Cat. no. D0911), 0.23 µM kinetin (SERVA Electrophoresis GmbH, Heidelberg, Germany, Cat. no.27190) and 2 % sucrose, and solidified with 7 g l⁻¹ phyto agar (Duchefa Biochemie, Haarlem, The Netherlands, Cat. no. P1003). Established calli were sub-cultured every 2 weeks under the same conditions as mentioned above.

### 1.2 Screening for coding sequences with potential esterase activity

The pool of transcriptome data of *Olea europaea* used for the screening for coding DNA sequences (CDSs) encoding potential esterases was published in 2016 by Cruz *et al.* (Cruz et al., Gigascience 5 (2016). doi:ARTN 29 10.1186/s13742-016-0134-5). The data set was analyzed for coding sequences sharing a high identity with the coding DNA sequence (CDS) of polyneuridine aldehyde esterase (PNAE) from *Rauvolfia serpetina* (SEQ ID NO: 2 (protein) and SEQ ID NO: 1 (CDS)). Amongst several candidate sequences with an identity ≥ 60 % the coding DNA sequence (CDS) with the denotation OE6A038228P1 (SEQ ID NO.: 4 (protein) and SEQ ID NO: 3 (CDS)) was selected and used for the design of appropriate primers. Sequence alignments were performed with the Basic Local Alignment Search Tool (BLAST) from the National Center for Biotechnology Information (NCBI) (Coordinators, Nucleic Acids Res, 44(D1) (2016), D7-19. doi:10.1093/nar/gkv1290).

### 1.3 Isolation of total RNA and cDNA synthesis

Total RNA was isolated from a callus culture of *Olea europaea* (unknown variety) as using the peqGOLD TriFast reagent (VWR International GmbH, Darmstadt, Germany, Cat. no. 30-2010). Therefore, the callus culture was ground in liquid nitrogen with a mortar and a pestle. Around 120 mg of the plant material was then treated according to the manufacturer's protocol. The quantification of the resulting RNA solution was accomplished by determination of the optical density at 260 nm using a Nanodrop ND-1000 device (Thermo Fisher Scientific, Waltham, USA), while the quality was estimated by formaldehyde agarose (FA) gel electrophoresis (Sambrook & Russell (2001), Molecular Cloning: A Laboratory Manual (3rd ed., Vol. 1, pp. 7.32 - 7.34). Cold Spring Harbor, N.Y.: Cold Spring Harbor Laboratory Press) using the fluorescent stain Nancy-520 (Sigma-Aldrich, St. Louis, MO, USA, Cat. no. EP01494). For the synthesis of cDNA from the isolated total RNA the RevertAID Reverse Transcriptase (Thermo Fisher Scientific) was used according to the provided protocol. Since the project was focused on a specific transcript the reaction was performed with the reverse primer OeEst228plain rev (5'-TTATTTTCCAACAATATCCAATATATATTGGCAAAGAGTGTTGGT-3' (SEQ ID NO: 13)).

### 1.4 Amplification and cloning of DNA fragments

The cDNA sample was amplified by PCR using *'Pfu* DNA Polymerase' (Thermo Fisher Scientific, Waltham, MA, USA, Cat. no. EP0501) and the oligonucleotide pair oligonucleotide pair OeEst228plain_fwd (5'-ATGGAAGCAAAGCAACAAATGCATTTTGTTCTC-3' (SEQ ID NO: 12)) and OeEst228plain_rev (5'-TTATTTTCCAACAATATCCAATATATATTGGCAAAGAGTGTTGGT-3' (SEQ ID NO: 13)). The resulting PCR product was analyzed by agarose gel electrophoresis (Sambrook & Russell (2001), Molecular Cloning: A Laboratory Manual (3rd ed., Vol. 1, pp. 5.04 - 5.16) and the expected fragment of 786 bp was extracted from gel using the 'PureLink Quick Gel Extraction Kit' (Thermo Fisher Scientific, Waltham, MA, USA, Cat. no. K2100-12) according to the manufacturer's protocol. Subsequently, the purified DNA-fragment was cloned into the high copy plasmid pCR^{®}-Blunt derived from the 'Zero Blunt PCR Cloning Kit' (Thermo Fisher Scientific, Waltham, MA, USA, Cat. no. K2750-20) via blunt-end ligation. The 10 µl ligation mix was used for the transformation of 50 µl competent cells of *E. Coli* TOP10 (Thermo Fisher Scientific, Waltham, MA, USA, Cat. no. C4040-03) by heat shock procedure (Sambrook & Russell (2001). Molecular Cloning: A Laboratory Manual (3rd ed., Vol. 1, pp. 1.118). Cold Spring Harbor, N.Y.: Cold Spring Harbor Laboratory Press.). Colonies tested positive by PCR with the aforementioned primers,OeEst228plain_fwd and OeEst228plain rev, were used to inoculate 4 ml of LB medium supplemented with 50 mg l⁻¹ kanamycin (Duchefa Biochemie, Haarlem, The Netherlands, Cat. no. K0126). After 16 - 18 h of incubation at 37°C and 160 rpm, cells were harvested by centrifugation at 17,000 ×*g* and room temperature (RT) for 1 min, followed by plasmid isolation by means of the 'E.Z.N.A. Plasmid DNA Mini Kit I' (Omega Bio-tek, Norcross, USA, Cat. no. D6943-02). Subsequently, purified plasmids were sequenced by Eurofins Genomics GmbH (Ebersberg, Germany) using the primers M13 uni (-21) (5'-TGTAAAACGACGGCCAGT-3' (SEQ ID NO: 14)) and M13 rev (-29) (5'-CAGGAAACAGCTATGACC-3' (SEQ ID NO: 15)). For further subcloning into the expression vector pET-32a(+) (Merck KGaA, Darmstadt, Germany, Cat. no. 69015-3; LaVallie et al., Biotechnology (N Y), 11(2) (1993), 187-193), it was necessary to add appropriate restriction sites to the coding sequence. Therefore, the CDS was amplified with the primers OeEst228*Nco*I_fwd (5'-TCACCATGGAAGCAAAGCAACAAATGCATTTTG-3' (SEQ ID NO: 16)) and OeEst228*Xho*I rev (5'-ATACCTCGAGTTATTTTCCAACAATATCCAATATATATTGGCAAAGA GTGTTGGGAT-3' (SEQ ID NO: 17)) adding the required *Ncol* and *Xhol* restriction sites to the 5'-end and the 3'-end, respectively. The PCR product was analyzed by agarose gel electrophoresis and the expected fragment of 801 bp was extracted from gel using the 'PureLink Quick Gel Extraction Kit' (Thermo Fisher Scientific, Waltham, MA, USA, Cat. no. K2100-12). The purified DNA fragment was cloned into pCR^{®}-Blunt and the resulting plasmid was then transformed into competent cells of *E. coli* TOP10, as described before. After 16 - 18 h of cultivation at 37°C, colonies harboring the constructed plasmid were identified by colony PCR using the primer pair OeEst228NcoI_fwd and OeEst228XhoI_rev. Positive clones were used to inoculate 4 ml LB medium supplemented with 50 mg l⁻¹ kanamycin. Plasmid DNA was isolated after 16 - 18 h of cultivation at 37°C and 160 rpm by means of the 'E.Z.N.A. Plasmid DNA Mini Kit I' (Omega Bio-Tek, Norcross, GA, USA, Cat. no. D6943-02). Subsequently, the inserted CDS was checked by sequencing. In a next step, an internal *Ncol* restriction site was removed by substitution of the base in position 51 of SEQ ID NO: 5 leading to a silent mutation. The exchange from thymine to cytosine could be achieved using the 'Q5 Site-Directed Mutagenesis Kit' (New England Biolabs GmbH, Ipswich, MA, USA, Cat. no. E0552S). The procedure was performed according to the provided manual using the primer pair OeEst_sdm1_fwd (5'-ATGGTATATGCCACGGAGCTTGGTGC-3' (SEQ ID NO: 18)) and OeEst sdm1 rev (5'-GTACGAGAACAAAGTGCCTTTGTTG-3' (SEQ ID NO: 19)) and competent cells of *E. coli* TOP10 as final host organism. The resulting CDS which contains a substitution at position 51 of SEQ ID NO: 5 is shown in SEQ ID NO: 6. Again, the resulting plasmid was checked by sequencing. The coding sequence was inserted between *Ncol* and *Xhol* sites of the pET-32a(+) vector and expressed in *E. coli* cells strain BL21(DE3) (New England Biolabs GmbH, Ipswich, MA, USA, Cat. no. C2527H).

### 1.5 Expression of OeEst1 and protein purification

*E. coli* cells strain BL21(DE3) harbouring the CDS of OeEst1 (SEQ ID NO: 6) within the pET-32a(+) vector, as well as *E. coli* BL21(DE3) cells containing an unmodified pET-32a(+) vector, were grown in 600 ml of LB medium (Carl Roth GmbH + Co. KG, Karlsruhe, Germany, Cat. no. X968.3; Bertani, J Bacteriol, 62(3) (1951), 293-300) supplemented with 100 µg ml⁻¹ ampicillin (Carl Roth GmbH + Co. KG, Karlsruhe, Germany, Cat. no. K029.2) at 25°C and 95 rpm, until an OD₆₀₀ of about 0.2. The expression of the transgene was then induced by addition of 0.4 mM of IPTG. After 16 - 18 h of further incubation under the same cultivation conditions, i.e. 600 ml LB + 100 Amp at 25°C bacteria cells were harvested by centrifugation at 5,000 × g and 4°C for 10 min and the resulting 3.4 g pellet was resuspended afterwards in 10.2 ml (3 volumes) of LEW buffer (50 mM NaH₂PO₄, 300 mM NaCl, pH 8.0). While kept on ice, the suspension was supplemented with 1 mM 4-(2-aminoethyl)benzenesulfonyl fluoride hydrochloride (AEBSF), 6 mM 3-[(3-Cholamidopropyl)dimethylammonio]-1-propanesulfonate (CHAPS) and 1 mg ml-1 of lysozyme, and incubated for 30 min on ice. The cells were then mechanically disrupted by sonication (6 × 10 s bursts with a 15 s cooling period between each step), mixed with 1 µg ml-1 DNaseI (Thermo Fisher Scientific, Waltham, MA, USA, Cat. no. AM2222) and incubated for further 20 min on ice. Afterwards, the crude lysate was centrifuged at 10,000 ×g and 4°C for 60 min. The resulting supernatant was loaded onto a Protino Column (Macherey-Nagel GmbH & Co. KG, Düren, Germany, Cat. no.745250.10) packed with 1 g of Protino Ni-TED Resin (Macherey-Nagel GmbH & Co. KG, Düren, Germany, Cat. no. 745200) for purification of soluble polyhistidine-tagged protein via immobilized metal ion affinity chromatography (IMAC) according to the manufacturer's protocol. All collected fractions were analyzed for the presence of the protein of interest by SDS-PAGE with following Coomassie Brilliant Blue staining (Sambrook & Russell (2001). Molecular Cloning: A Laboratory Manual (3rd ed., Vol. 3, pp. A8.40 - A48.47). Cold Spring Harbor, N.Y.: Cold Spring Harbor Laboratory Press.). Corresponding elution fractions were combined, desalted and concentrated with a Vivaspin^{®} Turbo 15 ultrafiltration device (Sartorius AG, Göttingen, Germany, Cat. no. VS15T02). The final enzyme preparation was recovered in sodium phosphate buffer (100 mM, pH 7.4) and analyzed again using SDS-PAGE visualized by silver nitrate staining (Sambrook & Russell (2001). Molecular Cloning: A Laboratory Manual (3rd ed., Vol. 3, pp. A8.47 - A48.49). Cold Spring Harbor, N.Y.: Cold Spring Harbor Laboratory Press). Determination of protein concentration was performed with the 'BCA Protein Assay Kit' (Thermo Fisher Scientific, Waltham, MA, USA, Cat. no. 23225), using bovine serum albumin (BSA) as a standard. In order to store aliquots of the protein preparations at -80°C, 25 % (v/v) glycerol was added.

### 1.6 Results

To date there is no coding sequence (CDS) of a functional esterase from *Olea europaea* published that could have been used for the identification of potential candidate sequences. Thus, the initial screening for candidates had to be performed using the coding sequence (CDS) of an esterase derived from another species. A well characterized enzyme able to hydrolyze the methylester of a compound structural similar to the olive oleosides ligstroside and oleuropein is the polyneuridine aldehyde esterase (PNAE) (Dogru et al., Eur J Biochem, 267(5) (2000), 1397-1406). PNAE, first isolated in 1983 from R. *serpentina* (LIT), catalyzes the transformation of polyneuridine aldehyde (PNA) into epi-vellosimine making it a key enzyme in the ajmaline biosynthesis. The structural similarities of polyneuridine aldehyde and the olive oleosides are the result of a common intermediate, 7-deoxyloganic acid, responsible for the irioid moiety in the aforementioned compounds. Using the coding sequence of the reference enzyme, transcriptome data of *O*. *europaea* were screened for highly homologous sequences. One of the identified candidates denoted within the data set as OE6A038228P1 possessed 60.5 % identity with the cDNA of PNAE. An alignment of the corresponding protein sequences showed 51.4 % identity and 70.0 % similarity. It has to be noted that an expressed sequence tag sequence obtained from an olive leaves and fruits extract (EMBL database accession no. GO245644; SEQ ID NO: 69) was found to be 97 % identical to OeEst1 on the nucleic acid molecule level, i.e. 97.5 % in case of SEQ ID NO: 5 and 97.7 % in case of SEQ ID NO: 6. However, no information was available to date with respect to the function of a polypeptide being encoded by this expressed sequence tag sequence. Based on the reference sequence OE6A038228P1 primers were designed and used for the synthesis of cDNA as well as for the subsequent DNA amplification. The corresponding protein sequence of the isolated candidate, hereinafter referred to as OeEst1, shared 99.6 % identity with the protein encoded by OE6A038228P1. A subsequent homology search against the protein database of NCBI showed also a high identity to the salicylic acid-binding protein 2 (SABP2) from various origins. An alignment of OeEst1 and the SABP2 from *Nicotiana tabacum* (Kumar et al., Proc. Natl. Acad. Sci. USA 100(26) (2003), 16101-16106; WO 2013/101339 A1; SEQ ID NO: 71), for example, revealed an identity of 54.5 % as well as the alignment of OeEst1 with Glymal6g26060 from *Glycine max* an identity of 50.2 % (WO 2013/101339 A1; SEQ ID NO: 70).

For heterologous expression in cells of *E. coli* strain BL21(DE3) and following purification via immobilized metal ion affinity chromatography (IMAC) the coding sequence was cloned into the pET-32a(+) vector. Thus, the inducible expression resulted in the biosynthesis of the recombinant protein fused with an N-terminal thioredoxin/polyhistidine tag leading to a protein with a size of around 46.3 kDa. After successful purification and desalting the concentration of the protein preparation was determined to be 1 mg mL⁻¹.

In addition, to serve as a control in all further experiments, the fusion peptide produced by the unmodified pET-32a(+) vector, was expressed and the resulting protein purified the same way.

### Example 2: Determination of enzyme activity towards olive polyphenols

For activity assays, purified OeEst 1 esterase (SEQ ID NO: 11 as encoded by SEQ ID NO: 10) was incubated with various substrates, i.e. ligstroside, oleuropein, and oleosid 11-methylester. During incubation, no conversion of ligstroside, oleuropein or oleosid 11 methylester could be detected.

In a second set of experiments, substrates were either incubated with a beta-glucosidase (Sigma, catalogue number: G0395) releasing the glucose moiety or co-incubated with esterase OeEst1 (SEQ ID NO: 11) and beta-glucosidase (Sigma, catalogue number: G0395). While the sole incubation with beta-glucosidase releases the respective aglycone, addition of esterase to the reaction mixture yielded in the formation of a new product (see Figures 3 to 11).

**Table 1: Different assay conditions tested. NaPB = 50 mM NaPhosphate buffer, NaAcB = 50 mM NaAcetate buffer, RT = room temperature, ^{∗} = also lower concentrations down to 2 µg.**

| | **Ligstroside** | **Oleuropein** | **Oleosid -11- Methylester** | **OeEst1** | **beta-Glu** | **pH** | **Buffer** | **Temperature** | **Time** |
|---|---|---|---|---|---|---|---|---|---|
| **Assay 1** | 400 µg | - | - | 100 µg | - | 7.2 | NaPB | 50° C, RT | 60 min, 24 h |
| **Assay 2** | - | 400 µg | - | 100 µg | - | 7.2 | NaPB | 50° C, RT | 60 min, 24 h |
| **Assay 3** | - | - | 400 µg | 100 µg | - | 7.2 | NaPB | 50° C, RT | 60 min, 24 h |
| **Assay 4** | 400 µg | - | - | - | 10 µg | 5 | NaAcB | 50° C | 60 min |
| **Assay 5** | - | 400 µg | - | - | 10 µg | 5 | NaAcB | 50° C | 60 min |
| **Assay 6** | - | - | 400 µg | - | 10 µg | 5 | NaAcB | 50° C | 60 min |
| **Assay 7** | 400 µg | - | - | 100 µg^{∗} | 10 µg | 5 | NaAcB | 50° C | 60 min |
| **Assay 8** | - | 400 µg | - | 100 µg^{∗} | 10 µg | 5 | NaAcB | 50° C | 60 min |
| **Assay 9** | - | - | 400 µg | 100 µg | 10 µg | 3.5-5 | NaAcB | 50° C | 60 min |

For the determination of the enzymatic reactions, the samples are diluted to achieve an end concentration of 50% methanol in water, and are centrifuged. The supernatant is transferred to a HPLC vessel by a syringe filter (0.2 µm). Seco-iridoid derivatives are analyzed by RP-HPLC-DAD (Agilent System). Separation of the seco-iridoid derivatives is carried out on a reversed-phase C18 column (Phenomenex, Luna 5u C18 10A; 250 × 4.6 mm) using a solvent gradient of 0.1% TFA in water (solvent A) and 0.1% TFA in 90% MeOH (solvent B) at a constant temperature of 25°C.10 µl of the sample were added to the system via an auto sampler (Agilent) and eluated at 0.5 ml/min with the following gradient: 0 min 30% solvent B, 50 min 50% solvent B, 55 min 60% solvent B, 60 min 70% solvent B, 65 min 30% solvent B. Detection was at 233 nm and 240 nm. The content of ligstroside and oleuropein was determined as peak area at a detection of 233 nm. In case derivatives of ligstroside and oleuropein were to be determined, this was done by detection at 240 nm. The analysis of OP / hydroxytyrosol (HT) / tyrosol (T) was done by external standards available by Extrasynthese, Genay Cedex, France. Further reference substances were extracted from olive oil based on Montedoro et al., J. Agric. Food Chem. 41 (1993), 2228-2234, Impellizzeri and Lin, J. Agric. Food Chem. 54 (9) (2006), 3204-3208, Carrasco-Pancorbo et al., J. Sep. Sci. 29 (14) (2006) 2221-2233.

### Example 3: Isolation and Purification of OeEst030 (SEQ ID NO: 24) and OeEst679 (SEQ ID NO: 37)

### 3.1 Screening for coding sequences with potential esterase activity

As described in Example 1, the data set published in 2016 by Cruz *et al.* (Cruz et al., Gigascience 5 (2016). doi:ARTN 29 10.1186/s13742-016-0134-5) was analyzed for coding sequences sharing a high identity with the CDS of PNAE (SEQ ID NO: 2 and SEQ ID NO: 1). Amongst sequences with an identity ≥ 55 %, the coding DNA sequences (CDS) with the denotation OE6A059030P1 (SEQ ID NO: 21 (protein) and SEQ ID NO: 20 (CDS)) and OE6A068679P2 (SEQ ID NO: 34 (protein) and SEQ ID NO: 33 (CDS)) were selected and used for the design of appropriate primers.

### 3.2 Isolation of total RNA and cDNA synthesis

Total RNA from leaf tissue of *Olea europaea* L. cv. Chemlali was isolated, reverse transcribed and amplified as described in Example 1. Synthesis of cDNA and amplification was performed with the forward primer OeEst030plain_fwd (SEQ ID NO: 27) and the reverse primer OeEst030plain_rv (SEQ ID NO: 28) and the forward primer OeEst679plain_fwd (SEQ ID NO: 40) and the reverse primer OeEst679plain_rev (SEQ ID NO: 41), respectively.

### 3.3 Cloning of DNA fragments and expression of OeEst030 and OeEst679

Cloning of the DNA fragments was performed in accordance with Example 1 except that the OeEst030 sequence (SEQ ID NO: 22) was amplified with the primers OeEst030*Nco*I_fwd (SEQ ID NO: 29) and OeEst030*Xho*I rev (SEQ ID NO: 30) adding the required *Ncol* and *Xhol* restriction sites to the 5'-end and the 3'-end, respectively. Similarly, the sequence of OeEst679 (SEQ ID NO: 35) was modified by PCR using the oligonucleotide sequences OeEst679*Nco*I fwd (SEQ ID NO: 42) and OeEst679*Hind*III rev (SEQ ID NO: 43). Furthermore, an internal *Ncol* restriction site was removed by substitution of the base in position 51 of SEQ ID NO: 22 and in position 51 of OeEst679 (SEQ ID NO: 35) leading to a silent mutation by exchanging thymine to cytosine using the primer pair OeEst030sdm fwd (SEQ ID NO: 31) and OeEst030sdm_rev (SEQ ID NO: 32) and the primer pair OeEst679sdm_fwd (SEQ ID NO: 44) and OeEst679sdm_rev (SEQ ID NO: 45), respectively. The resulting OeEst030sdm CDS shown in SEQ ID NO: 23 was inserted between *Nco*I and *Xho*I sites of the pET-32a(+) vector resulting in the open reading frame of SEQ ID NO. 25. Corresponding protein (SEQ ID NO: 26) was produced and purified the same way as described in Example 1. In contrast, the OeEst679sdm CDS was inserted between *Ncol* and *Hind*III sites of the pET-32a(+) expression vector. The resulting open reading frame of SEQ ID NO: 38 was also expressed and the corresponding protein (SEQ ID NO: 39) purified and characterized as described in Example 1.

### 3.4 Results

In accordance with Example 1, OE6A059030P1 and OE6A068679P2 were identified as further candidate genes based on a homology screen of published transcriptome data of *O. europaea* using the coding sequence of the reference enzyme PNAE. The protein sequence of the isolated candidate **OeEst030** shared 100 % identity with the protein encoded by OE6A059030P1. The amino acid sequence of OeEst030 (SEQ ID NO: 24) was 69.3 % identical and 81.2 % similar to the amino acid sequence of OeEst1 (SEQ ID NO: 7). The protein sequence of the isolated candidate OeEst679 shared 99.2 % identity with the protein encoded by OE6A068679P2. The amino acid sequence of OeEst679 (SEQ ID NO: 37) was 71.4 % identical and 83.8 % similar to the amino acid sequence of OeEst1 (SEQ ID NO: 7) and was 76.4 % identical and 86.5 % similar to the amino acid sequence of OeEst030 (SEQ ID NO: 24).

On the nucleic acid molecule level, OeEst1 (SEQ ID NO: 5) was 84.9 % identical to OeEst030 (SEQ ID NO: 22) and 85.5 % identical to OeEst679 (SEQ ID NO: 35), whereas OeEst030 (SEQ ID NO: 22) was 88.2 % identical to OeEst679 (SEQ ID NO: 35).

### Example 4: Determination of enzyme activity towards olive polyphenols

### 4.1 Methods

Assays verifying specific esterase activity towards olive polyphenols were performed using the substrates ligstroside, oleuropein and oleoside 11-methyl ester. Reaction mixtures (100µL) contained 50 mM sodium phosphate (pH 7.4), 0.5 mM of the aforementioned seco-iridoids and 100 µg/mL of recombinant protein. Enzyme activity assays were incubated at 30 °C for one hour and subsequently stopped by addition of 2 volumes of ice-cold methanol. Precipitated proteins were removed by centrifugation for 20 min at 17,000 ×g and 4 °C. Resulting supernatant was analyzed by LC-MS.

Also, the aglycon forms of the aforementioned seco-iridoids were tested as substrates for the purified enzymes. Therefore, reaction mixtures were buffered in 50 mM sodium citrate (pH 5.0) and additionally supplemented with 1 mg/mL of beta-glucosidase from almonds (Sigma Aldrich, Darmstadt, Germany).

**Table 2: Different assay conditions tested.**

| | **substrate** | **recombinant protein** | **beta-glucosidase** | **pH** | **buffer** |
|---|---|---|---|---|---|
| **Assay 10** | ligstroside | - | - | 7.4 | sodium phosphate |
| **Assay 11** | ligstroside | OeEst679 | - | 7.4 | sodium phosphate |
| **Assay 12** | ligstroside | OeEst030 | - | 7.4 | sodium phosphate |
| **Assay 13** | ligstroside | OeEst1 | - | 7.4 | sodium phosphate |
| **Assay 14** | oleuropein | - | - | 7.4 | sodium phosphate |
| **Assay 15** | oleuropein | OeEst679 | - | 7.4 | sodium phosphate |
| **Assay 16** | oleuropein | OeEst030 | - | 7.4 | sodium phosphate |
| **Assay 17** | oleuropein | OeEst1 | - | 7.4 | sodium phosphate |
| **Assay 18** | oleoside 11-methyl ester | - | - | 7.4 | sodium phosphate |
| **Assay 19** | oleoside 11-methyl ester | OeEst679 | - | 7.4 | sodium phosphate |
| **Assay 20** | oleoside 11-methy lester | OeEst030 | - | 7.4 | sodium phosphate |
| **Assay 21** | oleoside 11-methyl ester | OeEst1 | - | 7.4 | sodium phosphate |
| **Assay 22** | ligstroside | - | + | 5.0 | sodium citrate |
| **Assay 23** | oleuropein | - | + | 5.0 | sodium citrate |
| **Assay 24** | oleoside 11-methyl ester | - | + | 5.0 | sodium citrate |
| **Assay 25** | ligstroside | OeEst679 | + | 5.0 | sodium citrate |
| **Assay 26** | ligstroside | OeEst030 | + | 5.0 | sodium citrate |
| **Assay 27** | ligstroside | OeEst1 | + | 5.0 | sodium citrate |
| **Assay 28** | oleuropein | OeEst679 | + | 5.0 | sodium citrate |
| **Assay 29** | oleuropein | OeEst030 | + | 5.0 | sodium citrate |
| **Assay 30** | oleuropein | OeEst1 | + | 5.0 | sodium citrate |
| **Assay 31** | oleoside 11-methylester | OeEst679 | + | 5.0 | sodium citrate |
| **Assay 32** | oleoside 11-methylester | OeEst030 | + | 5.0 | sodium citrate |
| **Assay 33** | oleoside 11-methylester | OeEst1 | + | 5.0 | sodium citrate |

The instrument used for liquid chromatography was a 1260 Infinity System (Agilent Technologies, Santa Clara, CA, USA) consisting of the following components: G4225A high performance degasser, G1312B binary pump, G1329B autosampler, G1316C column thermostat, G4212B diode array detector. Samples were injected onto a ZORBAX Eclipse Plus C18 analytical column (4.6 × 250 mm, 5 µm) and separated using a solvent composition of 0.1% formic acid in ddH₂O (solvent A) and methanol (solvent B). The flow rate was set to 0.5 ml/min and the column temperature was adjusted to 30°C. Analysis was performed using the following gradient (solvent B): 10 min isocratic with 10 %, 60 min from 10 % to 90 %, 10 min isocratic with 100 % and again 20 min with 10 % to re-equilibrate the column. Eluting compounds were detected at 240 and 280 nm and further analyzed by mass spectrometry using a 6120 Single Quadrupole (Agilent Technologies, Santa Clara, CA, USA) applying the following parameters: atmospheric pressure ionization - electrospray ionization (API-ESI), negative ion mode, N2 as nebulizing gas at a flow rate of 12 L/min under a pressure of 35 psig, drying gas temperature of 350°C and capillary voltage of 3 kV.

### 4.2 Results

No enzymatic activity of OeEst030 and OeEst679 towards the seco-iridoids ligstroside (Assay 10 - 13), oleuropein (Assay 14 - 17) and oleoside 11-methyl ester (Assay 18 - 21) could be detected.

The incubation of the aforementioned substrates with beta-glucosidase from almonds resulted in the formation of corresponding aglycones (see Figure 12). Hydrolysis of the glucosidic bond within ligstroside (Assay 22) led to the formation of four peaks detectable with the applied LC-MS method at min 44.27, 44.74, 45.25 and 45.66. Mass spectrometry analysis of corresponding compounds revealed a major ion of 361 [M - H]⁻ as determined by means of negative ion mode indicating the successful generation of different derivatives of ligstroside aglycone. Similarly, deglucosylation of oleuropein (Assay 23) led to different derivatives of oleuropein aglycone represented by four peaks at min 40.59, 41.00, 41.45 and 41.86 featuring a major ion of 377 [M - H]⁻ when measured by mass spectrometry in the negative ion mode. Analysis of the conversion products of oleoside 11-methyl ester (Assay 24) revealed the presence of two peaks eluting at min 26.15 and 26.63 as well as a third peak at min 38.87. Also in this case, all detected peaks share the same major ion, 241 [M - H]⁻, leading to the assumption that corresponding substances are derivatives of elenolic acid. Furthermore, a peak featuring an elution time of 30.33 min and a m/z of 403 [M - H]⁻ was detectable, indicating remaining amounts of oleoside 11-methyl ester.

Addition of OeEst030 or OeEst679 to the assays containing beta-glucosidase and the aforementioned substrates, however, resulted in the formation of new products. The substance produced from ligstroside (Assay 25 - 27) eluted at min 44.4 featuring a major peak of 303 [M - H]⁻ in negative ion mode (see Figure 13), while the assays containing oleuropein (Assays 28 - 30) yielded a compound with a retention time of 40.3 min and a major ion of 319 [M - H]⁻(see Figure 14). By comparison with authentic standards, it could be verified that ligstroside and oleuropein were successfully converted into oleocanthal and oleacein, respectively.

Conversion of elenolic acid by esterase activity (Assays 31 - 33) resulted in a product eluting at 23.3 min (see Figure 15). Mass spectrometry analysis revealed a major ion of 183 [M - H]⁻ indicating the production of decarboxymethyl elenolic acid dialdehyde. However, lacking an authentic standard, it was not possible to confirm this assumption. Slight differences were observed between profiles and retention times of HPLC runs shown in Example 2 and Example 4 as different plattforms were used.

## Claims

1. A polypeptide selected from the group consisting of:
(a) a polypeptide comprising the amino acid sequence of SEQ ID NO: 7;
(b) a polypeptide that is at least 69% identical to the amino acid sequence of SEQ ID NO: 7 and which is **characterized by** having an esterase activity to convert (i) an oleuropein aglycone into oleacein, (ii) a ligstroside aglycone into oleocanthal, or (iii) elenolic acid to decarboxymethyl elenolic acid dialdehyde (DEDA);
(c) a polypeptide encoded by the nucleic acid molecule of SEQ ID NO: 5 or 6;
(d) a polypeptide encoded by a nucleic acid molecule which is at least 69% identical to the nucleic acid sequence of SEQ ID NO: 5 or 6 and wherein said polypeptide is **characterized by** having an esterase activity to convert (i) an oleuropein aglycone into oleacein, (ii) a ligstroside aglycone into oleocanthal, or (iii) elenolic acid to decarboxymethyl elenolic acid dialdehyde (DEDA); and
(e) a polypeptide encoded by the complementary sequence of a nucleic acid molecule that is able to hybridize with the nucleic acid molecule of SEQ ID NO: 5 or 6 and wherein said polypeptide is **characterized by** having an esterase activity to convert (i) an oleuropein aglycone into oleacein, (ii) a ligstroside aglycone into oleocanthal, or (iii) elenolic acid to decarboxymethyl elenolic acid dialdehyde (DEDA).

2. A nucleic acid molecule expressing an esterase as defined in claim 1.

3. A vector comprising the nucleic acid molecule as defined in claim 1 or 2.

4. A recombinant organism, microorganism or plant cell expressing an esterase encoded by a nucleic acid as defined in claim 1 or 2, or by the vector of claim 3.

5. The recombinant organism, microorganism or plant cell of claim 4 which further expresses an enzyme which is categorized under the EC number 3.2.1 (glucosidase).

6. The recombinant organism, microorganism or plant cell according to claim 5, wherein the enzyme is capable of converting oleuropein into an oleuropein aglycone.

7. The recombinant organism, microorganism or plant cell according to claim 5, wherein the enzyme is capable of converting ligstroside into a ligstroside aglycone.

8. The recombinant organism, microorganism or plant cell according to claim 5, wherein the enzyme is capable of converting oleoside-11-methylester into elenolic acid.

9. Use of a recombinant organism, microorganism or plant cell as defined in any one of claims 4 to 8 for the production of oleacein, oleocanthal, or decarboxymethyl elenolic acid dialdehyde (DEDA).

10. The plant cell according to any one of claims 4 to 9, wherein the plant cell belongs to the family *Oleaceae.*

11. Use of an esterase as defined in claim 1 for the production of oleacein from an oleuropein aglycone.

12. Use of an esterase as defined in claim 1 for the production of oleocanthal from a ligstroside aglycone.

13. Use of an esterase as defined in claim 1 for the production of decarboxymethyl elenolic acid dialdehyde (DEDA) from elenolic acid.

14. A method for the production of oleacein comprising the enzymatic conversion of an oleuropein aglycone into oleacein, wherein said method comprises the use of an esterase as defined in claim 1.

15. A method for the production of oleocanthal comprising the enzymatic conversion of a ligstroside aglycone into oleocanthal, wherein said method comprises the use of an esterase as defined in claim 1.

16. A method for the production of decarboxymethyl elenolic acid dialdehyde (DEDA) comprising the enzymatic conversion of elenolic acid, wherein said method comprises the use of an esterase as defined in claim 1.

## Patentansprüche

1. Polypeptid, das ausgewählt ist aus der Gruppe bestehend aus:
(a) Polypeptid, das die Aminosäuresequenz von SEQ ID NO:7 umfasst;
(b) Polypeptid, das zu mindestens 69% mit der Aminosäuresequenz von SEQ ID NO:7 identisch ist und das **dadurch gekennzeichnet ist, dass** es eine Esterease-Aktivität aufweist, um (i) ein Oleuropein-Aglycon in Oleacein, (ii) ein Ligstrosid-Aglycon in Oleocanthal oder (iii) Elenolsäure in Decarboxymethyl-Elenolsäure-Dialdehyd (DEDA) umzusetzen;
(c) Polypeptid, das durch das Nucleinsäuremolekül von SEQ ID NO:5 oder 6 codiert wird;
(d) Polypeptid, das durch ein Nucleinsäuremolekül codiert wird, das zu mindestens 69% mit der Nucleinsäuresequenz von SEQ ID NO:5 oder 6 identisch ist, und wobei das Polypeptid **dadurch gekennzeichnet ist, dass** es eine Esterease-Aktivität aufweist, um (i) ein Oleuropein-Aglycon in Oleacein, (ii) ein Ligstrosid-Aglycon in Oleocanthal oder (iii) Elenolsäure in Decarboxymethyl-Elenolsäure-Dialdehyd (DEDA) umzusetzen; und
(e) Polypeptid, das durch eine Komplementärsequenz eines Nucleinsäuremoleküls codiert wird, das fähig ist, mit dem Nucleinsäuremolekül von SEQ ID NO:5 oder 6 zu hybridisieren, und wobei das Polypeptid **dadurch gekennzeichnet ist, dass** es eine Esterease-Aktivität aufweist, um (i) ein Oleuropein-Aglycon in Oleacein, (ii) ein Ligstrosid-Aglycon in Oleocanthal oder (iii) Elenolsäure in Decarboxymethyl-Elenolsäure-Dialdehyd (DEDA) umzusetzen.

2. Nucleinsäuremolekül, dass eine wie in Anspruch 1 definierte Esterase exprimiert.

3. Vektor, der ein wie in Anspruch 1 oder 2 definiertes Nucleinsäuremolekül umfasst.

4. Rekombinanter Organismus, rekombinanter Mikroorganismus oder rekombinante Pflanzenzelle, die eine Esterase exprimiert, die von einer wie in Anspruch 1 oder 2 definierten Nucleinsäure oder von dem Vektor nach Anspruch 3 codiert wird.

5. Rekombinanter Organismus, rekombinanter Mikroorganismus oder rekombinante Pflanzenzelle nach Anspruch 4, der/die des Weiteren ein Enzym exprimiert, das unter der EC-Nummer 3.2.1 (Glucosidase) kategorisiert ist.

6. Rekombinanter Organismus, rekombinanter Mikroorganismus oder rekombinante Pflanzenzelle nach Anspruch 5, wobei das Enzym fähig ist, Oleuropein in ein Oleuropein-Aglycon umzusetzen.

7. Rekombinanter Organismus, rekombinanter Mikroorganismus oder rekombinante Pflanzenzelle nach Anspruch 5, wobei das Enzym fähig ist, Ligstrosid in ein Ligstrosid-Aglycon umzusetzen.

8. Rekombinanter Organismus, rekombinanter Mikroorganismus oder rekombinante Pflanzenzelle nach Anspruch 5, wobei das Enzym fähig ist, Oleosid-11-methylester in Elenolsäure umzusetzen.

9. Verwendung eines/einer wie in einem der Ansprüche 4 bis 8 definierten rekombinanten Organismus, rekombinanten Mikroorganismus oder rekombinanten Pflanzenzelle zur Herstellung von Oleacein, Oleocanthal oder Decarboxymethyl-Elenolsäure-Dialdehyd (DEDA).

10. Pflanzenzelle nach einem der Ansprüche 4 bis 9, wobei die Pflanzenzelle zu der Familie *Oleaceae* gehört.

11. Verwendung einer wie in Anspruch 1 definierten Esterase zur Herstellung von Oleacein aus einem Oleuropein-Aglycon.

12. Verwendung einer wie in Anspruch 1 definierten Esterase zur Herstellung von Oleocanthal aus einem Ligstrosid-Aglycon.

13. Verwendung einer wie in Anspruch 1 definierten Esterase zur Herstellung von Decarboxymethyl-Elenolsäure-Dialdehyd (DEDA) aus Elenolsäure.

14. Verfahren zur Herstellung von Oleacein, umfassend die enzymatische Umsetzung eines Oleuropein-Aglycons in Oleacein, wobei das Verfahren die Verwendung einer wie in Anspruch 1 definierten Esterase umfasst.

15. Verfahren zur Herstellung von Oleocanthal, umfassend die enzymatische Umsetzung eines Ligstrosid-Aglycons in Oleocanthal, wobei das Verfahren die Verwendung einer wie in Anspruch 1 definierten Esterase umfasst.

16. Verfahren zur Herstellung von Decarboxymethyl-Elenolsäure-Dialdehyd (DEDA), umfassend die enzymatische Umsetzung von Elenolsäure, wobei das Verfahren die Verwendung einer wie in Anspruch 1 definierten Esterase umfasst.

## Revendications

1. Polypeptide sélectionné dans le groupe constitué par :
(a) un polypeptide comprenant la séquence d'acides aminés de SEQ ID NO : 7 ;
(b) un polypeptide qui est identique à au moins 69 % à la séquence d'acides aminés de SEQ ID NO : 7 et qui est **caractérisé en ce qu'**il a une activité d'estérase pour convertir (i) une oleuropéine aglycone en oléacéine, (ii) un ligstroside aglycone en oléocanthal, ou (iii) l'acide élénolique en dialdéhyde d'acide décarboxyméthyl-élénolique (DEDA);
(c) un polypeptide codé par la molécule d'acide nucléique de SEQ ID NO : 5 ou 6;
(d) un polypeptide codé par une molécule d'acide nucléique qui est identique à au moins 69 % à la séquence d'acide nucléique de SEQ ID NO : 5 ou 6 et où ledit polypeptide est **caractérisé en ce qu'**il a une activité d'estérase pour convertir (i) une oleuropéine aglycone en oléacéine, (ii) un ligstroside aglycone en oléocanthal, ou (iii) l'acide élénolique en dialdéhyde d'acide décarboxyméthyl-élénolique (DEDA); et
(e) un polypeptide codé par la séquence complémentaire d'une molécule d'acide nucléique qui est capable de s'hybrider avec la molécule d'acide nucléique de SEQ ID NO : 5 ou 6, et où ledit polypeptide est **caractérisé en ce qu'**il a une activité d'estérase pour convertir (i) une oleuropéine aglycone en oléacéine, (ii) un ligstroside aglycone en oléocanthal, ou (iii) l'acide élénolique en dialdéhyde d'acide décarboxyméthyl-élénolique (DEDA).

2. Molécule d'acide nucléique exprimant une estérase telle que définie dans la revendication 1.

3. Vecteur comprenant la molécule d'acide nucléique telle que définie dans la revendication 1 ou 2.

4. Organisme, microorganisme ou cellule végétale recombinant, exprimant une estérase codée par un acide nucléique tel que défini dans la revendication 1 ou 2, ou par le vecteur selon la revendication 3.

5. Organisme, microorganisme ou cellule végétale recombinant selon la revendication 4 qui exprime en outre une enzyme qui est catégorisée sous le numéro EC 3.2.1 (glucosidase).

6. Organisme, microorganisme ou cellule végétale recombinant selon la revendication 5, dans lequel l'enzyme est capable de convertir l'oleuropéine en un oleuropéine aglycone.

7. Organisme, microorganisme ou cellule végétale recombinant selon la revendication 5, dans lequel l'enzyme est capable de convertir le ligstroside en un ligstroside aglycone.

8. Organisme, microorganisme ou cellule végétale recombinant selon la revendication 5, dans lequel l'enzyme est capable de convertir l'ester 11-méthylique d'oléoside en acide élénolique.

9. Utilisation d'un organisme, d'un microorganisme ou d'une cellule végétale recombinant tel que défini dans l'une quelconque des revendications 4 à 8, pour la production d'oléacéine, d'oléocanthal, ou de dialdéhyde d'acide décarboxyméthyl-élénolique (DEDA).

10. Cellule végétale selon l'une quelconque des revendications 4 à 9, laquelle cellule végétale appartient à la famille *Oleaceae.*

11. Utilisation d'une estérase telle que définie dans la revendication 1 pour la production d'oléacéine à partir d'un oleuropéine aglycone.

12. Utilisation d'une estérase telle que définie dans la revendication 1 pour la production d'oléocanthal à partir d'un ligstroside aglycone.

13. Utilisation d'une estérase telle que définie dans la revendication 1 pour la production de dialdéhyde d'acide décarboxyméthyl-élénolique (DEDA) à partir d'acide élénolique.

14. Procédé pour la production d'oléacéine comprenant la conversion enzymatique d'un oleuropéine aglycone en oléacéine, lequel procédé comprend l'utilisation d'une estérase telle que définie dans la revendication 1.

15. Procédé pour la production d'oléocanthal comprenant la conversion enzymatique d'un ligstroside aglycone en oléocanthal, lequel procédé comprend l'utilisation d'une estérase telle que définie dans la revendication 1.

16. Procédé pour la production de dialdéhyde d'acide décarboxyméthyl-élénolique (DEDA) comprenant la conversion enzymatique d'acide élénolique, dans lequel ledit procédé comprend l'utilisation d'une estérase telle que définie dans la revendication 1.


### SEQUENCE LISTING

<110> N-Zyme BioTec GmbH Technische Universitaet Darmstadt
<120> An esterase that is capable of converting iridoids and seco-iridoids
<130> Z2232 EP S3
<160> 71
<170> BiSSAP 1.3
<210> 1
   <211> 795
   <212> DNA
   <213> Rauvolfia serpentina
<220>
   <223> coding DNA sequence (CDS) of PNAE
<400> 1
   atgcattctg ctgcaaacgc caagcaacaa aagcattttg ttctggtaca cggcggatgt      60
   ctcggagctt ggatctggta caagctcaag ccgctgctcg agtcagccgg acataaggtc     120
   accgccgttg acctgtcggc cgccggcatc aacccaagaa ggctcgatga gattcacaca     180
   tttcgggact actcggagcc cttgatggaa gtcatggcta gtattcctcc tgatgagaag     240
   gttgttcttc ttggccatag ctttggtggc atgagtttgg gtcttgccat ggaaacctac     300
   ccagagaaga tatcagttgc tgtttttatg tctgcaatga tgcctgatcc taaccactca     360
   ctaacttatc cgtttgagaa gtacaatgag aagtgtccgg cagatatgat gttggactca     420
   cagttttcaa cctacggaaa cccagagaac ccaggaatgt caatgattct tggacctcag     480
   tttatggccc tcaaaatgtt ccagaattgc tcagtcgagg accttgaatt agccaaaatg     540
   ttgactcgac caggttcgtt atttttccaa gatttggcca aggccaaaaa gttctcaacc     600
   gagaggtacg gttcggtgaa gcgagcttat atcttttgca atgaagataa atcatttcca     660
   gttgagtttc agaaatggtt tgttgaaagt gttggagctg ataaagtaaa agaaatcaaa     720
   gaagcagatc atatgggaat gctttcgcag ccaagggaag tttgcaagtg cctgcttgat     780
   atatcagatt cataa                                                      795
<210> 2
   <211> 264
   <212> PRT
   <213> Rauvolfia serpentina
<220>
   <223> Protein sequence of PNAE
<400> 2
   Met His Ser Ala Ala Asn Ala Lys Gln Gln Lys His Phe Val Leu Val
   1               5                   10                  15
   His Gly Gly Cys Leu Gly Ala Trp Ile Trp Tyr Lys Leu Lys Pro Leu
                   20                  25                  30
   Leu Glu Ser Ala Gly His Lys Val Thr Ala Val Asp Leu Ser Ala Ala
           35                  40                  45
   Gly Ile Asn Pro Arg Arg Leu Asp Glu Ile His Thr Phe Arg Asp Tyr
       50                  55                  60
   Ser Glu Pro Leu Met Glu Val Met Ala Ser Ile Pro Pro Asp Glu Lys
   65                  70                  75                  80
   Val Val Leu Leu Gly His Ser Phe Gly Gly Met Ser Leu Gly Leu Ala
                   85                  90                  95
   Met Glu Thr Tyr Pro Glu Lys Ile Ser Val Ala Val Phe Met Ser Ala
               100                 105                 110
   Met Met Pro Asp Pro Asn His Ser Leu Thr Tyr Pro Phe Glu Lys Tyr
           115                 120                 125
   Asn Glu Lys Cys Pro Ala Asp Met Met Leu Asp Ser Gln Phe Ser Thr
       130                 135                 140
   Tyr Gly Asn Pro Glu Asn Pro Gly Met Ser Met Ile Leu Gly Pro Gln
   145                 150                 155                 160
   Phe Met Ala Leu Lys Met Phe Gln Asn Cys Ser Val Glu Asp Leu Glu
                   165                 170                 175
   Leu Ala Lys Met Leu Thr Arg Pro Gly Ser Leu Phe Phe Gln Asp Leu
               180                 185                 190
   Ala Lys Ala Lys Lys Phe Ser Thr Glu Arg Tyr Gly Ser Val Lys Arg
           195                 200                 205
   Ala Tyr Ile Phe Cys Asn Glu Asp Lys Ser Phe Pro Val Glu Phe Gln
       210                 215                 220
   Lys Trp Phe Val Glu Ser Val Gly Ala Asp Lys Val Lys Glu Ile Lys
   225                 230                 235                 240
   Glu Ala Asp His Met Gly Met Leu Ser Gln Pro Arg Glu Val Cys Lys
                   245                 250                 255
   Cys Leu Leu Asp Ile Ser Asp Ser
               260
<210> 3
   <211> 786
   <212> DNA
   <213> Olea europaea
<220>
   <223> coding DNA sequence (CDS) of OE6A038228P1
<400> 3
   atggaagcaa agcaacaaat gcattttgtt ctcgtacatg gtatatgcca tggagcttgg      60
   tgctggtaca agctacaacc gctgctggag gcggcaggtc accgtgtcac agcgtttgat     120
   ctctcagcct ccggcattga cctgaaacac ctagacgagc ttcgcacatt tccggactat     180
   acactgccgt tattgaacgt aatggagtcg attccaccag atgaaaaagt agttctagtt     240
   ggacatagtc ttgctggctt gaacttggct tttgcaatgg agaaataccc tgaaaagatt     300
   tcgatggcaa tttttcttgc ggctatgatg cctgacacta cacacaggcc atcctatgtt     360
   ttggaagagt acttcgcacg gaccccgtcg ggatcttctt cgaacactca aaccgcacca     420
   tttggaagac ccgaagatca actcgtgtca gtactctttg gtccgcagga cttagcatcc     480
   ggattatatc aacgctcccc acctgaggat cttgcattag caaacacttt agtgaggcca     540
   ggctcttttt ttatggaaga tttgtcaaaa aggagcccgt tttccaacga caagtatgga     600
   tccgtaaagc gtgctttcat tatagccggt gcaactgaag ccgaaaatga tttctatcgc     660
   tgggaaattg aaaattttga agtgccaatt gtgaaagaga tggaagcgga aaatactgat     720
   cacatggcaa tgatttcaaa tcccaacact ctttgccaat atatattgga tattgttgga     780
   aaataa                                                                 786
<210> 4
   <211> 786
   <212> PRT
   <213> Olea europaea
<220>
   <223> Protein sequence of OE6A038228P1
<400> 4
   Ala Thr Gly Gly Ala Ala Gly Cys Ala Ala Ala Gly Cys Ala Ala Cys
   1               5                   10                  15
   Ala Ala Ala Thr Gly Cys Ala Thr Thr Thr Thr Gly Thr Thr Cys Thr
               20                  25                  30
   Cys Gly Thr Ala Cys Ala Thr Gly Gly Thr Ala Thr Ala Thr Gly Cys
           35                  40                  45
   Cys Ala Thr Gly Gly Ala Gly Cys Thr Thr Gly Gly Thr Gly Cys Thr
       50                  55                  60
   Gly Gly Thr Ala Cys Ala Ala Gly Cys Thr Ala Cys Ala Ala Cys Cys
   65                  70                  75                  80
   Gly Cys Thr Gly Cys Thr Gly Gly Ala Gly Gly Cys Gly Gly Cys Ala
                   85                  90                  95
   Gly Gly Thr Cys Ala Cys Cys Gly Thr Gly Thr Cys Ala Cys Ala Gly
               100                 105                 110
   Cys Gly Thr Thr Thr Gly Ala Thr Cys Thr Cys Thr Cys Ala Gly Cys
           115                 120                 125
   Cys Thr Cys Cys Gly Gly Cys Ala Thr Thr Gly Ala Cys Cys Thr Gly
       130                 135                 140
   Ala Ala Ala Cys Ala Cys Cys Thr Ala Gly Ala Cys Gly Ala Gly Cys
   145                 150                 155                 160
   Thr Thr Cys Gly Cys Ala Cys Ala Thr Thr Thr Cys Cys Gly Gly Ala
                   165                 170                 175
   Cys Thr Ala Thr Ala Cys Ala Cys Thr Gly Cys Cys Gly Thr Thr Ala
               180                 185                 190
   Thr Thr Gly Ala Ala Cys Gly Thr Ala Ala Thr Gly Gly Ala Gly Thr
           195                 200                 205
   Cys Gly Ala Thr Thr Cys Cys Ala Cys Cys Ala Gly Ala Thr Gly Ala
       210                 215                 220
   Ala Ala Ala Ala Gly Thr Ala Gly Thr Thr Cys Thr Ala Gly Thr Thr
   225                 230                 235                 240
   Gly Gly Ala Cys Ala Thr Ala Gly Thr Cys Thr Thr Gly Cys Thr Gly
                   245                 250                 255
   Gly Cys Thr Thr Gly Ala Ala Cys Thr Thr Gly Gly Cys Thr Thr Thr
               260                 265                 270
   Thr Gly Cys Ala Ala Thr Gly Gly Ala Gly Ala Ala Ala Thr Ala Cys
           275                 280                 285
   Cys Cys Thr Gly Ala Ala Ala Ala Gly Ala Thr Thr Thr Cys Gly Ala
       290                 295                 300
   Thr Gly Gly Cys Ala Ala Thr Thr Thr Thr Thr Cys Thr Thr Gly Cys
   305                 310                 315                 320
   Gly Gly Cys Thr Ala Thr Gly Ala Thr Gly Cys Cys Thr Gly Ala Cys
                   325                 330                 335
   Ala Cys Thr Ala Cys Ala Cys Ala Cys Ala Gly Gly Cys Cys Ala Thr
               340                 345                 350
   Cys Cys Thr Ala Thr Gly Thr Thr Thr Thr Gly Gly Ala Ala Gly Ala
           355                 360                 365
   Gly Thr Ala Cys Thr Thr Cys Gly Cys Ala Cys Gly Gly Ala Cys Cys
       370                 375                 380
   Cys Cys Gly Thr Cys Gly Gly Gly Ala Thr Cys Thr Thr Cys Thr Thr
   385                 390                 395                 400
   Cys Gly Ala Ala Cys Ala Cys Thr Cys Ala Ala Ala Cys Cys Gly Cys
                   405                 410                 415
   Ala Cys Cys Ala Thr Thr Thr Gly Gly Ala Ala Gly Ala Cys Cys Cys
               420                 425                 430
   Gly Ala Ala Gly Ala Thr Cys Ala Ala Cys Thr Cys Gly Thr Gly Thr
           435                 440                 445
   Cys Ala Gly Thr Ala Cys Thr Cys Thr Thr Thr Gly Gly Thr Cys Cys
       450                 455                 460
   Gly Cys Ala Gly Gly Ala Cys Thr Thr Ala Gly Cys Ala Thr Cys Cys
   465                 470                 475                 480
   Gly Gly Ala Thr Thr Ala Thr Ala Thr Cys Ala Ala Cys Gly Cys Thr
                   485                 490                 495
   Cys Cys Cys Cys Ala Cys Cys Thr Gly Ala Gly Gly Ala Thr Cys Thr
               500                 505                 510
   Thr Gly Cys Ala Thr Thr Ala Gly Cys Ala Ala Ala Cys Ala Cys Thr
           515                 520                 525
   Thr Thr Ala Gly Thr Gly Ala Gly Gly Cys Cys Ala Gly Gly Cys Thr
       530                 535                 540
   Cys Thr Thr Thr Thr Thr Thr Thr Ala Thr Gly Gly Ala Ala Gly Ala
   545                 550                 555                 560
   Thr Thr Thr Gly Thr Cys Ala Ala Ala Ala Ala Gly Gly Ala Gly Cys
                   565                 570                 575
   Cys Cys Gly Thr Thr Thr Thr Cys Cys Ala Ala Cys Gly Ala Cys Ala
               580                 585                 590
   Ala Gly Thr Ala Thr Gly Gly Ala Thr Cys Cys Gly Thr Ala Ala Ala
           595                 600                 605
   Gly Cys Gly Thr Gly Cys Thr Thr Thr Cys Ala Thr Thr Ala Thr Ala
       610                 615                 620
   Gly Cys Cys Gly Gly Thr Gly Cys Ala Ala Cys Thr Gly Ala Ala Gly
   625                 630                 635                 640
   Cys Cys Gly Ala Ala Ala Ala Thr Gly Ala Thr Thr Thr Cys Thr Ala
                   645                 650                 655
   Thr Cys Gly Cys Thr Gly Gly Gly Ala Ala Ala Thr Thr Gly Ala Ala
               660                 665                 670
   Ala Ala Thr Thr Thr Thr Gly Ala Ala Gly Thr Gly Cys Cys Ala Ala
           675                 680                 685
   Thr Thr Gly Thr Gly Ala Ala Ala Gly Ala Gly Ala Thr Gly Gly Ala
       690                 695                 700
   Ala Gly Cys Gly Gly Ala Ala Ala Ala Thr Ala Cys Thr Gly Ala Thr
   705                 710                 715                 720
   Cys Ala Cys Ala Thr Gly Gly Cys Ala Ala Thr Gly Ala Thr Thr Thr
                   725                 730                 735
   Cys Ala Ala Ala Thr Cys Cys Cys Ala Ala Cys Ala Cys Thr Cys Thr
               740                 745                 750
   Thr Thr Gly Cys Cys Ala Ala Thr Ala Thr Ala Thr Ala Thr Thr Gly
           755                 760                 765
   Gly Ala Thr Ala Thr Thr Gly Thr Thr Gly Gly Ala Ala Ala Ala Thr
       770                 775                 780
   Ala Ala
   785
<210> 5
   <211> 786
   <212> DNA
   <213> Olea europaea
<220>
   <223> coding DNA sequence (CDS) of OeEst1
<400> 5
   atggaagcaa agcaacaaat gcattttgtt ctcgtacatg gtatatgcca tggagcttgg      60
   tgctggtaca agctacaacc gctgctggag gcggcaggtc accgtgtcac agcgtttgat     120
   ctctcagcct ccggcattga cctgaaacac ctagacgagc ttcgcacatt tccggactat     180
   acactgccgt tatttaacgt aatggagtcg attccaccag atgaaaaagt agttctagtt     240
   ggacatagtc ttgctggctt gaacttggct tttgcaatgg agaaataccc tgaaaagatt     300
   tcgatggcaa tttttcttgc ggctatgatg cctgacacta cacacaggcc atcctatgtt     360
   ttggaagagt acttcgcacg gaccccgtcg ggatcttctt cgaacactca aaccgcacca     420
   tttggaagac ccgaagatca actcgtgtca gtactctttg gtccgcagga cttagcatcc     480
   ggattatatc aacgctcccc acctgaggat cttgcattag caaacacttt agtgaggcca     540
   ggctcttttt ttatggaaga tttgtcaaaa aggagcccgt tttccaacga caagtatgga     600
   tccgtaaagc gtgctttcat tatagccggt gcaactgaag ccgaaaatga tttctatcgc     660
   tgggaaattg aaaattttga agtgccaatt gtgaaagaga tggaagcgga aaatactgat     720
   cacatggcaa tgatttcaaa tcccaacact ctttgccaat atatattgga tattgttgga     780
   aaataa                                                                786
<210> 6
   <211> 786
   <212> DNA
   <213> Olea europaea
<220>
   <223> coding DNA sequence (CDS) of OeEst1 after site-directed mutagenesis
<400> 6
   atggaagcaa agcaacaaat gcattttgtt ctcgtacatg gtatatgcca cggagcttgg      60
   tgctggtaca agctacaacc gctgctggag gcggcaggtc accgtgtcac agcgtttgat     120
   ctctcagcct ccggcattga cctgaaacac ctagacgagc ttcgcacatt tccggactat     180
   acactgccgt tatttaacgt aatggagtcg attccaccag atgaaaaagt agttctagtt     240
   ggacatagtc ttgctggctt gaacttggct tttgcaatgg agaaataccc tgaaaagatt     300
   tcgatggcaa tttttcttgc ggctatgatg cctgacacta cacacaggcc atcctatgtt     360
   ttggaagagt acttcgcacg gaccccgtcg ggatcttctt cgaacactca aaccgcacca     420
   tttggaagac ccgaagatca actcgtgtca gtactctttg gtccgcagga cttagcatcc     480
   ggattatatc aacgctcccc acctgaggat cttgcattag caaacacttt agtgaggcca     540
   ggctcttttt ttatggaaga tttgtcaaaa aggagcccgt tttccaacga caagtatgga     600
   tccgtaaagc gtgctttcat tatagccggt gcaactgaag ccgaaaatga tttctatcgc     660
   tgggaaattg aaaattttga agtgccaatt gtgaaagaga tggaagcgga aaatactgat     720
   cacatggcaa tgatttcaaa tcccaacact ctttgccaat atatattgga tattgttgga     780
   aaataa                                                                786
<210> 7
   <211> 261
   <212> PRT
   <213> Olea europaea
<220>
   <223> Protein sequence of OeEst1
<400> 7
   Met Glu Ala Lys Gln Gln Met His Phe Val Leu Val His Gly Ile Cys
   1               5                   10                  15
   His Gly Ala Trp Cys Trp Tyr Lys Leu Gln Pro Leu Leu Glu Ala Ala
               20                  25                  30
   Gly His Arg Val Thr Ala Phe Asp Leu Ser Ala Ser Gly Ile Asp Leu
           35                  40                  45
   Lys His Leu Asp Glu Leu Arg Thr Phe Pro Asp Tyr Thr Leu Pro Leu
       50                  55                  60
   Phe Asn Val Met Glu Ser Ile Pro Pro Asp Glu Lys Val Val Leu Val
   65                  70                  75                  80
   Gly His Ser Leu Ala Gly Leu Asn Leu Ala Phe Ala Met Glu Lys Tyr
                   85                  90                  95
   Pro Glu Lys Ile Ser Met Ala Ile Phe Leu Ala Ala Met Met Pro Asp
               100                 105                 110
   Thr Thr His Arg Pro Ser Tyr Val Leu Glu Glu Tyr Phe Ala Arg Thr
           115                 120                 125
   Pro Ser Gly Ser Ser Ser Asn Thr Gln Thr Ala Pro Phe Gly Arg Pro
       130                 135                 140
   Glu Asp Gln Leu Val Ser Val Leu Phe Gly Pro Gln Asp Leu Ala Ser
   145                 150                 155                 160
   Gly Leu Tyr Gln Arg Ser Pro Pro Glu Asp Leu Ala Leu Ala Asn Thr
                   165                 170                 175
   Leu Val Arg Pro Gly Ser Phe Phe Met Glu Asp Leu Ser Lys Arg Ser
               180                 185                 190
   Pro Phe Ser Asn Asp Lys Tyr Gly Ser Val Lys Arg Ala Phe Ile Ile
           195                 200                 205
   Ala Gly Ala Thr Glu Ala Glu Asn Asp Phe Tyr Arg Trp Glu Ile Glu
       210                 215                 220
   Asn Phe Glu Val Pro Ile Val Lys Glu Met Glu Ala Glu Asn Thr Asp
   225                 230                 235                 240
   His Met Ala Met Ile Ser Asn Pro Asn Thr Leu Cys Gln Tyr Ile Leu
                   245                 250                 255
   Asp Ile Val Gly Lys
               260
<210> 8
   <211> 570
   <212> DNA
   <213> Artificial Sequence
<220>
   <223> coding DNA sequence (CDS) of the pET-32a(+) thioredoxin fusion protein
<400> 8
   atgagcgata aaattattca cctgactgac gacagttttg acacggatgt actcaaagcg      60
   gacggggcga tcctcgtcga tttctgggca gagtggtgcg gtccgtgcaa aatgatcgcc     120
   ccgattctgg atgaaatcgc tgacgaatat cagggcaaac tgaccgttgc aaaactgaac     180
   atcgatcaaa accctggcac tgcgccgaaa tatggcatcc gtggtatccc gactctgctg     240
   ctgttcaaaa acggtgaagt ggcggcaacc aaagtgggtg cactgtctaa aggtcagttg     300
   aaagagttcc tcgacgctaa cctggccggt tctggttctg gccatatgca ccatcatcat     360
   catcattctt ctggtctggt gccacgcggt tctggtatga aagaaaccgc tgctgctaaa     420
   ttcgaacgcc agcacatgga cagcccagat ctgggtaccg acgacgacga caaggccatg     480
   gctgatatcg gatccgaatt cgagctccgt cgacaagctt gcggccgcac tcgagcacca     540
   ccaccaccac cactgagatc cggctgctaa                                      570
<210> 9
   <211> 189
   <212> PRT
   <213> Artificial Sequence
<220>
   <223> pET-32a(+) thioredoxin fusion protein
<400> 9
   Met Ser Asp Lys Ile Ile His Leu Thr Asp Asp Ser Phe Asp Thr Asp
   1               5                   10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly His Met His His His His His His Ser Ser Gly Leu Val Pro
           115                 120                 125
   Arg Gly Ser Gly Met Lys Glu Thr Ala Ala Ala Lys Phe Glu Arg Gln
       130                 135                 140
   His Met Asp Ser Pro Asp Leu Gly Thr Asp Asp Asp Asp Lys Ala Met
   145                 150                 155                 160
   Ala Asp Ile Gly Ser Glu Phe Glu Leu Arg Arg Gln Ala Cys Gly Arg
                   165                 170                 175
   Thr Arg Ala Pro Pro Pro Pro Pro Leu Arg Ser Gly Cys
               180                 185
<210> 10
   <211> 1263
   <212> DNA
   <213> Artificial Sequence
<220>
   <223> coding DNA sequence (CDS) encoding the fusion protein consisting of OeEst1 and the thioredoxin tag
<400> 10
   atgagcgata aaattattca cctgactgac gacagttttg acacggatgt actcaaagcg      60
   gacggggcga tcctcgtcga tttctgggca gagtggtgcg gtccgtgcaa aatgatcgcc     120
   ccgattctgg atgaaatcgc tgacgaatat cagggcaaac tgaccgttgc aaaactgaac     180
   atcgatcaaa accctggcac tgcgccgaaa tatggcatcc gtggtatccc gactctgctg     240
   ctgttcaaaa acggtgaagt ggcggcaacc aaagtgggtg cactgtctaa aggtcagttg     300
   aaagagttcc tcgacgctaa cctggccggt tctggttctg gccatatgca ccatcatcat     360
   catcattctt ctggtctggt gccacgcggt tctggtatga aagaaaccgc tgctgctaaa     420
   ttcgaacgcc agcacatgga cagcccagat ctgggtaccg acgacgacga caaggccatg     480
   gaagcaaagc aacaaatgca ttttgttctc gtacatggta tatgccacgg agcttggtgc     540
   tggtacaagc tacaaccgct gctggaggcg gcaggtcacc gtgtcacagc gtttgatctc     600
   tcagcctccg gcattgacct gaaacaccta gacgagcttc gcacatttcc ggactataca     660
   ctgccgttat ttaacgtaat ggagtcgatt ccaccagatg aaaaagtagt tctagttgga     720
   catagtcttg ctggcttgaa cttggctttt gcaatggaga aataccctga aaagatttcg     780
   atggcaattt ttcttgcggc tatgatgcct gacactacac acaggccatc ctatgttttg     840
   gaagagtact tcgcacggac cccgtcggga tcttcttcga acactcaaac cgcaccattt     900
   ggaagacccg aagatcaact cgtgtcagta ctctttggtc cgcaggactt agcatccgga     960
   ttatatcaac gctccccacc tgaggatctt gcattagcaa acactttagt gaggccaggc    1020
   tcttttttta tggaagattt gtcaaaaagg agcccgtttt ccaacgacaa gtatggatcc    1080
   gtaaagcgtg ctttcattat agccggtgca actgaagccg aaaatgattt ctatcgctgg    1140
   gaaattgaaa attttgaagt gccaattgtg aaagagatgg aagcggaaaa tactgatcac    1200
   atggcaatga tttcaaatcc caacactctt tgccaatata tattggatat tgttggaaaa    1260
   taa                                                                  1263
<210> 11
   <211> 420
   <212> PRT
   <213> Artificial Sequence
<220>
   <223> Fusion protein consisting of OeEst1 and the thioredoxin tag
<400> 11
   Met Ser Asp Lys Ile Ile His Leu Thr Asp Asp Ser Phe Asp Thr Asp
   1               5                   10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly His Met His His His His His His Ser Ser Gly Leu Val Pro
           115                 120                 125
   Arg Gly Ser Gly Met Lys Glu Thr Ala Ala Ala Lys Phe Glu Arg Gln
       130                 135                 140
   His Met Asp Ser Pro Asp Leu Gly Thr Asp Asp Asp Asp Lys Ala Met
   145                 150                 155                 160
   Glu Ala Lys Gln Gln Met His Phe Val Leu Val His Gly Ile Cys His
                   165                 170                 175
   Gly Ala Trp Cys Trp Tyr Lys Leu Gln Pro Leu Leu Glu Ala Ala Gly
               180                 185                 190
   His Arg Val Thr Ala Phe Asp Leu Ser Ala Ser Gly Ile Asp Leu Lys
           195                 200                 205
   His Leu Asp Glu Leu Arg Thr Phe Pro Asp Tyr Thr Leu Pro Leu Phe
       210                 215                 220
   Asn Val Met Glu Ser Ile Pro Pro Asp Glu Lys Val Val Leu Val Gly
   225                 230                 235                 240
   His Ser Leu Ala Gly Leu Asn Leu Ala Phe Ala Met Glu Lys Tyr Pro
                   245                 250                 255
   Glu Lys Ile Ser Met Ala Ile Phe Leu Ala Ala Met Met Pro Asp Thr
               260                 265                 270
   Thr His Arg Pro Ser Tyr Val Leu Glu Glu Tyr Phe Ala Arg Thr Pro
           275                 280                 285
   Ser Gly Ser Ser Ser Asn Thr Gln Thr Ala Pro Phe Gly Arg Pro Glu
       290                 295                 300
   Asp Gln Leu Val Ser Val Leu Phe Gly Pro Gln Asp Leu Ala Ser Gly
   305                 310                 315                 320
   Leu Tyr Gln Arg Ser Pro Pro Glu Asp Leu Ala Leu Ala Asn Thr Leu
                   325                 330                 335
   Val Arg Pro Gly Ser Phe Phe Met Glu Asp Leu Ser Lys Arg Ser Pro
               340                 345                 350
   Phe Ser Asn Asp Lys Tyr Gly Ser Val Lys Arg Ala Phe Ile Ile Ala
           355                 360                 365
   Gly Ala Thr Glu Ala Glu Asn Asp Phe Tyr Arg Trp Glu Ile Glu Asn
       370                 375                 380
   Phe Glu Val Pro Ile Val Lys Glu Met Glu Ala Glu Asn Thr Asp His
   385                 390                 395                 400
   Met Ala Met Ile Ser Asn Pro Asn Thr Leu Cys Gln Tyr Ile Leu Asp
                   405                 410                 415
   Ile Val Gly Lys
               420
<210> 12
   <211> 33
   <212> DNA
   <213> Artificial Sequence
<220>
   <223> OeEst228plain_fwd
<400> 12
   atggaagcaa agcaacaaat gcattttgtt ctc 33
<210> 13
   <211> 45
   <212> DNA
   <213> Artificial Sequence
<220>
   <223> OeEst228plain_rev
<400> 13
   ttattttcca acaatatcca atatatattg gcaaagagtg ttggt 45
<210> 14
   <211> 18
   <212> DNA
   <213> Artificial Sequence
<220>
   <223> primers M13 uni (-21)
<400> 14
   tgtaaaacga cggccagt 18
<210> 15
   <211> 18
   <212> DNA
   <213> Artificial Sequence
<220>
   <223> M13 rev (-29)
<400> 15
   caggaaacag ctatgacc 18
<210> 16
   <211> 33
   <212> DNA
   <213> Artificial Sequence
<220>
   <223> OeEst228NcoI_fwd
<400> 16
   tcaccatgga agcaaagcaa caaatgcatt ttg 33
<210> 17
   <211> 57
   <212> DNA
   <213> Artificial Sequence
<220>
   <223> OeEst228XhoI_rev
<400> 17
   atacctcgag ttattttcca acaatatcca atatatattg gcaaagagtg ttgggat 57
<210> 18
   <211> 26
   <212> DNA
   <213> Artificial Sequence
<220>
   <223> OeEst_sdm1_fwd
<400> 18
   atggtatatg ccacggagct tggtgc 26
<210> 19
   <211> 25
   <212> DNA
   <213> Artificial Sequence
<220>
   <223> OeEst_sdm1_rev
<400> 19
   gtacgagaac aaagtgcctt tgttg 25
<210> 20
   <211> 783
   <212> DNA
   <213> Olea europaea
<220>
   <223> coding DNA sequence (CDS) of OE6A059030P1
<400> 20
   atgggagata accaaaaaca gcatcttgtt ctcatacatg gtatatgcca tggagcttgg      60
   tgctggtaca agctacagcc actgctggag gacgccggtt accgtgtctc aaatcttgat     120
   atgtcagcct ccggcattga cccgaaaatc ctagtcaacc ttagcacatt tcctgaatat     180
   acagatccgt tacttaactt tctggagtcg cttccatcag gtgaaacggt agttctaatc     240
   ggacatagtc ttggtggcat gaacatggct tttgcaatgg agaaataccc taaaaaggtt     300
   tctctggcaa tttttcttgc ggctataatg cctgacacta caaatccgcc atcctatgtt     360
   ttggatcggt acttatcagg ggccacgccg gagtcttgga tggacactca attcgtatca     420
   tttggccccg acgatcaact ggtgtcatta ttctttggtc cgaaatactt gccggccaca     480
   atatattcac actccccacc tgaggatgtt gcattagcaa agactttagt gaggccaggc     540
   agtttttttt tggctgattt gtcaaaaagg agcccgtttt ccaacgacaa gtatggatcc     600
   gtaaagcgtg ctttcattat agctggtgca tctgaagccg aaaatgattt ctatcgttgg     660
   gaaattgaaa attttaacgt gtcaatagtg aaagagatgg acaccaaaaa aactgatcac     720
   atggcaatga tttcaaatcc caaagagctt tacaaatata tattggagat tgttggaaaa     780
   taa                                                                   783
<210> 21
   <211> 260
   <212> PRT
   <213> Olea europaea
<220>
   <223> Protein sequence of OE6A059030P1
<400> 21
   Met Gly Asp Asn Gln Lys Gln His Leu Val Leu Ile His Gly Ile Cys
   1               5                   10                  15
   His Gly Ala Trp Cys Trp Tyr Lys Leu Gln Pro Leu Leu Glu Asp Ala
               20                  25                  30
   Gly Tyr Arg Val Ser Asn Leu Asp Met Ser Ala Ser Gly Ile Asp Pro
           35                  40                  45
   Lys Ile Leu Val Asn Leu Ser Thr Phe Pro Glu Tyr Thr Asp Pro Leu
       50                  55                  60
   Leu Asn Phe Leu Glu Ser Leu Pro Ser Gly Glu Thr Val Val Leu Ile
   65                  70                  75                  80
   Gly His Ser Leu Gly Gly Met Asn Met Ala Phe Ala Met Glu Lys Tyr
                   85                  90                  95
   Pro Lys Lys Val Ser Leu Ala Ile Phe Leu Ala Ala Ile Met Pro Asp
               100                 105                 110
   Thr Thr Asn Pro Pro Ser Tyr Val Leu Asp Arg Tyr Leu Ser Gly Ala
           115                 120                 125
   Thr Pro Glu Ser Trp Met Asp Thr Gln Phe Val Ser Phe Gly Pro Asp
       130                 135                 140
   Asp Gln Leu Val Ser Leu Phe Phe Gly Pro Lys Tyr Leu Pro Ala Thr
   145                 150                 155                 160
   Ile Tyr Ser His Ser Pro Pro Glu Asp Val Ala Leu Ala Lys Thr Leu
                   165                 170                 175
   Val Arg Pro Gly Ser Phe Phe Leu Ala Asp Leu Ser Lys Arg Ser Pro
               180                 185                 190
   Phe Ser Asn Asp Lys Tyr Gly Ser Val Lys Arg Ala Phe Ile Ile Ala
           195                 200                 205
   Gly Ala Ser Glu Ala Glu Asn Asp Phe Tyr Arg Trp Glu Ile Glu Asn
       210                 215                 220
   Phe Asn Val Ser Ile Val Lys Glu Met Asp Thr Lys Lys Thr Asp His
   225                 230                 235                 240
   Met Ala Met Ile Ser Asn Pro Lys Glu Leu Tyr Lys Tyr Ile Leu Glu
                   245                 250                 255
   Ile Val Gly Lys
               260
<210> 22
   <211> 783
   <212> DNA
   <213> Olea europaea
<220>
   <223> coding DNA sequence (CDS) of OeEst030
<400> 22
   atgggagata accaaaaaca gcatcttgtt ctcatacatg gtatatgcca tggagcttgg      60
   tgctggtaca agctacagcc actgctggag gacgccggtt accgtgtctc aaatcttgat     120
   atgtcagcct ccggcattga cccgaaaatc ctagtcaacc ttagcacatt tcctgaatat     180
   acagatccgt tacttaactt tctggagtcg cttccatcag gtgaaacggt agttctaatc     240
   ggacatagtc ttggtggcat gaacatggct tttgcaatgg agaaataccc taaaaaggtt     300
   tctctggcaa tttttcttgc ggctataatg cctgacacta caaatccgcc atcctatgtt     360
   ttggatcggt acttatcagg ggccacgccg gagtcttgga tggacactca attcgtatca     420
   tttggccccg acgatcaact ggtgtcatta ttctttggtc cgaaatactt gccggccaca     480
   atatattcac actccccacc tgaggatgtt gcattagcaa agactttagt gaggccaggc     540
   agtttttttt tggctgattt gtcaaaaagg agcccgtttt ccaacgacaa gtatggatcc      600
   gtaaagcgtg ctttcattat agctggtgca tctgaagccg aaaatgattt ctatcgttgg     660
   gaaattgaaa attttaacgt gtcaatagtg aaagagatgg acaccaaaaa aactgatcac     720
   atggcaatga tttcaaatcc caaagagctt tacaaatata tattggatat tgttggaaaa     780
   taa                                                                   783
<210> 23
   <211> 783
   <212> DNA
   <213> Olea europaea
<220>
   <223> coding DNA sequence (CDS) of OeEst030 after site-directed mutagenesis
<400> 23
   atgggagata accaaaaaca gcatcttgtt ctcatacatg gtatatgcca cggagcttgg      60
   tgctggtaca agctacagcc actgctggag gacgccggtt accgtgtctc aaatcttgat     120
   atgtcagcct ccggcattga cccgaaaatc ctagtcaacc ttagcacatt tcctgaatat     180
   acagatccgt tacttaactt tctggagtcg cttccatcag gtgaaacggt agttctaatc     240
   ggacatagtc ttggtggcat gaacatggct tttgcaatgg agaaataccc taaaaaggtt     300
   tctctggcaa tttttcttgc ggctataatg cctgacacta caaatccgcc atcctatgtt     360
   ttggatcggt acttatcagg ggccacgccg gagtcttgga tggacactca attcgtatca     420
   tttggccccg acgatcaact ggtgtcatta ttctttggtc cgaaatactt gccggccaca     480
   atatattcac actccccacc tgaggatgtt gcattagcaa agactttagt gaggccaggc     540
   agtttttttt tggctgattt gtcaaaaagg agcccgtttt ccaacgacaa gtatggatcc     600
   gtaaagcgtg ctttcattat agctggtgca tctgaagccg aaaatgattt ctatcgttgg     660
   gaaattgaaa attttaacgt gtcaatagtg aaagagatgg acaccaaaaa aactgatcac     720
   atggcaatga tttcaaatcc caaagagctt tacaaatata tattggagat tgttggaaaa     780
   taa                                                                   783
<210> 24
   <211> 260
   <212> PRT
   <213> Olea europaea
<220>
   <223> Protein sequence of OeEst030
<400> 24
   Met Gly Asp Asn Gln Lys Gln His Leu Val Leu Ile His Gly Ile Cys
   1               5                   10                  15
   His Gly Ala Trp Cys Trp Tyr Lys Leu Gln Pro Leu Leu Glu Asp Ala
               20                  25                  30
   Gly Tyr Arg Val Ser Asn Leu Asp Met Ser Ala Ser Gly Ile Asp Pro
           35                  40                  45
   Lys Ile Leu Val Asn Leu Ser Thr Phe Pro Glu Tyr Thr Asp Pro Leu
       50                  55                  60
   Leu Asn Phe Leu Glu Ser Leu Pro Ser Gly Glu Thr Val Val Leu Ile
   65                  70                  75                  80
   Gly His Ser Leu Gly Gly Met Asn Met Ala Phe Ala Met Glu Lys Tyr
                   85                  90                  95
   Pro Lys Lys Val Ser Leu Ala Ile Phe Leu Ala Ala Ile Met Pro Asp
               100                 105                 110
   Thr Thr Asn Pro Pro Ser Tyr Val Leu Asp Arg Tyr Leu Ser Gly Ala
           115                 120                 125
   Thr Pro Glu Ser Trp Met Asp Thr Gln Phe Val Ser Phe Gly Pro Asp
       130                 135                 140
   Asp Gln Leu Val Ser Leu Phe Phe Gly Pro Lys Tyr Leu Pro Ala Thr
   145                 150                 155                 160
   Ile Tyr Ser His Ser Pro Pro Glu Asp Val Ala Leu Ala Lys Thr Leu
                   165                 170                 175
   Val Arg Pro Gly Ser Phe Phe Leu Ala Asp Leu Ser Lys Arg Ser Pro
               180                 185                 190
   Phe Ser Asn Asp Lys Tyr Gly Ser Val Lys Arg Ala Phe Ile Ile Ala
           195                 200                 205
   Gly Ala Ser Glu Ala Glu Asn Asp Phe Tyr Arg Trp Glu Ile Glu Asn
       210                 215                 220
   Phe Asn Val Ser Ile Val Lys Glu Met Asp Thr Lys Lys Thr Asp His
   225                 230                 235                 240
   Met Ala Met Ile Ser Asn Pro Lys Glu Leu Tyr Lys Tyr Ile Leu Glu
                   245                 250                 255
   Ile Val Gly Lys
               260
<210> 25
   <211> 1260
   <212> DNA
   <213> Artificial Sequence
<220>
   <223> coding DNA sequence (CDS) encoding the fusion protein consisting of OeEst030 and the thioredoxin tag
<400> 25
   atgagcgata aaattattca cctgactgac gacagttttg acacggatgt actcaaagcg      60
   gacggggcga tcctcgtcga tttctgggca gagtggtgcg gtccgtgcaa aatgatcgcc     120
   ccgattctgg atgaaatcgc tgacgaatat cagggcaaac tgaccgttgc aaaactgaac     180
   atcgatcaaa accctggcac tgcgccgaaa tatggcatcc gtggtatccc gactctgctg     240
   ctgttcaaaa acggtgaagt ggcggcaacc aaagtgggtg cactgtctaa aggtcagttg     300
   aaagagttcc tcgacgctaa cctggccggt tctggttctg gccatatgca ccatcatcat     360
   catcattctt ctggtctggt gccacgcggt tctggtatga aagaaaccgc tgctgctaaa     420
   ttcgaacgcc agcacatgga cagcccagat ctgggtaccg acgacgacga caaggccatg     480
   ggagataacc aaaaacagca tcttgttctc atacatggta tatgccacgg agcttggtgc     540
   tggtacaagc tacagccact gctggaggac gccggttacc gtgtctcaaa tcttgatatg     600
   tcagcctccg gcattgaccc gaaaatccta gtcaacctta gcacatttcc tgaatataca     660
   gatccgttac ttaactttct ggagtcgctt ccatcaggtg aaacggtagt tctaatcgga     720
   catagtcttg gtggcatgaa catggctttt gcaatggaga aataccctaa aaaggtttct     780
   ctggcaattt ttcttgcggc tataatgcct gacactacaa atccgccatc ctatgttttg     840
   gatcggtact tatcaggggc cacgccggag tcttggatgg acactcaatt cgtatcattt     900
   ggccccgacg atcaactggt gtcattattc tttggtccga aatacttgcc ggccacaata     960
   tattcacact ccccacctga ggatgttgca ttagcaaaga ctttagtgag gccaggcagt    1020
   tttttttggg ctgatttgtc aaaaaggagc ccgttttcca acgacaagta tggatccgta    1080
   aagcgtgctt tcattatagc tggtgcatct gaagccgaaa atgatttcta tcgttgggaa    1140
   attgaaaatt ttaacgtgtc aatagtgaaa gagatggaca ccaaaaaaac tgatcacatg    1200
   gcaatgattt caaatcccaa agagctttac aaatatatat tggagattgt tggaaaataa    1260
<210> 26
   <211> 419
   <212> PRT
   <213> Artificial Sequence
<220>
   <223> Fusion protein consisting of OeEst030 and the thioredoxin tag
<400> 26
   Met Ser Asp Lys Ile Ile His Leu Thr Asp Asp Ser Phe Asp Thr Asp
   1               5                   10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly His Met His His His His His His Ser Ser Gly Leu Val Pro
           115                 120                 125
   Arg Gly Ser Gly Met Lys Glu Thr Ala Ala Ala Lys Phe Glu Arg Gln
       130                 135                 140
   His Met Asp Ser Pro Asp Leu Gly Thr Asp Asp Asp Asp Lys Ala Met
   145                 150                 155                 160
   Gly Asp Asn Gln Lys Gln His Leu Val Leu Ile His Gly Ile Cys His
                   165                 170                 175
   Gly Ala Trp Cys Trp Tyr Lys Leu Gln Pro Leu Leu Glu Asp Ala Gly
               180                 185                 190
   Tyr Arg Val Ser Asn Leu Asp Met Ser Ala Ser Gly Ile Asp Pro Lys
           195                 200                 205
   Ile Leu Val Asn Leu Ser Thr Phe Pro Glu Tyr Thr Asp Pro Leu Leu
       210                 215                 220
   Asn Phe Leu Glu Ser Leu Pro Ser Gly Glu Thr Val Val Leu Ile Gly
   225                 230                 235                 240
   His Ser Leu Gly Gly Met Asn Met Ala Phe Ala Met Glu Lys Tyr Pro
                   245                 250                 255
   Lys Lys Val Ser Leu Ala Ile Phe Leu Ala Ala Ile Met Pro Asp Thr
               260                 265                 270
   Thr Asn Pro Pro Ser Tyr Val Leu Asp Arg Tyr Leu Ser Gly Ala Thr
           275                 280                 285
   Pro Glu Ser Trp Met Asp Thr Gln Phe Val Ser Phe Gly Pro Asp Asp
       290                 295                 300
   Gln Leu Val Ser Leu Phe Phe Gly Pro Lys Tyr Leu Pro Ala Thr Ile
   305                 310                 315                 320
   Tyr Ser His Ser Pro Pro Glu Asp Val Ala Leu Ala Lys Thr Leu Val
                   325                 330                 335
   Arg Pro Gly Ser Phe Phe Leu Ala Asp Leu Ser Lys Arg Ser Pro Phe
               340                 345                 350
   Ser Asn Asp Lys Tyr Gly Ser Val Lys Arg Ala Phe Ile Ile Ala Gly
           355                 360                 365
   Ala Ser Glu Ala Glu Asn Asp Phe Tyr Arg Trp Glu Ile Glu Asn Phe
       370                 375                 380
   Asn Val Ser Ile Val Lys Glu Met Asp Thr Lys Lys Thr Asp His Met
   385                 390                 395                 400
   Ala Met Ile Ser Asn Pro Lys Glu Leu Tyr Lys Tyr Ile Leu Glu Ile
                   405                 410                 415
   Val Gly Lys
<210> 27
   <211> 31
   <212> DNA
   <213> Artificial Sequence
<220>
   <223> OeEst030plain_fwd
<400> 27
   atgggagata accaaaaaca gcatcttgtt c 31
<210> 28
   <211> 40
   <212> DNA
   <213> Artificial Sequence
<220>
   <223> OeEst030plain_rv
<400> 28
   ttattttcca acaatctcca atatatattt gtaaagctct 40
<210> 29
   <211> 37
   <212> DNA
   <213> Artificial Sequence
<220>
   <223> OeEst030NcoI_fwd
<400> 29
   cgagccatgg gagataacca aaaacagcat cttgttc 37
<210> 30
   <211> 50
   <212> DNA
   <213> Artificial Sequence
<220>
   <223> OeEst030XhoI_rev
<400> 30
   atacctcgag ttattttcca acaatctcca atatatattt gtaaagctct 50
<210> 31
   <211> 22
   <212> DNA
   <213> Artificial Sequence
<220>
   <223> OeEst030sdm_fwd
<400> 31
   gtatatgcca cggagcttgg tg 22
<210> 32
   <211> 22
   <212> DNA
   <213> Artificial Sequence
<220>
   <223> OeEst030sdm_rev
<400> 32
   catgtatgag aacaagatgc tg 22
<210> 33
   <211> 795
   <212> DNA
   <213> Olea europaea
<220>
   <223> coding DNA sequence (CDS) of OE6A068679P2
<400> 33
   atgggagata accaaaaaca gcatcttgtt ctcgtacatg gtatatgcca tggagcttgg      60
   tgctggtaca agctacagac actgctggag gacgccggtt accgtgtctc aaatcttgat     120
   atgtcagcct ctggcattga cccgaaaagc ctagctgacc ttagcacatt tcctgaatat     180
   acagacccgt tacttaactt tatggagtcg attccatcag ttgaaactgt agttctaatc     240
   ggacatagtc ttgctggcat gaacttggct tttgcaatgg agaaataccc taaaaaggtt     300
   tctttggcaa tttttattgc ggctataatg cctgacgcta taaataagcc atcctttgtt     360
   ttggaagagt acttctcacg gatcccgccg ggagctttta tggactctca tttcccacca     420
   tttggccgcc ccgaaaataa gctgatggca ataaactttg gtccgcaata catggcagcc     480
   acaatatatt cacgctcccc acctgaggat cgtactttag caaataattt agtgaggcca     540
   ggctgtttct ttatggaaga tttgtcaaaa aggaagccat tttccaacaa caagtatgga     600
   tccgtaaagc gtgcttttat tatagctggt gcaactgatg ccgaacacaa attctatcgc     660
   tgggaaattg aaaattttga agtgccaata gtgaaagaga tggacaagga aactactgat     720
   cacatggcaa tgatttcaaa tcctaaaact ctttgccgat atatattgga tattgtggaa     780
   aataaaccaa attaa                                                      795
<210> 34
   <211> 264
   <212> PRT
   <213> Olea europaea
<220>
   <223> Protein sequence of OE6A068679P2
<400> 34
   Met Gly Asp Asn Gln Lys Gln His Leu Val Leu Val His Gly Ile Cys
   1               5                   10                  15
   His Gly Ala Trp Cys Trp Tyr Lys Leu Gln Thr Leu Leu Glu Asp Ala
               20                  25                  30
   Gly Tyr Arg Val Ser Asn Leu Asp Met Ser Ala Ser Gly Ile Asp Pro
           35                  40                  45
   Lys Ser Leu Ala Asp Leu Ser Thr Phe Pro Glu Tyr Thr Asp Pro Leu
       50                  55                  60
   Leu Asn Phe Met Glu Ser Ile Pro Ser Val Glu Thr Val Val Leu Ile
   65                  70                  75                  80
   Gly His Ser Leu Ala Gly Met Asn Leu Ala Phe Ala Met Glu Lys Tyr
                   85                  90                  95
   Pro Lys Lys Val Ser Leu Ala Ile Phe Ile Ala Ala Ile Met Pro Asp
               100                 105                 110
   Ala Ile Asn Lys Pro Ser Phe Val Leu Glu Glu Tyr Phe Ser Arg Ile
           115                 120                 125
   Pro Pro Gly Ala Phe Met Asp Ser His Phe Pro Pro Phe Gly Arg Pro
       130                 135                 140
   Glu Asn Lys Leu Met Ala Ile Asn Phe Gly Pro Gln Tyr Met Ala Ala
   145                 150                 155                 160
   Thr Ile Tyr Ser Arg Ser Pro Pro Glu Asp Arg Thr Leu Ala Asn Asn
                   165                 170                 175
   Leu Val Arg Pro Gly Cys Phe Phe Met Glu Asp Leu Ser Lys Arg Lys
               180                 185                 190
   Pro Phe Ser Asn Asn Lys Tyr Gly Ser Val Lys Arg Ala Phe Ile Ile
           195                 200                 205
   Ala Gly Ala Thr Asp Ala Glu His Lys Phe Tyr Arg Trp Glu Ile Glu
       210                 215                 220
   Asn Phe Glu Val Pro Ile Val Lys Glu Met Asp Lys Glu Thr Thr Asp
   225                 230                 235                 240
   His Met Ala Met Ile Ser Asn Pro Lys Thr Leu Cys Arg Tyr Ile Leu
                   245                 250                 255
   Asp Ile Val Glu Asn Lys Pro Asn
               260
<210> 35
   <211> 795
   <212> DNA
   <213> Olea europaea
<220>
   <223> coding DNA sequence (CDS) of OeEst679
<400> 35
   atgggagata accaaaaaca gcatcttgtt ctcgtacatg gtatatgcca tggagcttgg      60
   tgctggtaca agctacagac actgctggag gacgccggtt accgtgtctc aaatcttgat     120
   atgtcagcct ctggcattga cccgaaaagc ctagctgacc ttagcacatt tcctgaatat     180
   acagacccgt tacttaactt tatggagtcg attccatcag ttgaaactgt agttctaatc     240
   ggacatagtc ttgctggcat gaacttggct tttgcaatgg agaaataccc taaaaaggtt      300
   tctttggcaa tttttattgc ggctataatg cctgacgcta taaataagcc atcctttgtt      360
   ttggaagagt acttctcacg gatcccgccg ggagctttta tggactctca tttcccacca      420
   tttggccgcc ccgaaaataa gctgatggca ataaactttg gtccgcaata catggaagcc      480
   acaatatatt cacgctcccc acctgaggat cgtactttag caaataattt agtgaggcca      540
   ggctgtttct ttatggaaga tttgtcaaaa aggaagccgt tttccaacga caagtatgga      600
   tccgtaaagc gtgcttttat tatagctggt gcaactgatg ccgaacacaa attctatcgc      660
   tgggaaattg aaaattttga agtgccaata gtgaaagaga tggacaagga aactactgat      720
   cacatggcaa tgatttcaaa tcctaaaact ctttgccgat atatattgga tattgtggaa      780
   aataaaccaa attaa                                                       795
<210> 36
   <211> 795
   <212> DNA
   <213> Olea europaea
<220>
   <223> coding DNA sequence (CDS) of OeEst679 after site-directed mutagenesis
<400> 36
   atgggagata accaaaaaca gcatcttgtt ctcgtacatg gtatatgcca cggagcttgg       60
   tgctggtaca agctacagac actgctggag gacgccggtt accgtgtctc aaatcttgat      120
   atgtcagcct ctggcattga cccgaaaagc ctagctgacc ttagcacatt tcctgaatat      180
   acagacccgt tacttaactt tatggagtcg attccatcag ttgaaactgt agttctaatc      240
   ggacatagtc ttgctggcat gaacttggct tttgcaatgg agaaataccc taaaaaggtt      300
   tctttggcaa tttttattgc ggctataatg cctgacgcta taaataagcc atcctttgtt      360
   ttggaagagt acttctcacg gatcccgccg ggagctttta tggactctca tttcccacca      420
   tttggccgcc ccgaaaataa gctgatggca ataaactttg gtccgcaata catggaagcc      480
   acaatatatt cacgctcccc acctgaggat cgtactttag caaataattt agtgaggcca      540
   ggctgtttct ttatggaaga tttgtcaaaa aggaagccgt tttccaacga caagtatgga      600
   tccgtaaagc gtgcttttat tatagctggt gcaactgatg ccgaacacaa attctatcgc      660
   tgggaaattg aaaattttga agtgccaata gtgaaagaga tggacaagga aactactgat      720
   cacatggcaa tgatttcaaa tcctaaaact ctttgccgat atatattgga tattgtggaa      780
   aataaaccaa attaa                                                       795
<210> 37
   <211> 264
   <212> PRT
   <213> Olea europaea
<220>
   <223> Protein sequence of OeEst679
<400> 37
   Met Gly Asp Asn Gln Lys Gln His Leu Val Leu Val His Gly Ile Cys
   1               5                   10                  15
   His Gly Ala Trp Cys Trp Tyr Lys Leu Gln Thr Leu Leu Glu Asp Ala
               20                  25                  30
   Gly Tyr Arg Val Ser Asn Leu Asp Met Ser Ala Ser Gly Ile Asp Pro
           35                  40                  45
   Lys Ser Leu Ala Asp Leu Ser Thr Phe Pro Glu Tyr Thr Asp Pro Leu
       50                  55                  60
   Leu Asn Phe Met Glu Ser Ile Pro Ser Val Glu Thr Val Val Leu Ile
   65                  70                  75                  80
   Gly His Ser Leu Ala Gly Met Asn Leu Ala Phe Ala Met Glu Lys Tyr
                   85                  90                  95
   Pro Lys Lys Val Ser Leu Ala Ile Phe Ile Ala Ala Ile Met Pro Asp
               100                 105                 110
   Ala Ile Asn Lys Pro Ser Phe Val Leu Glu Glu Tyr Phe Ser Arg Ile
           115                 120                 125
   Pro Pro Gly Ala Phe Met Asp Ser His Phe Pro Pro Phe Gly Arg Pro
       130                 135                 140
   Glu Asn Lys Leu Met Ala Ile Asn Phe Gly Pro Gln Tyr Met Glu Ala
   145                 150                 155                 160
   Thr Ile Tyr Ser Arg Ser Pro Pro Glu Asp Arg Thr Leu Ala Asn Asn
                   165                 170                 175
   Leu Val Arg Pro Gly Cys Phe Phe Met Glu Asp Leu Ser Lys Arg Lys
               180                 185                 190
   Pro Phe Ser Asn Asp Lys Tyr Gly Ser Val Lys Arg Ala Phe Ile Ile
           195                 200                 205
   Ala Gly Ala Thr Asp Ala Glu His Lys Phe Tyr Arg Trp Glu Ile Glu
       210                 215                 220
   Asn Phe Glu Val Pro Ile Val Lys Glu Met Asp Lys Glu Thr Thr Asp
   225                 230                 235                 240
   His Met Ala Met Ile Ser Asn Pro Lys Thr Leu Cys Arg Tyr Ile Leu
                   245                 250                 255
   Asp Ile Val Glu Asn Lys Pro Asn
               260
<210> 38
   <211> 1272
   <212> DNA
   <213> Artificial Sequence
<220>
   <223> coding DNA sequence (CDS) encoding the fusion protein consisting of OeEst679 and the thioredoxin tag
<400> 38
   atgagcgata aaattattca cctgactgac gacagttttg acacggatgt actcaaagcg      60
   gacggggcga tcctcgtcga tttctgggca gagtggtgcg gtccgtgcaa aatgatcgcc     120
   ccgattctgg atgaaatcgc tgacgaatat cagggcaaac tgaccgttgc aaaactgaac     180
   atcgatcaaa accctggcac tgcgccgaaa tatggcatcc gtggtatccc gactctgctg     240
   ctgttcaaaa acggtgaagt ggcggcaacc aaagtgggtg cactgtctaa aggtcagttg     300
   aaagagttcc tcgacgctaa cctggccggt tctggttctg gccatatgca ccatcatcat     360
   catcattctt ctggtctggt gccacgcggt tctggtatga aagaaaccgc tgctgctaaa     420
   ttcgaacgcc agcacatgga cagcccagat ctgggtaccg acgacgacga caaggccatg     480
   ggagataacc aaaaacagca tcttgttctc gtacatggta tatgccacgg agcttggtgc     540
   tggtacaagc tacagacact gctggaggac gccggttacc gtgtctcaaa tcttgatatg     600
   tcagcctctg gcattgaccc gaaaagccta gctgacctta gcacatttcc tgaatataca     660
   gacccgttac ttaactttat ggagtcgatt ccatcagttg aaactgtagt tctaatcgga     720
   catagtcttg ctggcatgaa cttggctttt gcaatggaga aataccctaa aaaggtttct     780
   ttggcaattt ttattgcggc tataatgcct gacgctataa ataagccatc ctttgttttg     840
   gaagagtact tctcacggat cccgccggga gcttttatgg actctcattt cccaccattt     900
   ggccgccccg aaaataagct gatggcaata aactttggtc cgcaatacat ggaagccaca     960
   atatattcac gctccccacc tgaggatcgt actttagcaa ataatttagt gaggccaggc    1020
   tgtttcttta tggaagattt gtcaaaaagg aagccgtttt ccaacgacaa gtatggatcc    1080
   gtaaagcgtg cttttattat agctggtgca actgatgccg aacacaaatt ctatcgctgg    1140
   gaaattgaaa attttgaagt gccaatagtg aaagagatgg acaaggaaac tactgatcac    1200
   atggcaatga tttcaaatcc taaaactctt tgccgatata tattggatat tgtggaaaat    1260
   aaaccaaatt aa                                                        1272
<210> 39
   <211> 423
   <212> PRT
   <213> Artificial Sequence
<220>
   <223> Fusion protein consisting of OeEst679 and the thioredoxin tag
<400> 39
   Met Ser Asp Lys Ile Ile His Leu Thr Asp Asp Ser Phe Asp Thr Asp
   1               5                   10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly His Met His His His His His His Ser Ser Gly Leu Val Pro
           115                 120                 125
   Arg Gly Ser Gly Met Lys Glu Thr Ala Ala Ala Lys Phe Glu Arg Gln
       130                 135                 140
   His Met Asp Ser Pro Asp Leu Gly Thr Asp Asp Asp Asp Lys Ala Met
   145                 150                 155                 160
   Gly Asp Asn Gln Lys Gln His Leu Val Leu Val His Gly Ile Cys His
                   165                 170                 175
   Gly Ala Trp Cys Trp Tyr Lys Leu Gln Thr Leu Leu Glu Asp Ala Gly
               180                 185                 190
   Tyr Arg Val Ser Asn Leu Asp Met Ser Ala Ser Gly Ile Asp Pro Lys
           195                 200                 205
   Ser Leu Ala Asp Leu Ser Thr Phe Pro Glu Tyr Thr Asp Pro Leu Leu
       210                 215                 220
   Asn Phe Met Glu Ser Ile Pro Ser Val Glu Thr Val Val Leu Ile Gly
   225                 230                 235                 240
   His Ser Leu Ala Gly Met Asn Leu Ala Phe Ala Met Glu Lys Tyr Pro
                   245                 250                 255
   Lys Lys Val Ser Leu Ala Ile Phe Ile Ala Ala Ile Met Pro Asp Ala
               260                 265                 270
   Ile Asn Lys Pro Ser Phe Val Leu Glu Glu Tyr Phe Ser Arg Ile Pro
           275                 280                 285
   Pro Gly Ala Phe Met Asp Ser His Phe Pro Pro Phe Gly Arg Pro Glu
       290                 295                 300
   Asn Lys Leu Met Ala Ile Asn Phe Gly Pro Gln Tyr Met Glu Ala Thr
   305                 310                 315                 320
   Ile Tyr Ser Arg Ser Pro Pro Glu Asp Arg Thr Leu Ala Asn Asn Leu
                   325                 330                 335
   Val Arg Pro Gly Cys Phe Phe Met Glu Asp Leu Ser Lys Arg Lys Pro
               340                 345                 350
   Phe Ser Asn Asp Lys Tyr Gly Ser Val Lys Arg Ala Phe Ile Ile Ala
           355                 360                 365
   Gly Ala Thr Asp Ala Glu His Lys Phe Tyr Arg Trp Glu Ile Glu Asn
       370                 375                 380
   Phe Glu Val Pro Ile Val Lys Glu Met Asp Lys Glu Thr Thr Asp His
   385                 390                 395                 400
   Met Ala Met Ile Ser Asn Pro Lys Thr Leu Cys Arg Tyr Ile Leu Asp
                   405                 410                 415
   Ile Val Glu Asn Lys Pro Asn
               420
<210> 40
   <211> 50
   <212> DNA
   <213> Artificial Sequence
<220>
   <223> OeEst679plain_fwd
<400> 40
   atgggagata accaaaaaca gcatcttgtt ctcgtacatg gtatatgcca 50
<210> 41
   <211> 65
   <212> DNA
   <213> Artificial Sequence
<220>
   <223> OeEst679plain_rv
<400> 41
   ttaatttggt ttattttcca caatatccaa tatatatcgg caaagagttt taggatttga 60
   aatca 65
<210> 42
   <211> 34
   <212> DNA
   <213> Artificial Sequence
<220>
   <223> OeEst679NcoI_fwd
<400> 42
   cgagccatgg gagataacca aaaacagcat cttg 34
<210> 43
   <211> 50
   <212> DNA
   <213> Artificial Sequence
<220>
   <223> OeEst679HindIII_rev
<400> 43
   gctcaagctt ttaatttggt ttattttcca caatatccaa tatatatcgg 50
<210> 44
   <211> 22
   <212> DNA
   <213> Artificial Sequence
<220>
   <223> OeEst679sdm_fwd
<400> 44
   gtatatgcca cggagcttgg tg 22
<210> 45
   <211> 20
   <212> DNA
   <213> Artificial Sequence
<220>
   <223> OeEst679sdm_rev
<400> 45
   catgtacgag aacaagatgc 20
<210> 46
   <211> 780
   <212> DNA
   <213> Olea europaea
<220>
   <223> coding DNA sequence (CDS) of OE6A065391P1
<400> 46
   atggaagaaa agcaacagag gcattttgtt ctcgtacatg gtgcttgcct tggagcctgg      60
   tgctggtaca agctacaacc gctgctgaag gcggccggcc actctgtcac agtatttgat     120
   ctttcagcct ccggcattga ccccaaaagc ctggacgagc ttcgcacatt tcccgaatat     180
   acactgccat tgtttaaggt aatggagtcg attcctccag atgaaaaggt agttctagtt     240
   ggacatagtc ttggtggctt gaacttggct tttgcaatgg agaaataccc tgaaaagatc     300
   tctgtggctg tttttcttgc ggctacaatg cctgatacta tacataagcc atcctatgtt     360
   ttggaagagt actttgcacg ggcctcgccg gaagatttct tggacactaa attcgcaccg     420
   tttggtagac ctgaagatca tctcacatca atactctggg gtccaaaata cctagcatcc     480
   agattatatc aactctcccc acctgaggat gttgcattag caatatcatt agtgaggccg     540
   acctctttct ttatgcaaga tttgtcaaat aggagcccat tttccaaaga aaggtttgga     600
   tccgtaaatc gtgctttcat cataactggt gaagataaaa ccatatcacc agatttccag     660
   cgttgggaaa ttgaaaatac tggagtgaca atcgtgaaag agataaaaga tgctgaccat     720
   atggccatga tttcgaagcc tcatgcactt tgccaatatt tattggatat agcaaaataa     780
<210> 47
   <211> 259
   <212> PRT
   <213> Olea europaea
<220>
   <223> Protein sequence of OE6A065391P1
<400> 47
   Met Glu Glu Lys Gln Gln Arg His Phe Val Leu Val His Gly Ala Cys
   1               5                   10                  15
   Leu Gly Ala Trp Cys Trp Tyr Lys Leu Gln Pro Leu Leu Lys Ala Ala
               20                  25                  30
   Gly His Ser Val Thr Val Phe Asp Leu Ser Ala Ser Gly Ile Asp Pro
           35                  40                  45
   Lys Ser Leu Asp Glu Leu Arg Thr Phe Pro Glu Tyr Thr Leu Pro Leu
       50                  55                  60
   Phe Lys Val Met Glu Ser Ile Pro Pro Asp Glu Lys Val Val Leu Val
   65                  70                  75                  80
   Gly His Ser Leu Gly Gly Leu Asn Leu Ala Phe Ala Met Glu Lys Tyr
                   85                  90                  95
   Pro Glu Lys Ile Ser Val Ala Val Phe Leu Ala Ala Thr Met Pro Asp
               100                 105                 110
   Thr Ile His Lys Pro Ser Tyr Val Leu Glu Glu Tyr Phe Ala Arg Ala
           115                 120                 125
   Ser Pro Glu Asp Phe Leu Asp Thr Lys Phe Ala Pro Phe Gly Arg Pro
       130                 135                 140
   Glu Asp His Leu Thr Ser Ile Leu Trp Gly Pro Lys Tyr Leu Ala Ser
   145                 150                 155                 160
   Arg Leu Tyr Gln Leu Ser Pro Pro Glu Asp Val Ala Leu Ala Ile Ser
                   165                 170                 175
   Leu Val Arg Pro Thr Ser Phe Phe Met Gln Asp Leu Ser Asn Arg Ser
               180                 185                 190
   Pro Phe Ser Lys Glu Arg Phe Gly Ser Val Asn Arg Ala Phe Ile Ile
           195                 200                 205
   Thr Gly Glu Asp Lys Thr Ile Ser Pro Asp Phe Gln Arg Trp Glu Ile
       210                 215                 220
   Glu Asn Thr Gly Val Thr Ile Val Lys Glu Ile Lys Asp Ala Asp His
   225                 230                 235                 240
   Met Ala Met Ile Ser Lys Pro His Ala Leu Cys Gln Tyr Leu Leu Asp
                   245                 250                 255
   Ile Ala Lys
<210> 48
   <211> 780
   <212> DNA
   <213> Olea europaea
<220>
   <223> coding DNA sequence (CDS) of OeEst391
<400> 48
   atggaagaaa agcaacagag gcattttgtt ctcgtgcatg gtgcttgcct tggagcctgg      60
   tgctggtaca agctacaacc gctgctgaag gcggccggcc actctgtcac agtatttgat     120
   ctttcagcct ccggcattga ccccaaaagc ctggacgagc ttcgcacatt tcccgaatat     180
   acactgccat tatttaaggt aatggagtcg attcctccag atgaaaaggt agttctagtt     240
   ggacatagtc ttggtggctt gaacttggct tttgcaatgg agaaataccc tgaaaagatc     300
   tctgtggctg tttttcttgc ggctacaatg cctgatacca tacataaacc atcctatgtt     360
   ttggaagagt actttgcacg ggcctcgccg gaagattttt tggacactaa attcgcaccg     420
   tttggtagag ctgaagatca tctcacatca atactctggg gtccaaaata cctagcatcc     480
   agaatatatc aactctcccc acctgaggat gttgcattag caatatcatt agtgaggcca     540
   acctctttct ttttgcaaga tttgtcaaat aggagcccat tttccaatga aaggtttgga     600
   tctgtaaatc gtgctttcat cataactggc gaagataaaa ccataccacc agaattccaa     660
   cgctgggaaa ttgaaaatac tcgagtgaca atcgtgaaag agatggaaga tgctgaccac     720
   atagccatga tttcaaagcc tcatgcactt tgccaatatt tattggatat agcaaaataa     780
<210> 49
   <211> 259
   <212> PRT
   <213> Olea europaea
<220>
   <223> Protein sequence of OeEst391
<400> 49
   Met Glu Glu Lys Gln Gln Arg His Phe Val Leu Val His Gly Ala Cys
   1               5                   10                  15
   Leu Gly Ala Trp Cys Trp Tyr Lys Leu Gln Pro Leu Leu Lys Ala Ala
               20                  25                  30
   Gly His Ser Val Thr Val Phe Asp Leu Ser Ala Ser Gly Ile Asp Pro
           35                  40                  45
   Lys Ser Leu Asp Glu Leu Arg Thr Phe Pro Glu Tyr Thr Leu Pro Leu
       50                  55                  60
   Phe Lys Val Met Glu Ser Ile Pro Pro Asp Glu Lys Val Val Leu Val
   65                  70                  75                  80
   Gly His Ser Leu Gly Gly Leu Asn Leu Ala Phe Ala Met Glu Lys Tyr
                   85                  90                  95
   Pro Glu Lys Ile Ser Val Ala Val Phe Leu Ala Ala Thr Met Pro Asp
               100                 105                 110
   Thr Ile His Lys Pro Ser Tyr Val Leu Glu Glu Tyr Phe Ala Arg Ala
           115                 120                 125
   Ser Pro Glu Asp Phe Leu Asp Thr Lys Phe Ala Pro Phe Gly Arg Ala
       130                 135                 140
   Glu Asp His Leu Thr Ser Ile Leu Trp Gly Pro Lys Tyr Leu Ala Ser
   145                 150                 155                 160
   Arg Ile Tyr Gln Leu Ser Pro Pro Glu Asp Val Ala Leu Ala Ile Ser
                   165                 170                 175
   Leu Val Arg Pro Thr Ser Phe Phe Leu Gln Asp Leu Ser Asn Arg Ser
               180                 185                 190
   Pro Phe Ser Asn Glu Arg Phe Gly Ser Val Asn Arg Ala Phe Ile Ile
           195                 200                 205
   Thr Gly Glu Asp Lys Thr Ile Pro Pro Glu Phe Gln Arg Trp Glu Ile
       210                 215                 220
   Glu Asn Thr Arg Val Thr Ile Val Lys Glu Met Glu Asp Ala Asp His
   225                 230                 235                 240
   Ile Ala Met Ile Ser Lys Pro His Ala Leu Cys Gln Tyr Leu Leu Asp
                   245                 250                 255
   Ile Ala Lys
<210> 50
   <211> 1257
   <212> DNA
   <213> Artificial Sequence
<220>
   <223> coding DNA sequence (CDS) encoding the fusion protein consisting of OeEst391 and the thioredoxin tag
<400> 50
   atgagcgata aaattattca cctgactgac gacagttttg acacggatgt actcaaagcg      60
   gacggggcga tcctcgtcga tttctgggca gagtggtgcg gtccgtgcaa aatgatcgcc     120
   ccgattctgg atgaaatcgc tgacgaatat cagggcaaac tgaccgttgc aaaactgaac     180
   atcgatcaaa accctggcac tgcgccgaaa tatggcatcc gtggtatccc gactctgctg     240
   ctgttcaaaa acggtgaagt ggcggcaacc aaagtgggtg cactgtctaa aggtcagttg     300
   aaagagttcc tcgacgctaa cctggccggt tctggttctg gccatatgca ccatcatcat     360
   catcattctt ctggtctggt gccacgcggt tctggtatga aagaaaccgc tgctgctaaa     420
   ttcgaacgcc agcacatgga cagcccagat ctgggtaccg acgacgacga caaggccatg     480
   gaagaaaagc aacagaggca ttttgttctc gtgcatggtg cttgccttgg agcctggtgc     540
   tggtacaagc tacaaccgct gctgaaggcg gccggccact ctgtcacagt atttgatctt     600
   tcagcctccg gcattgaccc caaaagcctg gacgagcttc gcacatttcc cgaatataca     660
   ctgccattat ttaaggtaat ggagtcgatt cctccagatg aaaaggtagt tctagttgga     720
   catagtcttg gtggcttgaa cttggctttt gcaatggaga aataccctga aaagatctct     780
   gtggctgttt ttcttgcggc tacaatgcct gataccatac ataaaccatc ctatgttttg     840
   gaagagtact ttgcacgggc ctcgccggaa gattttttgg acactaaatt cgcaccgttt     900
   ggtagagctg aagatcatct cacatcaata ctctggggtc caaaatacct agcatccaga     960
   atatatcaac tctccccacc tgaggatgtt gcattagcaa tatcattagt gaggccaacc    1020
   tctttctttt tgcaagattt gtcaaatagg agcccatttt ccaatgaaag gtttggatct    1080
   gtaaatcgtg ctttcatcat aactggcgaa gataaaacca taccaccaga attccaacgc    1140
   tgggaaattg aaaatactcg agtgacaatc gtgaaagaga tggaagatgc tgaccacata    1200
   gccatgattt caaagcctca tgcactttgc caatatttat tggatatagc aaaataa       1257
<210> 51
   <211> 418
   <212> PRT
   <213> Artificial Sequence
<220>
   <223> Fusion protein consisting of OeEst391 and the thioredoxin tag
<400> 51
   Met Ser Asp Lys Ile Ile His Leu Thr Asp Asp Ser Phe Asp Thr Asp
   1               5                   10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly His Met His His His His His His Ser Ser Gly Leu Val Pro
           115                 120                 125
   Arg Gly Ser Gly Met Lys Glu Thr Ala Ala Ala Lys Phe Glu Arg Gln
       130                 135                 140
   His Met Asp Ser Pro Asp Leu Gly Thr Asp Asp Asp Asp Lys Ala Met
   145                 150                 155                 160
   Glu Glu Lys Gln Gln Arg His Phe Val Leu Val His Gly Ala Cys Leu
                   165                 170                 175
   Gly Ala Trp Cys Trp Tyr Lys Leu Gln Pro Leu Leu Lys Ala Ala Gly
               180                 185                 190
   His Ser Val Thr Val Phe Asp Leu Ser Ala Ser Gly Ile Asp Pro Lys
           195                 200                 205
   Ser Leu Asp Glu Leu Arg Thr Phe Pro Glu Tyr Thr Leu Pro Leu Phe
       210                 215                 220
   Lys Val Met Glu Ser Ile Pro Pro Asp Glu Lys Val Val Leu Val Gly
   225                 230                 235                 240
   His Ser Leu Gly Gly Leu Asn Leu Ala Phe Ala Met Glu Lys Tyr Pro
                   245                 250                 255
   Glu Lys Ile Ser Val Ala Val Phe Leu Ala Ala Thr Met Pro Asp Thr
               260                 265                 270
   Ile His Lys Pro Ser Tyr Val Leu Glu Glu Tyr Phe Ala Arg Ala Ser
           275                 280                 285
   Pro Glu Asp Phe Leu Asp Thr Lys Phe Ala Pro Phe Gly Arg Ala Glu
       290                 295                 300
   Asp His Leu Thr Ser Ile Leu Trp Gly Pro Lys Tyr Leu Ala Ser Arg
   305                 310                 315                 320
   Ile Tyr Gln Leu Ser Pro Pro Glu Asp Val Ala Leu Ala Ile Ser Leu
                   325                 330                 335
   Val Arg Pro Thr Ser Phe Phe Leu Gln Asp Leu Ser Asn Arg Ser Pro
               340                 345                 350
   Phe Ser Asn Glu Arg Phe Gly Ser Val Asn Arg Ala Phe Ile Ile Thr
           355                 360                 365
   Gly Glu Asp Lys Thr Ile Pro Pro Glu Phe Gln Arg Trp Glu Ile Glu
       370                 375                 380
   Asn Thr Arg Val Thr Ile Val Lys Glu Met Glu Asp Ala Asp His Ile
   385                 390                 395                 400
   Ala Met Ile Ser Lys Pro His Ala Leu Cys Gln Tyr Leu Leu Asp Ile
                   405                 410                 415
   Ala Lys
<210> 52
   <211> 31
   <212> DNA
   <213> Artificial Sequence
<220>
   <223> OeEst391_fwd
<400> 52
   atggaagaaa agcaacagag gcattttgtt c 31
<210> 53
   <211> 40
   <212> DNA
   <213> Artificial Sequence
<220>
   <223> OeEst391_rev
<400> 53
   ttattttgct atatccaata aatattggca aagtgcatga 40
<210> 54
   <211> 44
   <212> DNA
   <213> Artificial Sequence
<220>
   <223> OeEst391NcoI_fwd
<400> 54
   ttcaccatgg aagaaaagca acagaggcat tttgttctcg tgca 44
<210> 55
   <211> 49
   <212> DNA
   <213> Artificial Sequence
<220>
   <223> OeEst391HindIII_rev
<400> 55
   gcggtaagct tttattttgc tatatccaat aaatattggc aaagtgcat 49
<210> 56
   <211> 786
   <212> DNA
   <213> Olea europaea
<220>
   <223> coding DNA sequence (CDS) of OE6A025905P1
<400> 56
   atggaaggtg aaaagaagca aatacatttt gtcctagttc atggtgcatg ccatggatct      60
   tggtgctggt acaagctaca accgctgctg gaggcagcgg gccaccgcgt cacagcgatt     120
   gacctatcgg cctccggcat caacccaaaa aggctcgacg agcttcgtac attttccgac     180
   tataccttgc cattgctgga ggtaatggag tcaattccac ccgaggaaaa ggtagttcta     240
   gttggacata gtcttggtgg cttgaacttg gctcttgcaa tggagaaata cccacaaaag     300
   atttctgttg ctgtttttgt ttctgccttt atgcctgata ctgtacatag gccatcctat     360
   gttttggaac agtactttga gcggacgccg gccaaagagt ggttagacac tcaatttgaa     420
   ccgtttggta gactcgaaga ccatctcacg tcaatgctct ttggttccca attcatatca     480
   ttcaagctat atcaactctg ctcccccgag gatgtcgcgt tagctaaagc gttgttgagg     540
   ccaggctctc tttttgtgga agatttgtca aaggagagcc cattttcgaa taaaagattt     600
   ggatctgtaa agcgtgcttc catcatatgc gccaaagatg aagctataac aaaagaattt     660
   caacgctggg aaattaaaaa taccggagtg gctataacaa aagagataga agatgccgac     720
   cacatggcaa tgatctcaaa gccccaaaaa ctttatcaat gtttattgga tataatagca     780
   aactaa                                                                786
<210> 57
   <211> 261
   <212> PRT
   <213> Olea europaea
<220>
   <223> Protein sequence of OE6A025905P1
<400> 57
   Met Glu Gly Glu Lys Lys Gln Ile His Phe Val Leu Val His Gly Ala
   1               5                   10                  15
   Cys His Gly Ser Trp Cys Trp Tyr Lys Leu Gln Pro Leu Leu Glu Ala
               20                  25                  30
   Ala Gly His Arg Val Thr Ala Ile Asp Leu Ser Ala Ser Gly Ile Asn
           35                  40                  45
   Pro Lys Arg Leu Asp Glu Leu Arg Thr Phe Ser Asp Tyr Thr Leu Pro
       50                  55                  60
   Leu Leu Glu Val Met Glu Ser Ile Pro Pro Glu Glu Lys Val Val Leu
   65                  70                  75                  80
   Val Gly His Ser Leu Gly Gly Leu Asn Leu Ala Leu Ala Met Glu Lys
                   85                  90                  95
   Tyr Pro Gln Lys Ile Ser Val Ala Val Phe Val Ser Ala Phe Met Pro
               100                 105                 110
   Asp Thr Val His Arg Pro Ser Tyr Val Leu Glu Gln Tyr Phe Glu Arg
           115                 120                 125
   Thr Pro Ala Lys Glu Trp Leu Asp Thr Gln Phe Glu Pro Phe Gly Arg
       130                 135                 140
   Leu Glu Asp His Leu Thr Ser Met Leu Phe Gly Ser Gln Phe Ile Ser
   145                 150                 155                 160
   Phe Lys Leu Tyr Gln Leu Cys Ser Pro Glu Asp Ile Thr Leu Ala Lys
                   165                 170                 175
   Ala Leu Leu Arg Pro Gly Ser Leu Phe Val Glu Asp Leu Ser Lys Glu
               180                 185                 190
   Ser Pro Phe Ser Asn Glu Arg Phe Gly Ser Val Lys Arg Ala Ser Ile
           195                 200                 205
   Ile Cys Ala Lys Asp Glu Ala Ile Thr Glu Glu Phe Gln Arg Trp Glu
       210                 215                 220
   Ile Glu Asn Thr Gly Val Ala Ile Thr Lys Glu Ile Glu Asp Ala Asp
   225                 230                 235                 240
   His Met Ala Met Ile Ser Lys Pro Gln Lys Leu Tyr Gln Cys Leu Leu
                   245                 250                 255
   Asp Ile Ile Ala Asn
               260
<210> 58
   <211> 786
   <212> DNA
   <213> Olea europaea
<220>
   <223> coding DNA sequence (CDS) of OeEst905
<400> 58
   atggaaggtg aaaagaagca aatacatttt gtcctagttc atggtgcatg ccatggatct      60
   tggtgctggt acaagctaca accgctgctg gaggcagcgg gccaccgcgt cacagcgatt     120
   gacctatcgg cctccggcat caacccaaaa aggctcgacg agcttcgtac attttccgac     180
   tataccttgc cattgctgga ggtaatggag tcaattccac ccgaggaaaa ggtagttcta     240
   gttggacata gtcttggtgg cttgaacttg gctcttgcaa tggagaaata cccacaaaag     300
   atttctgttg ctgtttttgt ttctgccttt atgcctgata ctgtacatag gccatcctat     360
   gttttggaac agtactttga gcggacgccg gccaaagagt ggttagacac tcaatttgaa     420
   ccgtttggta gactcgaaga ccatctcacg tcaatgctct ttggttccca gttcatatca     480
   ttcaagctat atcaactctg ctcccccgag gatatcacgt tagctaaagc gttgttgagg     540
   ccaggctctc tttttgtgga agatttgtca aaggagagcc cattttcgaa tgaaagattt     600
   ggatctgtaa aacgtgcttc catcatatgc gccaaagatg aagctataac agaagaattt     660
   caacgctggg aaattgaaaa taccggagtg gctataacaa aagagataga agatgccgac     720
   cacatggcaa tgatctcaaa gccccaaaaa ctttatcaat gtttattgga tataatagca     780
   aactaa                                                                786
<210> 59
   <211> 786
   <212> DNA
   <213> Olea europaea
<220>
   <223> coding DNA sequence (CDS) of OeEst905 after site-directed mutagenesis
<400> 59
   atggaaggtg aaaagaagca aatacatttt gtcctagttc atggtgcatg ccacggatct      60
   tggtgctggt acaagctaca accgctgctg gaggcagcgg gccaccgcgt cacagcgatt     120
   gacctatcgg cctccggcat caacccaaaa aggctcgacg agcttcgtac attttccgac 180
   tataccttgc cattgctgga ggtaatggag tcaattccac ccgaggaaaa ggtagttcta 240
   gttggacata gtcttggtgg cttgaacttg gctcttgcaa tggagaaata cccacaaaag 300
   atttctgttg ctgtttttgt ttctgccttt atgcctgata ctgtacatag gccatcctat 360
   gttttggaac agtactttga gcggacgccg gccaaagagt ggttagacac tcaatttgaa 420
   ccgtttggta gactcgaaga ccatctcacg tcaatgctct ttggttccca gttcatatca 480
   ttcaagctat atcaactctg ctcccccgag gatatcacgt tagctaaagc gttgttgagg 540
   ccaggctctc tttttgtgga agatttgtca aaggagagcc cattttcgaa tgaaagattt 600
   ggatctgtaa aacgtgcttc catcatatgc gccaaagatg aagctataac agaagaattt 660
   caacgctggg aaattgaaaa taccggagtg gctataacaa aagagataga agatgccgac 720
   cacatggcaa tgatctcaaa gccccaaaaa ctttatcaat gtttattgga tataatagca 780
   aactaa 786
<210> 60
   <211> 261
   <212> PRT
   <213> Olea europaea
<220>
   <223> Protein sequence of OeEst905
<400> 60
   Met Glu Gly Glu Lys Lys Gln Ile His Phe Val Leu Val His Gly Ala
   1               5                   10                  15
   Cys His Gly Ser Trp Cys Trp Tyr Lys Leu Gln Pro Leu Leu Glu Ala
               20                  25                  30
   Ala Gly His Arg Val Thr Ala Ile Asp Leu Ser Ala Ser Gly Ile Asn
           35                  40                  45
   Pro Lys Arg Leu Asp Glu Leu Arg Thr Phe Ser Asp Tyr Thr Leu Pro
       50                  55                  60
   Leu Leu Glu Val Met Glu Ser Ile Pro Pro Glu Glu Lys Val Val Leu
   65                  70                  75                  80
   Val Gly His Ser Leu Gly Gly Leu Asn Leu Ala Leu Ala Met Glu Lys
                   85                  90                  95
   Tyr Pro Gln Lys Ile Ser Val Ala Val Phe Val Ser Ala Phe Met Pro
               100                 105                 110
   Asp Thr Val His Arg Pro Ser Tyr Val Leu Glu Gln Tyr Phe Glu Arg
           115                 120                 125
   Thr Pro Ala Lys Glu Trp Leu Asp Thr Gln Phe Glu Pro Phe Gly Arg
       130                 135                 140
   Leu Glu Asp His Leu Thr Ser Met Leu Phe Gly Ser Gln Phe Ile Ser
   145                 150                 155                 160
   Phe Lys Leu Tyr Gln Leu Cys Ser Pro Glu Asp Ile Thr Leu Ala Lys
                   165                 170                 175
   Ala Leu Leu Arg Pro Gly Ser Leu Phe Val Glu Asp Leu Ser Lys Glu
               180                 185                 190
   Ser Pro Phe Ser Asn Glu Arg Phe Gly Ser Val Lys Arg Ala Ser Ile
           195                 200                 205
   Ile Cys Ala Lys Asp Glu Ala Ile Thr Glu Glu Phe Gln Arg Trp Glu
       210                 215                 220
   Ile Glu Asn Thr Gly Val Ala Ile Thr Lys Glu Ile Glu Asp Ala Asp
   225                 230                 235                 240
   His Met Ala Met Ile Ser Lys Pro Gln Lys Leu Tyr Gln Cys Leu Leu
                   245                 250                 255
   Asp Ile Ile Ala Asn
               260
<210> 61
   <211> 1263
   <212> DNA
   <213> Artificial Sequence
<220>
   <223> coding DNA sequence (CDS) encoding the fusion protein consisting of OeEst905 and the thioredoxin tag
<400> 61
   atgagcgata aaattattca cctgactgac gacagttttg acacggatgt actcaaagcg      60
   gacggggcga tcctcgtcga tttctgggca gagtggtgcg gtccgtgcaa aatgatcgcc     120
   ccgattctgg atgaaatcgc tgacgaatat cagggcaaac tgaccgttgc aaaactgaac     180
   atcgatcaaa accctggcac tgcgccgaaa tatggcatcc gtggtatccc gactctgctg     240
   ctgttcaaaa acggtgaagt ggcggcaacc aaagtgggtg cactgtctaa aggtcagttg     300
   aaagagttcc tcgacgctaa cctggccggt tctggttctg gccatatgca ccatcatcat     360
   catcattctt ctggtctggt gccacgcggt tctggtatga aagaaaccgc tgctgctaaa     420
   ttcgaacgcc agcacatgga cagcccagat ctgggtaccg acgacgacga caaggccatg     480
   gaaggtgaaa agaagcaaat acattttgtc ctagttcatg gtgcatgcca cggatcttgg     540
   tgctggtaca agctacaacc gctgctggag gcagcgggcc accgcgtcac agcgattgac     600
   ctatcggcct ccggcatcaa cccaaaaagg ctcgacgagc ttcgtacatt ttccgactat     660
   accttgccat tgctggaggt aatggagtca attccacccg aggaaaaggt agttctagtt     720
   ggacatagtc ttggtggctt gaacttggct cttgcaatgg agaaataccc acaaaagatt     780
   tctgttgctg tttttgtttc tgcctttatg cctgatactg tacataggcc atcctatgtt     840
   ttggaacagt actttgagcg gacgccggcc aaagagtggt tagacactca atttgaaccg     900
   tttggtagac tcgaagacca tctcacgtca atgctctttg gttcccagtt catatcattc     960
   aagctatatc aactctgctc ccccgaggat atcacgttag ctaaagcgtt gttgaggcca    1020
   ggctctcttt ttgtggaaga tttgtcaaag gagagcccat tttcgaatga aagatttgga    1080
   tctgtaaaac gtgcttccat catatgcgcc aaagatgaag ctataacaga agaatttcaa    1140
   cgctgggaaa ttgaaaatac cggagtggct ataacaaaag agatagaaga tgccgaccac    1200
   atggcaatga tctcaaagcc ccaaaaactt tatcaatgtt tattggatat aatagcaaac    1260
   taa                                                                  1263
<210> 62
   <211> 420
   <212> PRT
   <213> Artificial Sequence
<220>
   <223> Fusion protein consisting of OeEst905 and the thioredoxin tag
<400> 62
   Met Ser Asp Lys Ile Ile His Leu Thr Asp Asp Ser Phe Asp Thr Asp
   1               5                   10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly His Met His His His His His His Ser Ser Gly Leu Val Pro
           115                 120                 125
   Arg Gly Ser Gly Met Lys Glu Thr Ala Ala Ala Lys Phe Glu Arg Gln
       130                 135                 140
   His Met Asp Ser Pro Asp Leu Gly Thr Asp Asp Asp Asp Lys Ala Met
   145                 150                 155                 160
   Glu Gly Glu Lys Lys Gln Ile His Phe Val Leu Val His Gly Ala Cys
                   165                 170                 175
   His Gly Ser Trp Cys Trp Tyr Lys Leu Gln Pro Leu Leu Glu Ala Ala
               180                 185                 190
   Gly His Arg Val Thr Ala Ile Asp Leu Ser Ala Ser Gly Ile Asn Pro
           195                 200                 205
   Lys Arg Leu Asp Glu Leu Arg Thr Phe Ser Asp Tyr Thr Leu Pro Leu
       210                 215                 220
   Leu Glu Val Met Glu Ser Ile Pro Pro Glu Glu Lys Val Val Leu Val
   225                 230                 235                 240
   Gly His Ser Leu Gly Gly Leu Asn Leu Ala Leu Ala Met Glu Lys Tyr
                   245                 250                 255
   Pro Gln Lys Ile Ser Val Ala Val Phe Val Ser Ala Phe Met Pro Asp
               260                 265                 270
   Thr Val His Arg Pro Ser Tyr Val Leu Glu Gln Tyr Phe Glu Arg Thr
           275                 280                 285
   Pro Ala Lys Glu Trp Leu Asp Thr Gln Phe Glu Pro Phe Gly Arg Leu
       290                 295                 300
   Glu Asp His Leu Thr Ser Met Leu Phe Gly Ser Gln Phe Ile Ser Phe
   305                 310                 315                 320
   Lys Leu Tyr Gln Leu Cys Ser Pro Glu Asp Ile Thr Leu Ala Lys Ala
                   325                 330                 335
   Leu Leu Arg Pro Gly Ser Leu Phe Val Glu Asp Leu Ser Lys Glu Ser
               340                 345                 350
   Pro Phe Ser Asn Glu Arg Phe Gly Ser Val Lys Arg Ala Ser Ile Ile
           355                 360                 365
   Cys Ala Lys Asp Glu Ala Ile Thr Glu Glu Phe Gln Arg Trp Glu Ile
       370                 375                 380
   Glu Asn Thr Gly Val Ala Ile Thr Lys Glu Ile Glu Asp Ala Asp His
   385                 390                 395                 400
   Met Ala Met Ile Ser Lys Pro Gln Lys Leu Tyr Gln Cys Leu Leu Asp
                   405                 410                 415
   Ile Ile Ala Asn
               420
<210> 63
   <211> 30
   <212> DNA
   <213> Artificial Sequence
<220>
   <223> OeEst905plain_fwd
<400> 63
   atggaaggtg aaaagaagca aatacatttt 30
<210> 64
   <211> 40
   <212> DNA
   <213> Artificial Sequence
<220>
   <223> OeEst905plain_rev
<400> 64
   ttagtttgct attatatcca ataaacattg ataaagtttt 40
<210> 65
   <211> 36
   <212> DNA
   <213> Artificial Sequence
<220>
   <223> OeEst905NcoI_fwd
<400> 65
   cgagccatgg aaggtgaaaa gaagcaaata catttt 36
<210> 66
   <211> 50
   <212> DNA
   <213> Artificial Sequence
<220>
   <223> OeEst905XhoI_rev
<400> 66
   atacctcgag ttagtttgct attatatcca ataaacattg ataaagtttt 50
<210> 67
   <211> 21
   <212> DNA
   <213> Artificial Sequence
<220>
   <223> OeEst905sdm_fwd
<400> 67
   gtgcatgcca cggatcttgg t 21
<210> 68
   <211> 25
   <212> DNA
<210> 70
   <211> 261
   <212> PRT
   <213> Glycine max
<220>
   <223> Glyma16g26060
<400> 70
   Met Gly Ser Gln Asn Cys Met Asp Arg Lys His Tyr Val Leu Val His
   1               5                   10                  15
   Gly Ala Cys His Gly Ala Trp Cys Trp Tyr Lys Leu Lys Pro Arg Leu
               20                  25                  30
   Glu Ser Ala Gly His Lys Val Thr Val Leu Asp Leu Ala Ala Ser Gly
           35                  40                  45
   Thr Asn Met Lys Lys Ile Glu Asp Val Asp Thr Phe Ser Glu Tyr Ser
       50                  55                  60
   Ala Pro Leu Leu Gln Leu Met Ala Thr Ile Pro Ser Asn Glu Lys Leu
   65                  70                  75                  80
   Val Leu Val Gly His Ser Leu Gly Gly Leu Asn Ile Ala Leu Ala Met
                   85                  90                  95
   Glu Lys Phe Pro Glu Lys Val Ala Val Gly Val Phe Leu Thr Ala Phe
               100                 105                 110
   Ala Pro Asp Thr Glu His His Pro Ser Tyr Val Leu Glu Lys Tyr Asn
           115                 120                 125
   Glu Arg Thr Pro Leu Ala Ala Trp Leu Asp Thr Glu Phe Ala Pro Ser
       130                 135                 140
   Gly Asn Lys Thr Ser Met Phe Phe Gly Pro Asn Phe Leu Ser Asp Lys
   145                 150                 155                 160
   Leu Tyr Gln Leu Ser Pro Ile Glu Asp Leu Glu Leu Ala Lys Thr Leu
                   165                 170                 175
   Ala Arg Pro Ser Ser Leu Phe Met Glu Asp Leu Thr Lys Gln Lys Asn
               180                 185                 190
   Phe Ser Lys Glu Gly Tyr Gly Ser Val Pro Arg Ala Phe Ile Val Cys
           195                 200                 205
   Thr Glu Asp Leu Gly Ile Pro Leu Glu Tyr Gln Leu Leu Met Ile Gln
       210                 215                 220
   Asn Val Gly Phe Asn Asp Val Val Glu Val Lys Asp Ala Asp His Met
   225                 230                 235                 240
   Val Met Leu Cys Lys Pro Gln Glu Leu Phe Asp Ser Leu Gln Gln Ile
                   245                 250                 255
   Ala Thr Lys Tyr Ala
               260
<210> 71
   <211> 260
   <212> PRT
   <213> Nicotiana tabacum
<220>
   <223> SABP2
<400> 71
   Met Lys Glu Gly Lys His Phe Val Leu Val His Gly Ala Cys His Gly
   1               5                   10                  15
   Gly Trp Ser Trp Tyr Lys Leu Lys Pro Leu Leu Glu Ala Ala Gly His
               20                  25                  30
   Lys Val Thr Ala Leu Asp Leu Ala Ala Ser Gly Thr Asp Leu Arg Lys
           35                  40                  45
   Ile Glu Glu Leu Arg Thr Leu Tyr Asp Tyr Thr Leu Pro Leu Met Glu
       50                  55                  60
   Leu Met Glu Ser Leu Ser Ala Asp Glu Lys Val Ile Leu Val Gly His
   65                  70                  75                  80
   Ser Leu Gly Gly Met Asn Leu Gly Leu Ala Met Glu Lys Tyr Pro Gln
                   85                  90                  95
   Lys Ile Tyr Ala Ala Val Phe Leu Ala Ala Phe Met Pro Asp Ser Val
               100                 105                 110
   His Asn Ser Ser Phe Val Leu Glu Gln Tyr Asn Glu Arg Thr Pro Ala
           115                 120                 125
   Glu Asn Trp Leu Asp Thr Gln Phe Leu Pro Tyr Gly Ser Pro Glu Glu
       130                 135                 140
   Pro Leu Thr Ser Met Phe Phe Gly Pro Lys Phe Leu Ala His Lys Leu
   145                 150                 155                 160
   Tyr Gln Leu Cys Ser Pro Glu Asp Leu Ala Leu Ala Ser Ser Leu Val
                   165                 170                 175
   Arg Pro Ser Ser Leu Phe Met Glu Asp Leu Ser Lys Ala Lys Tyr Phe
               180                 185                 190
   Thr Asp Glu Arg Phe Gly Ser Val Lys Arg Val Tyr Ile Val Cys Thr
           195                 200                 205
   Glu Asp Lys Gly Ile Pro Glu Glu Phe Gln Arg Trp Gln Ile Asp Asn
       210                 215                 220
   Ile Gly Val Thr Glu Ala Ile Glu Ile Lys Gly Ala Asp His Met Ala
   225                 230                 235                 240
   Met Leu Cys Glu Pro Gln Lys Leu Cys Ala Ser Leu Leu Glu Ile Ala
                   245                 250                 255
   His Lys Tyr Asn
               260